# EUROPEAN PATENT APPLICATION

(11) **EP 4 012 037 A1**
(43) Date of publication of application: **15.06.2022**
(21) Application number: 20849939.2
(22) Date of filing: 07.08.2020
(51) Int. Cl.: C12N 15/90, C12N 15/864, C12N 15/10, C12N 9/22, C12N 15/113

(54) **CRISPR/CAS9 GENE EDITING SYSTEM AND APPLICATION THEREOF**

(30) Priority: 08.08.2019 CN 201910731803; 08.08.2019 CN 201910731794; 08.08.2019 CN 201910731795; 08.08.2019 CN 201910731802; 08.08.2019 CN 201910731401; 08.08.2019 CN 201910731402; 08.08.2019 CN 201910731390; 08.08.2019 CN 201910731412; 08.08.2019 CN 201910731396; 08.08.2019 CN 201910731398
(71) Applicant: Fudan University, Shanghai 200433 (CN)
(72) Inventor: WANG, Yongming, Shanghai 200433 (CN); HU, Ziying, Shanghai 200433 (CN); WANG, Daqi, Shanghai 200433 (CN); WANG, Shuai, Shanghai 200433 (CN)
(74) Representative: Ström & Gulliksson AB
(86) International application number: PCT/CN2020/107880
(87) International publication number: WO 2021/023307

(57) **Abstract**

A CRISPR/Cas9 gene editing system and application thereof. The gene editing system is a complex formed by a specific Cas9 protein and sgRNA, and can precisely position a target DNA sequence and cause cutting so that double strand breaks occur to the target DNA sequence; the gene editing refers to intracellular or in vitro gene editing. The used specific Cas9 protein is small and only has about 1000 amino acids, and thus an identified PAM sequence is simple; the Cas9 protein has an amino acid sequence represented by any one of SEQ ID NOs: 1-10 and 58; the sgRNA has a nucleotide sequence represented by SEQ ID NO: 11.

## Description

### TECHNICAL FIELD

The present disclosure belongs to the technical field of gene editing, and particularly relates to a CRISPR/Cas9 system capable of performing gene editing in cells; and related applications thereof.

### BACKGROUND OF THE INVENTION

CRISPR/Cas9 is an acquired immune system that bacteria and archaebacteria have evolved to resist the invasion of foreign viruses or plasmids. In the CRISPR/Cas9 system, crRNA (CRISPR-derived RNA), tracrRNA (trans-activating RNA) and a Cas9 protein forms a complex which recognizes the PAM (Protospacer Adjacent Motif) sequence of the target site, crRNA forms a complementary structure with the target DNA sequence, and the Cas9 protein performs the function of DNA cleavage, causing DNA break damage. In the complex, tracrRNA and crRNA can be fused into a single guide RNA (sgRNA) through a linker sequence. When DNA is broken and damaged, there are two main repair mechanisms for DNA damage in cells which are responsible for repairing: non-homologous end-joining (NHEJ) and homologous recombination (HR). The NHEJ repair may cause the deletion or insertion of base(s), and thus can be used for gene knockout. In the case that a homologous template is provided, the HR repair can be used for site-specific insertion and precise base substitution for a gene.

In addition to basic scientific research, CRISPR/Cas9 also has a wide range of clinical applications. When the CRISPR/Cas9 system is used for gene therapy, Cas9 and sgRNA need to be introduced into a body. At present, AAV virus is the most effective delivery vector for gene therapy. However, the DNA capable of being packaged by the AAV virus generally does not exceed 4.5 kb. SpCas9 is widely used because it recognizes a simple PAM sequence (recognizing NGG) and has high activity. However, a SpCas9 protein itself has 1368 amino acids, and thus when it is in complex with sgRNA and a promoter, it cannot be effectively packaged into AAV virus, thereby limiting the clinical applications thereof. In order to overcome the above problems, researchers have invented several small Cas9 proteins, comprising SaCas9 (the PAM sequence is NNGRRT), St1Cas9 (the PAM sequence is NNAGAW), NmCas9 (the PAM sequence is NNNNGATT), Nme2Cas9 (the PAM sequence is NNNNCC), and CjCas9 (the PAM sequence is NNNNRYAC). However, these Cas9 proteins either tend to be off-target (*i.e.*, performing cleavage at a non-target site), or recognize complicated PAM sequences, or have low editing activity, and thus are difficult to be widely used.

Therefore, a small CRISPR/Cas9 system having high editing activity and high specificity, and recognizing a simple PAM sequence is the hope for solving the above problems.

### SUMMARY OF THE INVENTION

In view of the above problems, the present disclosure aims to provide a new CRISPR/Cas9 gene editing system having high editing activity, high specificity, and a small Cas9 protein, and recognizing a simple PAM sequence; and the applications thereof.

Thus, in a first aspect, the present disclosure provides a CRISPR/Cas9 system for gene editing in cells or *in vitro,* wherein the CRISPR/Cas9 system is a complex of a Cas9 protein and a sgRNA, which is capable of accurately locating and cleaving a target DNA sequence so as to cause double-strand break damage to the target DNA sequence;
the Cas9 protein is:
   *a Sauri*Cas9 protein having an amino acid sequence represented by SEQ ID NO: 1,
   a *Sha*Cas9 protein having an amino acid sequence represented by SEQ ID NO: 2,
   a *Slug*Cas9 protein having an amino acid sequence represented by SEQ ID NO: 3,
   a *Slut*Cas9 protein having an amino acid sequence represented by SEQ ID NO: 4,
   a *Sa-Sauri*Cas9 protein having an amino acid sequence represented by SEQ ID NO: 5,
   a *Sa-Sep*Cas9 protein having an amino acid sequence represented by SEQ ID NO: 6,
   a *Sa-Seq*Cas9 protein having an amino acid sequence represented by SEQ ID NO: 7,
   a *Sa-Sha*Cas9 protein having an amino acid sequence represented by SEQ ID NO: 8,
   a *Sa-Slug*Cas9 protein having an amino acid sequence represented by SEQ ID NO: 9,
   a *Sa-Slut*Cas9 protein having an amino acid sequence represented by SEQ ID NO: 10, or
   a *Slug*Cas9-*HF* protein having an amino acid sequence represented by SEQ ID NO: 58, or
   a Cas9 protein having an amino acid sequence that is at least 80% identical to the amino acid sequence represented by any one of SEQ ID NOs: 1-10 and SEQ ID NO: 58; and
the sgRNA has a nucleotide sequence represented by SEQ ID NO: 11, or is a modified sgRNA sequence based on SEQ ID NO: 11.

In a second aspect, the present disclosure provides a method for gene editing in cells with the CRISPR/Cas9 system for gene editing according to the first aspect of the present disclosure, wherein the method edits a target DNA sequence by recognizing and locating the target DNA sequence with a complex of a Cas9 protein and a sgRNA, and the method comprises the steps of:
(1) synthesizing a Cas9 gene sequence and cloning it into an expression vector such as pAAV2_ITR, to obtain an expression vector cloned with the Cas9 gene sequence, such as pAAV2_Cas9_ITR, wherein the Cas9 gene sequence:
   (a) has a nucleotide sequence encoding an amino acid sequence represented by any one of SEQ ID NOs: 1-10 and SEQ ID NO: 58;
   (b) has a nucleotide sequence encoding an amino acid sequence that is at least 80% identical to the amino acid sequence represented by any one of SEQ ID NOs: 1-10 and SEQ ID NO: 58; or
   (c) is a humanized Cas9 gene sequence, for example, the one having a nucleotide sequence represented by any one of SEQ ID NOs: 23-32 and SEQ ID NO: 112;
(2) synthesizing oligo single-stranded DNAs corresponding to the sgRNA, *i.e.,* an oligo forward-strand sequence and an oligo reverse-strand sequence, and annealing and ligating the oligo forward-strand sequence and the oligo reverse-strand sequence to a restriction site of the expression vector cloned with the Cas9 gene sequence, such as the BsaI digestion site of plasmid pAAV2_Cas9_U6_BsaI, to obtain an expression vector, such as pAAV2_Cas9-hU6-sgRNA, for expressing the Cas9 protein and the sgRNA; wherein the sgRNA has a nucleotide sequence represented by SEQ ID NO: 11, or a nucleotide sequence that is at least 80% identical to a nucleotide sequence represented by SEQ ID NO: 11, or a modification comprising, e.g., phosphorylation, shortening, lengthening, sulfurization, methylation or hydroxylation, based on the nucleotide sequence represented by SEQ ID NO: 11;
(3) delivering the expression vector expressing the Cas9 protein and the sgRNA into cells comprising a target site to edit the target site.

In a third aspect, the present disclosure provides a kit of a CRISPR/Cas9 system for gene editing, the kit comprising:
(1) a Cas9 protein and a sgRNA, wherein the Cas9 protein is:
   a *SauriCas9* protein having an amino acid sequence represented by SEQ ID NO: 1,
   a *Sha*Cas9 protein having an amino acid sequence represented by SEQ ID NO: 2,
   a *Slug*Cas9 protein having an amino acid sequence represented by SEQ ID NO: 3,
   a *Slut*Cas9 protein having an amino acid sequence represented by SEQ ID NO: 4,
   a *Sa-Sauri*Cas9 protein having an amino acid sequence represented by SEQ ID NO: 5,
   a *Sa-Sep*Cas9 protein having an amino acid sequence represented by SEQ ID NO: 6,
   a *Sa-Seq*Cas9 protein having an amino acid sequence represented by SEQ ID NO: 7,
   a *Sa-Sha*Cas9 protein having an amino acid sequence represented by SEQ ID NO: 8,
   a *Sa-Slug*Cas9 protein having an amino acid sequence represented by SEQ ID NO: 9,
   a *Sa-Slut*Cas9 protein having an amino acid sequence represented by SEQ ID NO: 10, or
   a *Slug*Cas9*-HF* protein having an amino acid sequence represented by SEQ ID NO: 58, or
   a Cas9 protein having an amino acid sequence that is at least 80% identical to the amino acid sequence represented by any one of SEQ ID NOs: 1-10 and SEQ ID NO: 58; and
   the sgRNA has a nucleotide sequence represented by SEQ ID NO: 11, or is a modified sgRNA sequence based on SEQ ID NO: 11;
      or
(2) an expression vector cloned with a Cas9 gene sequence and a sequence expressing a sgRNA, wherein
   the Cas9 gene sequence:
      (a) has a nucleotide sequence encoding an amino acid sequence represented by any one of SEQ ID NO: 1-10 and SEQ ID NO: 58;
      (b) has a nucleotide sequence encoding an amino acid sequence that is at least 80% identical to the amino acid sequence represented by any one of SEQ ID NO: 1-10 and SEQ ID NO: 58; or
      (c) is a humanized Cas9 gene sequence, for example, the one having a nucleotide sequence represented by any one of SEQ ID NOs: 23-32 and SEQ ID NO: 112; and
   the sgRNA has a nucleotide sequence represented by SEQ ID NO: 11, or a modified sgRNA sequence based on SEQ ID NO: 11.

In a fourth aspect, the present disclosure provides use of the CRISPR/Cas9 system for gene editing according to the first aspect of the present disclosure in gene knockout, site-directed base change, site-directed insertion, regulation of gene transcription level, regulation of DNA methylation, modification of DNA acetylation, modification of histone acetylation, single-base conversion or chromatin imaging tracking.

Compared with the existing CRISPR/Cas9 systems for gene editing in the prior art, the CRISPR/Cas9 gene editing system of the present disclosure comprises a smaller Cas9 protein with fewer amino acids than the prior art, and thus can be effectively packaged. Furthermore, the CRISPR/Cas9 gene editing system of the present disclosure can recognize a relatively simple PAM sequence, and thus can target more DNA sequences in the genome and has higher editing efficiency.

### BRIEF DESCRIPTION OF THE FIGURES

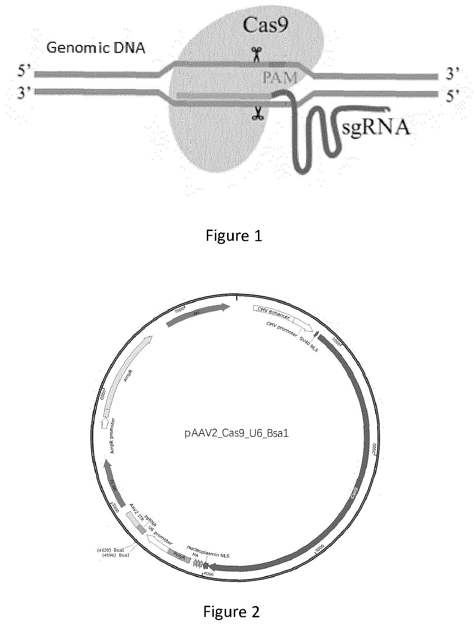

Figure 1 is a schematic diagram of the cleavage of the target DNA sequence by using a CRISPR/Cas9 gene editing system, wherein the gray oval represents a Cas9 protein, the black curve represents a sgRNA sequence, and the darkened area in one chain of the genome represents a PAM sequence.
Figure 2 is a schematic diagram of the map of plasmids pAAV2_Cas9_U6_BsaI, comprising elements such as AAV2 ITR, CMV enhancer, CMV promoter, SV40 NLS, Cas9, nucleoplasmin NLS, 3x HA, bGH poly(A), human U6 promoter (hU6), BsaI endonuclease site, and sgRNA scaffold sequence, and the like.

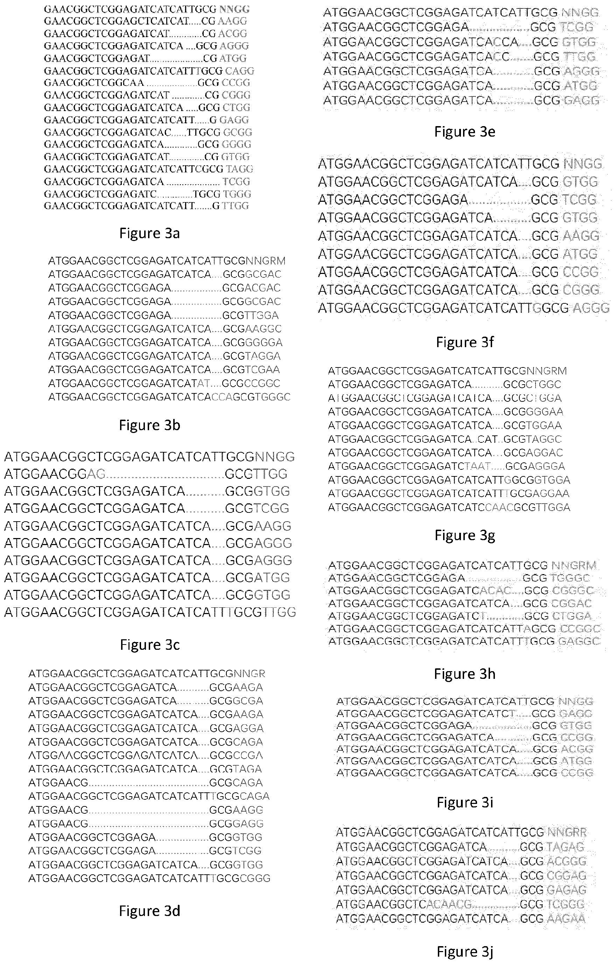

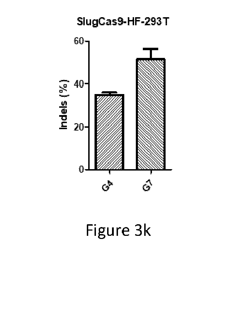

Figure 3a to Figure 3j represent some results from next-generation sequencing of the DNA sequence of the target site upon editing, wherein the edited results comprise deletions, insertions or mismatches, and the last 4 bp or 5 bp represents the PAM sequence, which is NNGG, NNGRM, NNGG, NNGR, NNGG, NNGG, NNGRM, NNGRM, NNGG and NNGRR, respectively, from Figures 3a to 3j. Figure 3k shows the editing of two target sites by the *SlugCas9-HF* gene editing system, wherein the X axis represents the two target sites, G4 and G7, and the Y axis represents indel efficiency.

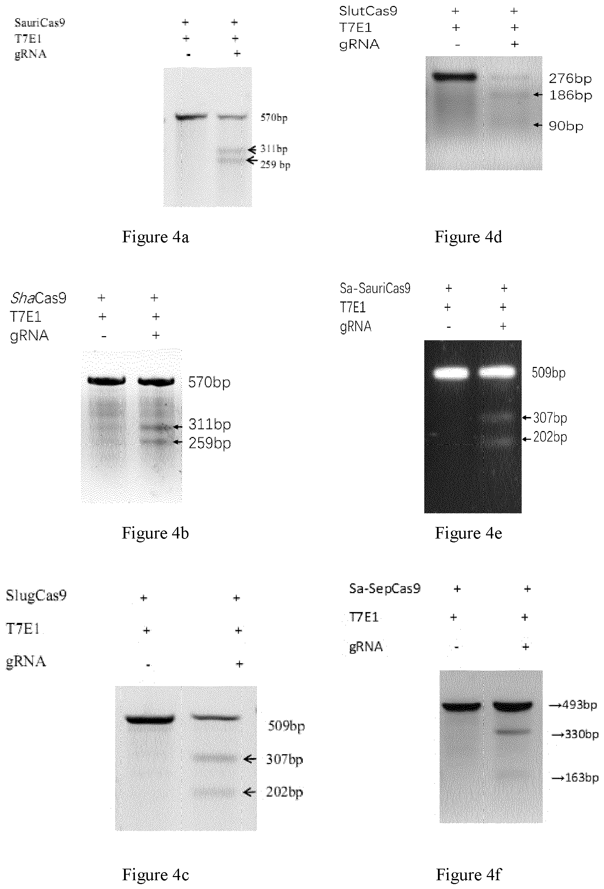

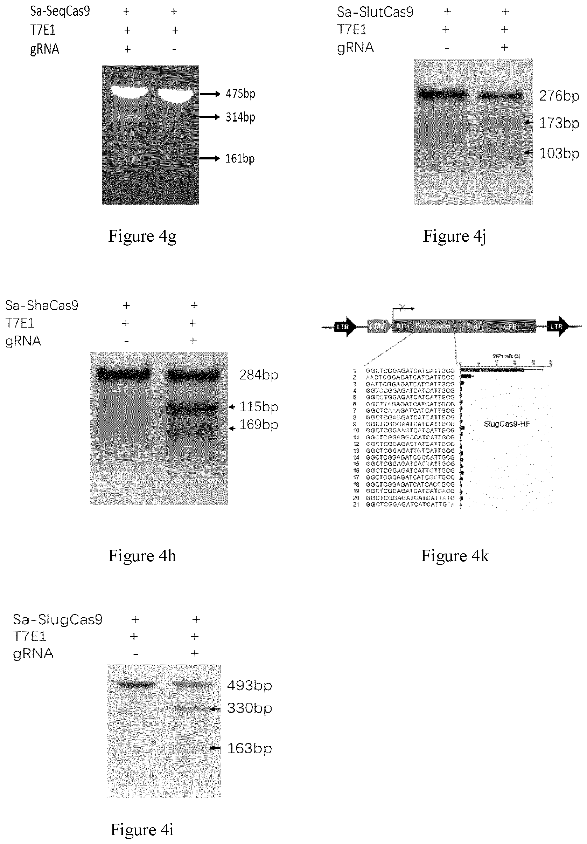

Figure 4a to Figure 4j represent digestion results of T7 Endonuclease I at the endogenous site, wherein the arrows indicate the size of the cleaved fragments. Figure 4k shows the detection result of the specificity of *SlugCas9-HF* gene editing system in a GFP-reporter cell line, wherein the top shows the schematic diagram of the GFP reporter system. A specific target DNA sequence and a PAM are inserted between the start codon ATG and the GFP-coding sequence to cause a GFP frameshift mutation. When the target DNA is cleaved by the gene editing system, some cells can restore the GFP reading frame through the self-repairing system of the cells to produce green fluorescence. In the histogram of Figure 4k, the Y-axis represents the GFP positive ratio, and the X-axis represents the sequences of on-target sgRNA and mismatch sgRNAs.

### DETAILED DESCRIPTION OF THE INVENTION

The following embodiments are provided to further illustrate the present disclosure, but not to limit the protection scope of the present disclosure in any form; on the contrary, the protection scope of the present disclosure is defined by the appended claims.

As described in the Background Section, current CRISPR/Cas9 gene editing systems have various problems. For example, the Cas9 protein is too large, so that the system cannot be effectively packaged into a vector such as a virus. For another example, the current PAM sequences are relatively complicated, resulting in a small editing range, and it is difficult for the current CRISPR/Cas9 gene editing systems to be widely used. For another example, the current small Cas9 proteins generally have low editing activity.

For the above problems, the present disclosure aims to provide a new CRISPR/Cas9 gene editing system having high editing activity, high specificity, and a small Cas9 protein, and recognizing a simple PAM sequence; and the applications thereof.

Thus, in the first aspect, the present disclosure provides a CRISPR/Cas9 system for gene editing in cells or *in vitro,* wherein the CRISPR/Cas9 system is a complex of a Cas9 protein and a sgRNA, and is capable of accurately locating and cleaving a target DNA sequence so as to cause double-strand break damage to the target DNA sequence;
the Cas9 protein is:
   a *SauriCas9* protein having an amino acid sequence represented by SEQ ID NO: 1,
   a *Sha*Cas9 protein having an amino acid sequence represented by SEQ ID NO: 2,
   a *Slug*Cas9 protein having an amino acid sequence represented by SEQ ID NO: 3,
   a *Slut*Cas9 protein having an amino acid sequence represented by SEQ ID NO: 4,
   a *Sa-Sauri*Cas9 protein having an amino acid sequence represented by SEQ ID NO: 5,
   a *Sa-Sep*Cas9 protein having an amino acid sequence represented by SEQ ID NO: 6,
   a *Sa-Seq*Cas9 protein having an amino acid sequence represented by SEQ ID NO: 7,
   a *Sa-Sha*Cas9 protein having an amino acid sequence represented by SEQ ID NO: 8,
   a *Sa-Slug*Cas9 protein having an amino acid sequence represented by SEQ ID NO: 9,
   a *Sa-Slut*Cas9 protein having an amino acid sequence represented by SEQ ID NO: 10, or
   a *SlugCas9-HF* protein having an amino acid sequence represented by SEQ ID NO: 58, or
the Cas9 protein having an amino acid sequence that is at least 80% identical to the amino acid sequence represented by any one of SEQ ID NOs: 1-10 and SEQ ID NO: 58; and
the sgRNA has a nucleotide sequence represented by SEQ ID NO: 11, or is a modified sgRNA sequence based on SEQ ID NO: 11.

In the context of the present disclosure, the sequences of SEQ ID NOs: 1-11 and SEQ ID NO: 58 are as follows.
**SEQ ID NO: 1** - the amino acid sequence of *SauriCas9* protein: 
MQENQQKQNYILGLDIGITSVGYGLIDSKTREVIDAGVRLFPEADSENNSNRRSKRGA
RRLKRRRIHRLNRVKDLLADYQMIDLNNVPKSTDPYTIRVKGLREPLTKEEFAIALLHI
AKRRGLHNISVSMGDEEQDNELSTKQQLQKNAQQLQDKYVCELQLERLTNINKVRGE
KNRFKTEDFVKEVKQLCETQRQYHNIDDQFIQQYIDLVSTRREYFEGPGNGSPYGWDG
 
DLLKWYEKLMGRCTYFPEELRSVKYAYSADLFNALNDLNNLVVTRDDNPKLEYYEK
YHIIENVFKQKKNPTLKQIAKEIGVQDYDIRGYRITKSGKPQFTSFKLYHDLKNIFEQAK
YLEDVEMLDEIAKILTIYQDEISIKKALDQLPELLTESEKSQIAQLTGYTGTHRLSLKCIHI
VIDELWESPENQMEIFTRLNLKPKKVEMSEIDSIPTTLVDEFILSPVVKRAFIQSIKVINAV
INRFGLPEDIIIELAREKNSKDRRKFINKLQKQNEATRKKIEQLLAKYGNTNAKYMIEKI
KLHDMQEGKCLYSLEAIPLEDLLSNPTHYEVDHIIPRSVSFDNSLNNKVLVKQSENSKK
GNRTPYQYLSSNESKISYNQFKQHILNLSKAKDRISKKKRDMLLEERDINKFEVQKEFI
NRNLVDTRYATRELSNLLKTYFSTHDYAVKVKTINGGFTNHLRKVWDFKKHRNHGYK
HHAEDALVIANADFLFKTHKALRRTDKILEQPGLEVNDTTVKVDTEEKYQELFETPKQ
VKNIKQFRDFKYSHRVDKKPNRQLINDTLYSTREIDGETYVVQTLKDLYAKDNEKVKK
LFTERPQKILMYQHDPKTFEKLMTILNQYAEAKNPLAAYYEDKGEYVTKYAKKGNGP
AIHKIKYIDKKLGSYLDVSNKYPETQNKLVKLSLKSFRFDIYKCEQGYKMVSIGYLDVL
KKDNYYYIPKDKYEAEKQKKKIKESDLFVGSFYYNDLIMYEDELFRVIGVNSDINNLV
ELNMVDITYKDFCEVNNVTGEKRIKKTIGKRVVLIEKYTTDILGNLYKTPLPKKPQLIFK
RGEL;
**SEQ ID NO: 2** - the amino acid sequence of *ShaCas9* protein: 
MKTDYILGLDIGITSVGYGIINYNDKSIIDAGVRLFPEANVENNEGRRSKRGARRLKRR
RIHRLERVKQLLLDYKLLDSIDVIPQSTNPYEIRVRGLREKLTKDELVIALLHLAKRRGI
HNIDVIDQEEDASNELSTKEQLSKNNLLLRDKSICEVLLERYNEGKVRGEKNRFKTSDI
VNEIRQILETQKEVHHLDDSFIDKYIELVETRREYYEGPGEGSPYGWGADLKKWYEHL
MGRCTYFPEELRSVKYAYSADLFNALNDLNNLVIQRDGLNKLEYHEKYHIIENVFKQK
KKPTLKQISNEIGVNPEDIKGYRITKSGKENFTEFKLYHDLKKILKDQSILENIQLLDQIA
EIITIYQDKESIKKELEQLTEPINEIDKESISDLAGYNGTHRLSLKCINLVLEELWHTSRNQ
MEIFAYLNIKPRKIDLQKANKIPKDMIDEFILSPVVKRTFGQAINVINKVIEKYGVPKDIII
ELARENNTKDKQKFINELQKKNEKTRQRINEIIGKTGNQNAKRLVEKIRLHDEQEGKCL
YSLESIPLEDLLNNPNHYEVDHIIPRSVSFDNSYQNKVLVKQTENSKKGNRTPYQYLNS
GEAKLSYNQFKQHVLNLSKSKDRISKKKKEYLLEERDINKFEVQKEFINRNLVDTRYAT
RELTNYLKAYFSANDMDVKVKTINGSFTNHLRKVWDFNKERNHGYKHHAEDALIIAN
ADFLFKENKKLKAANKVLEQPERKEIETKIEVNSDENYQDLFVIPQQVKEIKEFRDFKY
SHRVDKKPNRQLINDTLYSTRVKDDDLYIISPIKNIYSKDNTDLKKHFNKNPEKFLMYQ
 
KDPKTFEKLEVIMKQYANEKNPLAKYHEETGEYLTKYSKTNNGPIVKSIKMFGKKVG
NHLDVTNQYNNSRNKLVKLSFKSYRFDVYLTDKGYKFVSITYLDVLKKENYYYISEA
KYDKLKLNKGIDDKAKFIGSFYYNDLIELDGEVYTVIGVNNDKNNVIELNLPEIRYKEY
CEINNIKGSGRLRITIGKKVNSIRKLSTDVLGNRYYQSFAKKPQLVFKKGI*;
**SEQ ID NO: 3** - the amino acid sequence of *Slug*Cas9 protein: 
MNQKFILGLDIGITSVGYGLIDYETKNIIDAGVRLFPEANVENNEGRRSKRGSRRLKRR
RIHRLERVKKLLEDYNLLDQSQIPQSTNPYAIRVKGLSEALSKDELVIALLHIAKRRGIH
KIDVIDSNDDVGNELSTKEQLNKNSKLLKDKFVCQIQLERMNEGQVRGEKNRFKTADI
IKEIIQLLNVQKNFHQLDENFINKYIELVEMRREYFEGPGKGSPYGWEGDPKAWYETL
MGHCTYFPDELRSVKYAYSADLFNALNDLNNLVIQRDGLSKLEYHEKYHIIENVFKQK
KKPTLKQIANEINVNPEDIKGYRITKSGKPQFTEFKLYHDLKSVLFDQSILENEDVLDQI
AEILTIYQDKDSIKSKLTELDILLNEEDKENIAQLTGYTGTHRLSLKCIRLVLEEQWYSSR
NQMEIFTHLNIKPKKINLTAANKIPKAMIDEFILSPVVKRTFGQAINLINKIIEKYGVPEDI
IIELARENNSKDKQKFINEMQKKNENTRKRINEIIGKYGNQNAKRLVEKIRLHDEQEGK
CLYSLESIPLEDLLNNPNHYEVDHIIPRSVSFDNSYHNKVLVKQSENSKKSNLTPYQYFN
SGKSKLSYNQFKQHILNLSKSQDRISKKKKEYLLEERDINKFEVQKEFINRNLVDTRYA
TRELTNYLKAYFSANNMNVKVKTINGSFTDYLRKVWKFKKERNHGYKHHAEDALIIA
NADFLFKENKKLKAVNSVLEKPEIESKQLDIQVDSEDNYSEMFIIPKQVQDIKDFRNFK
YSHRVDKKPNRQLINDTLYSTRKKDNSTYIVQTIKDIYAKDNTTLKKQFDKSPEKFLMY
QHDPRTFEKLEVIMKQYANEKNPLAKYHEETGEYLTKYSKKNNGPIVKSLKYIGNKLG
SHLDVTHQFKSSTKKLVKLSIKPYRFDVYLTDKGYKFITISYLDVLKKDNYYYIPEQKY
DKLKLGKAIDKNAKFIASFYKNDLIKLDGEIYKIIGVNSDTRNMIELDLPDIRYKEYCEL
NNIKGEPRIKKTIGKKVNSIEKLTTDVLGNVFTNTQYTKPQLLFKRGN*;
**SEQ ID NO: 4** - the amino acid sequence of *Slut*Cas9 protein: 
MRNSYILGLDIGITSVGYGIIDRVTREVIDAGVRLFPEANVENNEGRRSKRGARRLKRR
RIHRLNRIKQLLKNAGLLEGDVLPKSTNPYEIRVRGLRSPLTKDELVIALLHIAKRRGIH
NINIVGDDEETDSTLSTTAQLKKNEKALKGQFVCELQLDRLANAHQVRGEKNRFKTE
DIVKEVRALLQQQQNFHNIDNSFVEQYIALLESRRTYYEGPGEGSPYGWDGDIKKWYE
MLMGYCTYFPEELRSVKYAYTADLFNALNDLNNLVITRDDNSKLTYAEKYHIIENVFK
QKKVPTLKQIAKEIGVNESDIKGYRINKSEKPLFTSFKLYHDLKSVFSDPTKLEDIDLLD
 
RIAVVLTMYQDAESMKAALNTFPEVFSEAEKEKLSALTGYAGTHRLSLKCMNLLIPDL
WQTSLNQMELFVKLNLKPQKLDLSQCHQIPTQLVDEFILSPVVKRAFTQSIKVINAIIQK
YGLPDDIIIELAREKNSADKRKFLNQLQKKNEKARHEINTLVAQYGQPNAKRLVEKITL
HQQQEGKCLYSLKDIPLEQLLKQPYLYEVDHIIPRSVSFDNSMQNKVLVLAEENAKKG
NQTPYQYLNSREASMTYPEFKQHILNLSKAKDRISKKKRNYLLEERDINKFDVQKDFI
NRNLVDTRYATRELASLLKAYFKTHELPVKVKTINGGFTHYLRKVWKFDKDRNKGYK
HHAEDALIIANADFLFKNQTLNKIEAILNEPGREVESDTVKVQSEDNYQDLFENTKKAF
AIKNFKDFKFSHRVDQKPNRQLVNDTLYSTREVNEDLYVVQTLKDIYSKDNKDVKRLF
DKQPEKFLMFQHDPETFKKFELAMKQYAEEKNPLARYYEEQGYITKYAKKGDGPPVK
SLKYIGKKVGKHLDVTGDYEDSNRKLVKLSLKSFRFDIYHTDKGYKMVPITYLDVQK
KEKYYYIPTEKYEALKQEKGINQNAQFIGSFYYNDLIEFDGELYRVIGINNGDKNLVEL
DMVDIRYKEYCELNSITTTPRIVKTISPKTQSIEKYTTDILGNLYKAQPGKKPQFIFNKDE
D*;
**SEQ ID NO: 5-** the amino acid sequence of *Sa-Sauri*Cas9 protein: 
MKRNYILGLDIGITSVGYGIIDYETRDVIDAGVRLFKEANVENNEGRRSKRGARRLKR
RRRHRIQRVKKLLFDYNLLTDHSELSGINPYEARVKGLSQKLSEEEFSAALLHLAKRRG
VHNVNEVEEDTGNELSTKEQISRNSKALEEKYVAELQLERLKKDGEVRGSINRFKTSD
YVKEAKQLLKVQKAYHQLDQSFIDTYIDLLETRRTYYEGPGEGSPFGWKDIKEWYEM
LMGHCTYFPEELRSVKYAYNADLYNALNDLNNLVITRDENEKLEYYEKFQIIENVFKQ
KKKPTLKQIAKEILVNEEDIKGYRVTSTGKPEFTNLKVYHDIKDITARKEIIENAELLDQI
AKILTIYQSSEDIQEELTNLNSELTQEEIEQISNLKGYTGTHNLSLKAINLILDELWHTND
NQIAIFNRLKLVPKKVDLSQQKEIPTTLVDDFILSPVVKRSFIQSIKVINAIIKKYGLPNDII
IELAREKNSKDAQKMINEMQKRNAATNERIEEIIRTTGKENAKYLIEKIKLHDMQEGKC
LYSLEAIPLEDLLNNPFNYEVDHIIPRSVSFDNSFNNKVLVKQEENSKKGNRTPFQYLSS
SDSKISYETFKKHILNLAKGKGRISKTKKEYLLEERDINRFSVQKDFINRNLVDTRYATA
ALMNLLRSYFRVNNLDVKVKSINGGFTSFLRRKWKFKKERNKGYKHHAEDALIIANA
DFIFKEWKKLDKAKKVMENQMFEEKQAESMPEIETEQEYKEIFITPHQIKHIKDFKDY
KYSHRVDKKPNRELINDTLYSTRKDDKGNTLIVNNLNGLYDKDNDKLKKLINKSPEKL
LMYHHDPQTYQKLKLIMEQYGDEKNPLYKYYEETGNYLTKYSKKDNGPVIKKIKYYG
NKLNAHLDITDDYPNSRNKVVKLSLKSFRFDIYKCEQGYKMVSIGYLDVLKKDNYYY
 
IPKDKYEAEKQKKKIKESDLFVGSFYYNDLIMYEDELFRVIGVNSDINNLVELNMVDIT
YKDFCEVNNVTGEKRIKKTIGKRVVLIEKYTTDILGNLYKTPLPKKPQLIFKRGEL*;
**SEQ ID NO: 6** - the amino acid sequence of *Sa-Sep*Cas9 protein: 
MKRNYILGLDIGITSVGYGIIDYETRDVIDAGVRLFKEANVENNEGRRSKRGARRLKR
RRRHRIQRVKKLLFDYNLLTDHSELSGINPYEARVKGLSQKLSEEEFSAALLHLAKRRG
VHNVNEVEEDTGNELSTKEQISRNSKALEEKYVAELQLERLKKDGEVRGSINRFKTSD
YVKEAKQLLKVQKAYHQLDQSFIDTYIDLLETRRTYYEGPGEGSPFGWKDIKEWYEM
LMGHCTYFPEELRSVKYAYNADLYNALNDLNNLVITRDENEKLEYYEKFQIIENVFKQ
KKKPTLKQIAKEILVNEEDIKGYRVTSTGKPEFTNLKVYHDIKDITARKEIIENAELLDQI
AKILTIYQSSEDIQEELTNLNSELTQEEIEQISNLKGYTGTHNLSLKAINLILDELWHTND
NQIAIFNRLKLVPKKVDLSQQKEIPTTLVDDFILSPVVKRSFIQSIKVINAIIKKYGLPNDII
IELAREKNSKDAQKMINEMQKRNAATNERIEEIIRTTGKENAKYLIEKIKLHDMQEGKC
LYSLEAIPLEDLLNNPFNYEVDHIIPRSVSFDNSFNNKVLVKQEENSKKGNRTPFQYLSS
SDSKISYETFKKHILNLAKGKGRISKTKKEYLLEERDINRFSVQKDFINRNLVDTRYATA
ALMNLLRSYFRVNNLDVKVKSINGGFTSFLRRKWKFKKERNKGYKHHAEDALIIANA
DFIFKEWKKLDKAKKVMENQMFEEKQAESMPEIETEQEYKEIFITPHQIKHIKDFKDY
KYSHRVDKKPNRELINDTLYSTRKDDKGNTLIVNNLNGLYDKDNDKLKKLINKSPEKL
LMYHHDPQTYQKLKLIMEQYGDEKNPLYKYYEETGNYLTKYSKKDNGPVIKKIKYYG
NKLNAHLDITDDYPNSRNKVVKLSLKNYRFDVYLTEKGYKFVTIAYLNVFKKDNYYY
IPKDMYQELKAKKKIKDTDQFIASFYKNDLIKLNGDLYKIIGVNSDDRNIIELDYYDIKY
KDYCEINNIKGEPRIKKTIGKKTESIEKLTTDVLGNLYLHSTEKAPQLIFKRGL*;
**SEQ ID NO: 7**- the amino acid sequence of *Sa-Seq*Cas9 protein: 
MKRNYILGLDIGITSVGYGIIDYETRDVIDAGVRLFKEANVENNEGRRSKRGARRLKR
RRRHRIQRVKKLLFDYNLLTDHSELSGINPYEARVKGLSQKLSEEEFSAALLHLAKRRG
VHNVNEVEEDTGNELSTKEQISRNSKALEEKYVAELQLERLKKDGEVRGSINRFKTSD
YVKEAKQLLKVQKAYHQLDQSFIDTYIDLLETRRTYYEGPGEGSPFGWKDIKEWYEM
LMGHCTYFPEELRSVKYAYNADLYNALNDLNNLVITRDENEKLEYYEKFQIIENVFKQ
KKKPTLKQIAKEILVNEEDIKGYRVTSTGKPEFTNLKVYHDIKDITARKEIIENAELLDQI
AKILTIYQSSEDIQEELTNLNSELTQEEIEQISNLKGYTGTHNLSLKAINLILDELWHTND
NQIAIFNRLKLVPKKVDLSQQKEIPTTLVDDFILSPVVKRSFIQSIKVINAIIKKYGLPNDII
 
IELAREKNSKDAQKMINEMQKRNAATNERIEEIIRTTGKENAKYLIEKIKLHDMQEGKC
LYSLEAIPLEDLLNNPFNYEVDHIIPRSVSFDNSFNNKVLVKQEENSKKGNRTPFQYLSS
SDSKISYETFKKHILNLAKGKGRISKTKKEYLLEERDINRFSVQKDFINRNLVDTRYATA
ALMNLLRSYFRVNNLDVKVKSINGGFTSFLRRKWKFKKERNKGYKHHAEDALIIANA
DFIFKEWKKLDKAKKVMENQMFEEKQAESMPEIETEQEYKEIFITPHQIKHIKDFKDY
KYSHRVDKKPNRELINDTLYSTRKDDKGNTLIVNNLNGLYDKDNDKLKKLINKSPEKL
LMYHHDPQTYQKLKLIMEQYGDEKNPLYKYYEETGNYLTKYSKKDNGPVIKKIKYYG
NKLNAHLDITDDYPNSRNKVVKLSLKPYRFDVYLDNGVYKFVTVKNLNVIKKENYYE
VNSKCYEKAKKLKKISDQAEFIASFYNNDLIKIDGELYRVIGVNTDLINRIEVNMVDITY
REYLENMNDKRSPRIFKTIASKTQSIKKYSTDILGTLYEVNSKKHPQMIMKG*;
**SEQ ID NO: 8** - the amino acid sequence of *Sa-Sha*Cas9 protein: 
MKRNYILGLDIGITSVGYGIIDYETRDVIDAGVRLFKEANVENNEGRRSKRGARRLKR
RRRHRIQRVKKLLFDYNLLTDHSELSGINPYEARVKGLSQKLSEEEFSAALLHLAKRRG
VHNVNEVEEDTGNELSTKEQISRNSKALEEKYVAELQLERLKKDGEVRGSINRFKTSD
YVKEAKQLLKVQKAYHQLDQSFIDTYIDLLETRRTYYEGPGEGSPFGWKDIKEWYEM
LMGHCTYFPEELRSVKYAYNADLYNALNDLNNLVITRDENEKLEYYEKFQIIENVFKQ
KKKPTLKQIAKEILVNEEDIKGYRVTSTGKPEFTNLKVYHDIKDITARKEIIENAELLDQI
AKILTIYQSSEDIQEELTNLNSELTQEEIEQISNLKGYTGTHNLSLKAINLILDELWHTND
NQIAIFNRLKLVPKKVDLSQQKEIPTTLVDDFILSPVVKRSFIQSIKVINAIIKKYGLPNDII
IELAREKNSKDAQKMINEMQKRNAATNERIEEIIRTTGKENAKYLIEKIKLHDMQEGKC
LYSLEAIPLEDLLNNPFNYEVDHIIPRSVSFDNSFNNKVLVKQEENSKKGNRTPFQYLSS
SDSKISYETFKKHILNLAKGKGRISKTKKEYLLEERDINRFSVQKDFINRNLVDTRYATA
ALMNLLRSYFRVNNLDVKVKSINGGFTSFLRRKWKFKKERNKGYKHHAEDALIIANA
DFIFKEWKKLDKAKKVMENQMFEEKQAESMPEIETEQEYKEIFITPHQIKHIKDFKDY
KYSHRVDKKPNRELINDTLYSTRKDDKGNTLIVNNLNGLYDKDNDKLKKLINKSPEKL
LMYHHDPQTYQKLKLIMEQYGDEKNPLYKYYEETGNYLTKYSKKDNGPVIKKIKYYG
NKLNAHLDITDDYPNSRNKVVKLSLKSYRFDVYLTDKGYKFVSITYLDVLKKENYYYI
SEAKYDKLKLNKGIDDKAKFIGSFYYNDLIELDGEVYTVIGVNNDKNNVIELNLPEIRY
KEYCEINNIKGSGRLRITIGKKVNSIRKLSTDVLGNRYYQSFAKKPQLVFKKGI*;
**SEQ ID NO: 9-** the amino acid sequence of *Sa-Slug*Cas9 protein: 
MKRNYILGLDIGITSVGYGIIDYETRDVIDAGVRLFKEANVENNEGRRSKRGARRLKR
RRRHRIQRVKKLLFDYNLLTDHSELSGINPYEARVKGLSQKLSEEEFSAALLHLAKRRG
VHNVNEVEEDTGNELSTKEQISRNSKALEEKYVAELQLERLKKDGEVRGSINRFKTSD
YVKEAKQLLKVQKAYHQLDQSFIDTYIDLLETRRTYYEGPGEGSPFGWKDIKEWYEM
LMGHCTYFPEELRSVKYAYNADLYNALNDLNNLVITRDENEKLEYYEKFQIIENVFKQ
KKKPTLKQIAKEILVNEEDIKGYRVTSTGKPEFTNLKVYHDIKDITARKEIIENAELLDQI
AKILTIYQSSEDIQEELTNLNSELTQEEIEQISNLKGYTGTHNLSLKAINLILDELWHTND
NQIAIFNRLKLVPKKVDLSQQKEIPTTLVDDFILSPVVKRSFIQSIKVINAIIKKYGLPNDII
IELAREKNSKDAQKMINEMQKRNAATNERIEEIIRTTGKENAKYLIEKIKLHDMQEGKC
LYSLEAIPLEDLLNNPFNYEVDHIIPRSVSFDNSFNNKVLVKQEENSKKGNRTPFQYLSS
SDSKISYETFKKHILNLAKGKGRISKTKKEYLLEERDINRFSVQKDFINRNLVDTRYATA
ALMNLLRSYFRVNNLDVKVKSINGGFTSFLRRKWKFKKERNKGYKHHAEDALIIANA
DFIFKEWKKLDKAKKVMENQMFEEKQAESMPEIETEQEYKEIFITPHQIKHIKDFKDY
KYSHRVDKKPNRELINDTLYSTRKDDKGNTLIVNNLNGLYDKDNDKLKKLINKSPEKL
LMYHHDPQTYQKLKLIMEQYGDEKNPLYKYYEETGNYLTKYSKKDNGPVIKKIKYYG
NKLNAHLDITDDYPNSRNKVVKLSLKPYRFDVYLTDKGYKFITISYLDVLKKDNYYYI
PEQKYDKLKLGKAIDKNAKFIASFYKNDLIKLDGEIYKIIGVNSDTRNMIELDLPDIRYK
EYCELNNIKGEPRIKKTIGKKVNSIEKLTTDVLGNVFTNTQYTKPQLLFKRGN*;
**SEQ ID NO: 10-** the amino acid sequence of *Sa-Slut*Cas9 protein: 
MKRNYILGLDIGITSVGYGIIDYETRDVIDAGVRLFKEANVENNEGRRSKRGARRLKR
RRRHRIQRVKKLLFDYNLLTDHSELSGINPYEARVKGLSQKLSEEEFSAALLHLAKRRG
VHNVNEVEEDTGNELSTKEQISRNSKALEEKYVAELQLERLKKDGEVRGSINRFKTSD
YVKEAKQLLKVQKAYHQLDQSFIDTYIDLLETRRTYYEGPGEGSPFGWKDIKEWYEM
LMGHCTYFPEELRSVKYAYNADLYNALNDLNNLVITRDENEKLEYYEKFQIIENVFKQ
KKKPTLKQIAKEILVNEEDIKGYRVTSTGKPEFTNLKVYHDIKDITARKEIIENAELLDQI
AKILTIYQSSEDIQEELTNLNSELTQEEIEQISNLKGYTGTHNLSLKAINLILDELWHTND
NQIAIFNRLKLVPKKVDLSQQKEIPTTLVDDFILSPVVKRSFIQSIKVINAIIKKYGLPNDII
IELAREKNSKDAQKMINEMQKRNAATNERIEEIIRTTGKENAKYLIEKIKLHDMQEGKC
LYSLEAIPLEDLLNNPFNYEVDHIIPRSVSFDNSFNNKVLVKQEENSKKGNRTPFQYLSS
SDSKISYETFKKHILNLAKGKGRISKTKKEYLLEERDINRFSVQKDFINRNLVDTRYATA
 
ALMNLLRSYFRVNNLDVKVKSINGGFTSFLRRKWKFKKERNKGYKHHAEDALIIANA
DFIFKEWKKLDKAKKVMENQMFEEKQAESMPEIETEQEYKEIFITPHQIKHIKDFKDY
KYSHRVDKKPNRELINDTLYSTRKDDKGNTLIVNNLNGLYDKDNDKLKKLINKSPEKL
LMYHHDPQTYQKLKLIMEQYGDEKNPLYKYYEETGNYLTKYSKKDNGPVIKKIKYYG
NKLNAHLDITDDYPNSRNKVVKLSLKSFRFDIYHTDKGYKMVPITYLDVQKKEKYYY
IPTEKYEALKQEKGINQNAQFIGSFYYNDLIEFDGELYRVIGINNGDKNLVELDMVDIRY
KEYCELNSITTTPRIVKTISPKTQSIEKYTTDILGNLYKAQPGKKPQFIFNKDED*;
**SEQ ID NO: 11** - nucleotide sequence of sgRNA: 
NNNNNNNNNNNNNNNNNNNNGUUUUAGUACUCUGGAAACAGAAUCUACUAAAA
CAAGGCAAAAUGCCGUGUUUAUCUCGUCAACUUGUUGGCGAGAUUUUU;
**SEQ ID NO: 58** - the amino acid sequence of *SlugCas9-HF* protein:

MNQKFILGLDIGITSVGYGLIDYETKNIIDAGVRLFPEANVENNEGRRSKRGSRRLKRR
RIHRLERVKKLLEDYNLLDQSQIPQSTNPYAIRVKGLSEALSKDELVIALLHIAKRRGIH
KIDVIDSNDDVGNELSTKEQLNKNSKLLKDKFVCQIQLERMNEGQVRGEKNRFKTADI
IKEIIQLLNVQKNFHQLDENFINKYIELVEMRREYFEGPGKGSPYGWEGDPKAWYETL
MGHCTYFPDELASVKYAYSADLFNALNDLNNLVIQRDGLSKLEYHEKYHIIENVFKQK
KKPTLKQIANEINVNPEDIKGYRITKSGKPQFTEFKLYHDLKSVLFDQSILENEDVLDQI
AEILTIYQDKDSIKSKLTELDILLNEEDKENIAQLTGYTGTHRLSLKCIRLVLEEQWYSSR
AQMEIFAHLNIKPKKINLTAANKIPKAMIDEFILSPVVKRTFGQAINLINKIIEKYGVPEDI
IIELARENNSKDKQKFINEMQKKNENTRKRINEIIGKYGNQNAKRLVEKIRLHDEQEGK
CLYSLESIPLEDLLNNPNHYEVDHIIPRSVSFDNSYHNKVLVKQSENSKKSNLTPYQYFN
SGKSKLSYNQFKQHILNLSKSQDRISKKKKEYLLEERDINKFEVQKEFINRNLVDTRYAT
AELTNYLKAYFSANNMNVKVKTINGSFTDYLRKVWKFKKERNHGYKHHAEDALIIAN
ADFLFKENKKLKAVNSVLEKPEIESKQLDIQVDSEDNYSEMFIIPKQVQDIKDFRNFKY
SHRVDKKPNRQLINDTLYSTRKKDNSTYIVQTIKDIYAKDNTTLKKQFDKSPEKFLMYQ
HDPRTFEKLEVIMKQYANEKNPLAKYHEETGEYLTKYSKKNNGPIVKSLKYIGNKLGS
HLDVTHQFKSSTKKLVKLSIKPYRFDVYLTDKGYKFITISYLDVLKKDNYYYIPEQKYD
KLKLGKAIDKNAKFIASFYKNDLIKLDGEIYKIIGVNSDTRNMIELDLPDIRYKEYCELN
NIKGEPRIKKTIGKKVNSIEKLTTDVLGNVFTNTQYTKPQLLFKRGN.

As described above, the present inventors have discovered a variety of Cas9 proteins that can be complexed with a single-stranded guide RNA (sgRNA). For the *Sauri*Cas9 protein, the complex thereof formed with sgRNA is referred to as CRISPR/*Sauri*Cas9 gene editing system in the present application (that is, a system in which the *SauriCas9* protein and the single-stranded guide RNA (sgRNA) work together to achieve gene editing). The complexes formed by other Cas9 proteins and sgRNA can be named in a similar way, such as the CRISPR/*Sha*Cas9 gene editing system, the CRISPR/*Slug*Cas9 gene editing system, and so on.

All the Cas9 proteins of the present disclosure are very small, with only less than one thousand and one hundred amino acids. Particularly, *SauriCas9* protein has 1061 amino acids; *ShaCas9* protein, *Sa-Sep*Cas9 protein, *Sa-Sha*Cas9 protein and *Sa-Slug*Cas9 protein have 1055 amino acids; *Sa-Seq*Cas9 protein has 1053 amino acids; *Slug*Cas9 protein, *SlugCas9-HF* protein and *Slut*Cas9 protein all have 1054 amino acids; and *Sa-Sauri*Cas9 and *Sa-Slut*Cas9 proteins have 1056 amino acids.

In one embodiment, the Cas9 protein of the present disclosure has an amino acid sequence which is at least 80%, 81%, 82%, 83%, 84%, 85%, 86%, 87%, 88%, 89%, 90%, 91%, 92%, 93%, 94%, 95%, 96%, 97%, 98%, 99%, 100%, or any percentage in the range of 80%-100% identical to the amino acid sequences represented by any one of SEQ ID NOs: 1-10 and SEQ ID NO: 58.

In one embodiment, the cell comprises eukaryotic cells and prokaryotic cells. The eukaryotic cells comprise, for example, mammalian cells and plant cells. The mammalian cells comprise, for example, Chinese hamster ovary cells, baby hamster kidney cells, mouse Sertoli cells, mouse breast tumor cells, buffalo rat hepatic cells, rat hepatoma cells, SV40-transformed monkey kidney CVI lines, monkey kidney cells, canine kidney cells, human cervical cancer cells, human lung cells, human liver cells, HIH/3T3 cells, human U2-OS osteosarcoma cells, human A549 cells, human K562 cells, human HEK293 cells, human HEK293T cells, human HCT116 cells, or human MCF-7 cells or TRI cells, but are not limited to these.

In one embodiment, the CRISPR/Cas9 system comprises *Staphylococcus auricularis*Cas9 (*Sauri*Cas9) protein, which has an amino acid sequence represented by SEQ ID NO: 1, and works with a single-stranded guide RNA (sgRNA) to achieve gene editing.

In one embodiment, the *SauriCas9* protein is derived from *Staphylococcus auricularis*, and has a UniProt accession number of A0A2T4M4R5.

In one embodiment, the *Sauri*Cas9 protein comprises a *Sauri*Cas9 protein that has no cleavage activity, or only has single-stranded cleavage activity, or has double-stranded cleavage activity.

In one embodiment, the CRISPR/Cas9 system comprises a *Staphylococcus haemolyticus* Cas9 (*Sha*Cas9) protein, which has an amino acid sequence represented by SEQ ID NO: 2, and works with single-stranded guide RNA (sgRNA) to achieve gene editing.

In one embodiment, the ShaCas9 protein is derived from *Staphylococcus haemolyticus,* and has a UniProt accession number of A0A2T4SLN6.

In one embodiment, the *Sha*Cas9 protein comprises a *Sha*Cas9 protein that has no cleavage activity, or only has single-stranded cleavage activity, or has double-stranded cleavage activity.

In one embodiment, the CRISPR/Cas9 system comprises a *Staphylococcus lugdunensis* Cas9 (*Slug*Cas9) protein, which has an amino acid sequence represented by SEQ ID NO: 3, and works with a single-stranded guide RNA (sgRNA) to achieve gene editing.

In one embodiment, the *Slug*Cas9 protein is derived from *Staphylococcus lugdunensis,* and has a UniProt accession number of A0A133QCR3.

In one embodiment, the *Slug*Cas9 protein comprises a *Slug*Cas9 protein that has no cleavage activity, or only has single-stranded cleavage activity, or has double-stranded cleavage activity.

In one embodiment, the CRISPR/Cas9 system comprises a *Staphylococcus lutrae* Cas9 (SlutCas9) protein, which has an amino acid sequence represented by SEQ ID NO: 4, and works with a single-stranded guide RNA (sgRNA) to achieve gene editing.

In one embodiment, the *SlutCas9* protein is derived from *Staphylococcus lutrae*, and has a UniProt accession number of A0A1W6BMI2.

In one embodiment, the *Slut*Cas9 protein comprises a *SlutCas9* protein that has no cleavage activity, or only has single-stranded cleavage activity, or has double-stranded cleavage activity.

In one embodiment, the CRISPR/Cas9 system comprises a *Sa-Sauri*Cas9 protein, which is a fusion protein obtained by replacing the PI domain of SaCas9 with the PI domain of *SauriCas9,* wherein *SauriCas9* is *Staphylococcus auricularis* Cas9. The *Sa-Sauri*Cas9 protein has an amino acid sequence represented by SEQ ID NO: 5. The *Sa-Sauri*Cas9 fusion protein works with a single-stranded guide RNA (sgRNA) to achieve gene editing.

In one embodiment, the *SauriCas9* protein is derived from *Staphylococcus auricularis*, and has a UniProt accession number of A0A2T4M4R5.

In one embodiment, the *Sa-Sauri*Cas9 protein comprises a *Sa-Sauri*Cas9 protein that has no cleavage activity, or only has single-stranded cleavage activity, or has double-stranded cleavage activity.

In one embodiment, the CRISPR/Cas9 system comprises a *Sa-Sep*Cas9 protein, which is a fusion protein obtained by replacing the PI domain of *Sa*Cas9 with the PI domain of *Sep*Cas9, wherein *Sep*Cas9 is *Staphylococcus epidermidis* Cas9. The *Sa-Sep*Cas9 protein has an amino acid sequence represented by SEQ ID NO: 6. The *Sa-Sep*Cas9 fusion protein works with a single-stranded guide RNA (sgRNA) to achieve gene editing.

In one embodiment, the *Sep*Cas9 protein is derived from *Staphylococcus epidermidis,* and has a UniProt accession number of A0A1Q9MLU4 and a NCBI accession number of WP_075777761.1.

In one embodiment, the *Sa-Sep*Cas9 protein comprises a *Sa-Sep*Cas9 protein that has no cleavage activity, or only has single-stranded cleavage activity, or has double-stranded cleavage activity.

In one embodiment, the CRISPR/Cas9 system comprises a *Sa-Seq*Cas9 protein, which is a fusion protein obtained by replacing the PI domain of *Sa*Cas9 with the PI domain of SeqCas9, wherein SeqCas9 is *Staphylococcus equorum* Cas9. The *Sa-Seq*Cas9 protein has an amino acid sequence represented by SEQ ID NO: 7. The *Sa-Seq*Cas9 fusion protein works with a single-stranded guide RNA (sgRNA) to achieve gene editing.

In one embodiment, the SeqCas9 protein is derived from *Staphylococcus equorum,* and has a UniProt accession number of A0A1E5TL62.

In one embodiment, the *Sa-Seq*Cas9 protein comprises a *Sa-Seq*Cas9 protein that has no cleavage activity, or only has single-stranded cleavage activity, or has double-stranded cleavage activity.

In one embodiment, the CRISPR/Cas9 system comprises a *Sa-Sha*Cas9 protein, which is a fusion protein obtained by replacing the PI domain of *Sa*Cas9 with the PI domain of *ShaCas9,* wherein *ShaCas9* is *Staphylococcus haemolyticus* Cas9. The *Sa-Sha*Cas9 protein has an amino acid sequence represented by SEQ ID NO: 8. The *Sa-Sha*Cas9 fusion protein works with a single-stranded guide RNA (sgRNA) to achieve gene editing.

In one embodiment, the *Sha*Cas9 protein is derived from *Staphylococcus haemolyticus,* and has a UniProt accession number of A0A2T4SLN6.

In one embodiment, the *Sa-Sha*Cas9 protein comprises a *Sa-Sha*Cas9 protein that has no cleavage activity, or only has single-stranded cleavage activity, or has double-stranded cleavage activity.

In one embodiment, the CRISPR/Cas9 system comprises a *Sa-Slug*Cas9 protein, which is a fusion protein obtained by replacing the PI domain of *Sa*Cas9 with the PI domain of *Slug*Cas9*,* wherein *Slug*Cas9 is *Staphylococcus lugdunensis* Cas9. The *Sa-Slug*Cas9 protein has an amino acid sequence represented by SEQ ID NO: 9. The *Slug*Cas9 fusion protein works with a single-stranded guide RNA (sgRNA) to achieve gene editing.

In one embodiment, the *Slug*Cas9 protein is derived from *Staphylococcus lugdunensis,* and has a UniProt accession number of A0A133QCR3.

In one embodiment, the *Sa-Slug*Cas9 protein comprises a *Sa-Slug*Cas9 protein that has no cleavage activity, or only has single-stranded cleavage activity, or has double-stranded cleavage activity.

In one embodiment, the CRISPR/Cas9 system comprises a *Sa-Slut*Cas9 protein, which is a fusion protein obtained by replacing the PI domain of *Sa*Cas9 with the PI domain of *Slut*Cas9, wherein SlutCas9 is *Staphylococcus lutrae* Cas9. The *Sa-Slut*Cas9 protein has an amino acid sequence represented by SEQ ID NO: 10. The *Sa-Slut*Cas9 fusion protein works with a single-stranded guide RNA (sgRNA) to achieve gene editing.

In one embodiment, the *SlutCas9* protein is derived from *Staphylococcus lutrae*, and has a UniProt accession number of A0A1W6BMI2.

In one embodiment, the *Sa-Slut*Cas9 protein comprises a *Sa-Slut*Cas9 protein that has no cleavage activity, or only has single-stranded cleavage activity, or has double-stranded cleavage activity.

In one embodiment, the CRISPR/Cas9 system comprises a *SlugCas9-HF* protein, which is an amino-acid-modified protein obtained by introducing R247A, N415A, T421A, and R656A mutations to *Slug*Cas9. The *SlugCas9-HF* is *Staphylococcus lugdunensis* Cas9-HiFi. *SlugCas9-HF* works with a single-stranded guide RNA (sgRNA) to achieve gene editing. The complex of *the SlugCas9-HF* protein and sgRNA has a low off-target rate and high specificity, and exhibits low tolerance for non-target DNA sequences, that is, the complex is essentially incapable of or is incapable of the cleave the non-targeted DNA sequences.

In one embodiment, the *Slug*Cas9 protein is derived from *Staphylococcus lugdunensis,* and has a UniProt accession number of A0A133QCR3.

In one embodiment, the *Slug*Ca*s9-HF* protein comprises a *SlugCas9-HF* protein that has no cleavage activity, or only has single-stranded cleavage activity, or has double-stranded cleavage activity.

In one embodiment, accurately locating the target DNA sequence comprises forming a complementary base pairing structure from a 20 bp or 21 bp sequence at the 5' end of the sgRNA and the target DNA sequence.

In one embodiment, accurately locating the target DNA sequence comprises recognizing, by the complex of the Cas9 protein and the sgRNA, a PAM sequence on the target DNA sequence.

In one embodiment, a 20 bp or 21 bp sequence at the 5' end of the sgRNA in the complex of the *SlugCas9-HF* protein and the sgRNA is capable of forming an imperfect complementary base pairing structure with a non-target DNA sequence. Particularly, in the present disclosure, the imperfect complementary base pairing structure comprises a part of complementary base pairing structure and a part of non-complementary base pairing structure. In a preferred embodiment, there are two or more base mismatches between the non-targeted DNA sequence and the sgRNA.

In yet another embodiment, the complex of the Cas9 protein and the sgRNA is capable of recognizing the PAM sequence on the non-target DNA sequence.

In one embodiment, the PAM sequence and the target DNA sequence are respectively as follows:
for the *Sauri*Cas9 protein, the PAM is NNGG, and the target DNA sequence is represented by SEQ ID NO: 12;
for the *ShaCas9* protein, the PAM is NNGRM, and the target DNA sequence is represented by SEQ ID NO: 13;
for the *Slug*Cas9 protein, the PAM is NNGG, and the target DNA sequence is represented by SEQ ID NO: 12;
for the *Slut*Cas9 protein, the PAM is NNGR, and the target DNA sequence is represented by SEQ ID NO: 14;
for the *Sa-Sauri*Cas9 protein, the PAM is NNGG, and the target DNA sequence is represented by SEQ ID NO: 12;
for the *Sa-Sep*Cas9 protein, the PAM is NNGG, and the target DNA sequence is represented by SEQ ID NO: 12;
for the *Sa-Seq*Cas9 protein, the PAM is NNGRM, and the target DNA sequence is represented by SEQ ID NO: 13;
for the *Sa-Sha*Cas9 protein, the PAM is NNGRM, and the target DNA sequence is represented by SEQ ID NO: 13;
for the *Sa-Slug*Cas9 protein, the PAM is NNGG, and the target DNA sequence is represented by SEQ ID NO: 12;
for the *Sa-Slut*Cas9 protein, the PAM is NNGRR, and the target DNA sequence is represented by SEQ ID NO: 15; and
for the *SlugCas9-HF* protein, the PAM is NNGG, and the target DNA sequence is represented by SEQ ID NO: 12.

The nucleotide sequences of SEQ ID NOs: 12-15 are as follows:
NNNNNNNNNNNNNNNNNNNNNNNGG (SEQ ID NO: 12);
NNNNNNNNNNNNNNNNNNNNNNNGRM (SEQ ID NO: 13);
NNNNNNNNNNNNNNNNNNNNNNNGR (SEQ ID NO: 14); and
NNNNNNNNNNNNNNNNNNNNNNNGRR (SEQ ID NO: 15).

Those skilled in the art can understand that the base N above represents any one of the four bases, A (adenine), T (thymine), C (cytosine) and G (guanine); the base M above represents any one of the two bases, A and C; and the base R above represents any one of the two bases, A and G.

In one embodiment, the complex of the Cas9 protein and the sgRNA is capable of accurately locating the target DNA sequence, which means that the complex of the Cas9 protein and the sgRNA is capable of recognizing and binding to the target DNA sequence, or that the complex of the Cas9 protein and the sgRNA is capable of carrying a further protein fused with the Cas9 protein or a protein that specifically recognizes the sgRNA to the place where the target DNA sequence is located.

In one embodiment, the complex of the Cas9 protein and the sgRNA, or the further protein fused with the Cas9 protein, or the protein that specifically recognizes the sgRNA is capable of making modification and regulation to the target DNA region, and the modification and regulation comprises, but is not limited to, regulation of gene transcription level, DNA methylation regulation, DNA acetylation modification, histone acetylation modification, single-base editing, or chromatin imaging tracking.

In one embodiment, the complex of the *SlugCas9-HF* protein and the sgRNA has low tolerance for the non-target DNA sequence. That is, the complex of the *SlugCas9-HF* protein and the sgRNA is essentially incapable of or is incapable of recognizing and binding to the non-target DNA sequence, or the complex of the *SlugCas9-HF* protein and the sgRNA is essentially incapable of or is incapable of carrying the further protein fused with the *SlugCas9-HF* protein or a protein that specifically recognizes the sgRNA to the place where the non-target DNA sequence is located.

In the context of the present disclosure, the term "essentially" in the expression "the complex of the *SlugCas9-HF* protein and the sgRNA is essentially incapable of recognizing and binding to the non-targeted DNA sequences" means that there is little or no biological and/or statistical significance for the extent of recognition and binding, if any, of the complex of the SlugCas9-HF protein and the sgRNA to the non-targeted DNA sequences.

In yet another embodiment, the complex of the *SlugCas9-HF* protein and the sgRNA, or the other protein fused with the *SlugCas9-HF* protein, or the protein that specifically recognizes the sgRNA is essentially incapable of or is incapable of making modification and regulation to the non-targeted DNA region, and the modification and regulation comprises, but is not limited to, regulation of gene transcription level, DNA methylation regulation, DNA acetylation modification, histone acetylation modification, single-base editing, or chromatin imaging tracking.

Similarly, in the context of the present disclosure, the term "essentially" in the expression "the complex of the *SlugCas9-HF* protein and the sgRNA, or the other protein fused with the *SlugCas9-HF* protein, or the protein that specifically recognizes the sgRNA is essentially incapable of or is incapable of making modification and regulation to the non-targeted DNA region" means that there is little or no biological and/or statistical significance for the extent of modification and regulation, if any, of the complex of the *SlugCas9-HF* protein and sgRNA, or the other protein fused with the *SlugCas9-HF* protein, or the protein that specifically recognizes the sgRNA to the non-targeted DNA region.

In one embodiment, the single-base editing comprises, but is not limited to, conversion of adenine to guanine, or conversion of cytosine to thymine, or conversion of cytosine to uracil, or conversion between other bases.

The CRISPR/Cas9 gene editing system of the present disclosure has high editing activity and high specificity, and shows significant advantages compared with the existing CRISPR/Cas9 gene editing systems.

In the present disclosure, the editing efficiency and off-target rate of the CRISPR/Cas9 system are detected by technologies such as gene synthesis, molecular cloning, cell transfection, PCR product deep sequencing, flow cytometry analysis technology, bioinformatics analysis and the like.

The CRISPR/Cas9 gene editing system of the present disclosure is verified in a GFP-reporter cell line containing target sites, and it is found that the gene editing system can edit target genes with high specificity and a low off-target rate.

Thus, in the second aspect, the present disclosure provides a method for gene editing in cells with the CRISPR/Cas9 system for gene editing according to the first aspect of the present disclosure, wherein the method edits a target DNA sequence by recognizing and locating the target DNA sequence with the complex of a Cas9 protein and a sgRNA, and the method comprises the steps of :
(1) synthesizing a Cas9 gene sequence and cloning it into an expression vector such as pAAV2_ITR, to obtain an expression vector cloned with the Cas9 gene sequence, such as pAAV2_Cas9_ITR, wherein the Cas9 gene sequence:
   (a) has a nucleotide sequence encoding an amino acid sequence represented by any one of SEQ ID NOs: 1-10 and SEQ ID NO: 58;
   (b) has a nucleotide sequence encoding an amino acid sequence that is at least 80% identical to the amino acid sequence represented by any one of SEQ ID NOs: 1-10 and SEQ ID NO: 58; or
   (c) is a humanized Cas9 gene sequence, for example, the one having a nucleotide sequence represented by any one of SEQ ID NOs: 23-32 and SEQ ID NO: 112;
(2) synthesizing oligo single-stranded DNAs corresponding to the sgRNA, *i.e.*, an oligo forward-strand sequence (Oligo-F) and an oligo reverse-strand sequence (Oligo-R), and annealing and ligating the oligo forward-strand sequence and the oligo reverse-strand sequence to a restriction site, such as the BsaI digestion site of plasmid pAAV2_Cas9_U6_BsaI, of the expression vector cloned with the Cas9 gene sequence to obtain an expression vector, such as pAAV2_Cas9-hU6-sgRNA, for expressing the Cas9 protein and the sgRNA; wherein the sgRNA has a nucleotide sequence represented by SEQ ID NO: 11, or a nucleotide sequence that is at least 80% identical to the nucleotide sequence represented by SEQ ID NO: 11, or a modification comprising, for example, phosphorylation, truncation, addition, sulfurization, methylation or hydroxylation, based on the nucleotide sequence represented by SEQ ID NO: 11;
(3) delivering the expression vector expressing the Cas9 protein and the sgRNA into cells comprising a target site to edit the target site.

SEQ ID NOs: 23-32 and SEQ ID NO: 112 are as follows.
**SEQ ID NO: 23** - the humanized *SauriCas9* gene sequence: 
ATGCAGGAGAACCAGCAGAAGCAGAACTACATCCTGGGCCTGGACATCGGAATCA
CCAGCGTCGGCTACGGACTGATCGATAGCAAGACAAGAGAAGTGATCGACGCCGG
CGTTAGACTCTTTCCAGAAGCTGATAGCGAGAACAACTCCAACCGCAGAAGCAAG
CGGGGCGCCAGACGGTTAAAACGGAGAAGAATCCACCGGCTGAACCGGGTCAAAG
ACCTGCTCGCTGATTACCAGATGATCGATCTTAACAATGTTCCTAAGAGCACCGACC
CCTACACCATCAGAGTGAAGGGCCTCCGGGAGCCTCTGACAAAAGAAGAATTCGC
CATCGCCCTCCTGCATATCGCTAAGAGAAGAGGCCTGCACAACATCAGTGTGTCCAT
GGGCGACGAAGAGCAGGACAATGAACTGAGCACCAAGCAGCAGCTGCAAAAGAA
TGCCCAGCAACTGCAGGACAAGTATGTGTGCGAACTGCAGTTAGAACGGCTGACC
AACATCAACAAGGTCAGAGGCGAGAAGAACAGATTTAAGACAGAGGACTTTGTGA
AAGAAGTGAAACAGCTGTGCGAAACCCAGAGACAGTACCACAACATCGACGACCA
ATTCATCCAGCAGTACATCGACCTGGTGTCTACAAGACGGGAGTACTTCGAGGGCC
CCGGCAACGGCTCTCCATACGGCTGGGACGGCGACCTGCTGAAGTGGTACGAGAA
 
GCTGATGGGCAGATGCACCTATTTCCCCGAAGAACTGAGGTCCGTGAAGTACGCCT
ACAGCGCCGACCTCTTCAACGCCCTGAACGACCTGAACAACCTCGTTGTGACCAG
GGATGACAATCCAAAGCTTGAGTACTACGAGAAGTACCACATTATTGAGAACGTGT
TCAAGCAAAAGAAGAATCCCACACTCAAACAAATCGCCAAAGAGATCGGCGTGCA
AGATTACGACATCCGGGGCTATAGAATCACAAAGAGCGGCAAACCTCAGTTCACCT
CTTTTAAGCTGTATCACGACCTGAAGAACATCTTCGAGCAGGCCAAATACCTGGAA
GATGTGGAAATGCTGGACGAGATCGCCAAGATCCTGACCATCTACCAGGATGAGAT
TAGCATCAAGAAAGCCCTGGACCAGCTGCCCGAACTGCTGACAGAGAGCGAGAAA
TCTCAGATCGCACAGCTCACCGGCTATACAGGCACCCACAGACTGAGCCTGAAGTG
CATCCACATTGTGATCGACGAGCTGTGGGAGAGCCCCGAGAACCAGATGGAAATCT
TTACCAGACTGAATCTGAAACCTAAGAAGGTGGAAATGAGCGAGATCGACAGCATA
CCCACCACCCTGGTCGACGAGTTCATCCTCTCACCTGTGGTGAAGCGGGCCTTCAT
CCAGAGCATCAAGGTAATCAACGCAGTGATCAATCGGTTCGGCCTGCCAGAGGACA
TCATCATCGAGCTGGCCAGAGAAAAGAATAGCAAGGATCGGAGAAAGTTCATTAAC
AAGCTGCAGAAACAAAATGAGGCCACAAGAAAGAAAATCGAACAGCTGCTGGCC
AAGTACGGCAACACCAATGCCAAGTACATGATCGAGAAGATCAAGCTGCACGACAT
GCAGGAGGGCAAGTGCCTGTACAGCCTGGAGGCTATTCCTCTGGAAGACCTGCTGA
GCAACCCGACACACTACGAAGTTGACCACATTATCCCCAGATCTGTGAGCTTTGAC
AACAGCCTGAACAACAAAGTGCTGGTGAAACAAAGCGAAAACAGCAAGAAGGGC
AATCGCACCCCTTACCAGTACCTGAGCAGCAACGAGTCTAAGATTAGCTACAACCA
GTTTAAGCAGCACATCCTGAACCTGAGCAAGGCCAAGGACAGAATCAGCAAGAAA
AAAAGAGATATGCTGCTGGAAGAGAGAGATATCAACAAGTTCGAAGTGCAGAAGG
AATTCATTAACCGGAACCTGGTGGATACACGGTACGCCACCAGAGAACTGTCTAAC
CTGCTGAAGACCTACTTCAGCACCCATGACTACGCCGTGAAGGTGAAGACCATCAA
CGGCGGCTTCACTAACCACCTGAGGAAGGTGTGGATTTCAAGAAGCACAGAAAC
CACGGCTACAAGCACCACGCCGAAGATGCCCTGGTGATCGCCAACGCCGACTTCCT
GTTTAAGACACATAAGGCCCTGCGGAGAACCGATAAGATCCTGGAACAACCTGGCC
TGGAAGTGAATGATACAACCGTGAAAGTGGACACCGAGGAAAAATACCAGGAGCT
GTTCGAGACACCTAAGCAAGTGAAGAACATCAAGCAGTTCCGGGACTTCAAGTAC
AGCCACCGAGTGGACAAGAAGCCTAACCGGCAGCTTATCAACGACACACTGTACTC
 
CACCAGAGAGATTGATGGCGAAACCTACGTGGTGCAGACCCTTAAGGATCTGTACG
CCAAGGACAACGAGAAAGTGAAGAAGCTGTTCACCGAAAGACCTCAGAAGATCCT
GATGTACCAGCACGACCCTAAGACCTTCGAGAAACTGATGACAATCCTGAACCAGT
ACGCTGAGGCCAAGAACCCTCTGGCTGCTTATTACGAGGACAAAGGCGAGTACGTG
ACCAAGTACGCCAAGAAAGGCAATGGACCTGCCATCCACAAGATCAAGTATATCGA
TAAGAAGCTTGGATCTTACCTGGATGTTAGCAACAAGTATCCTGAGACACAGAACA
AGCTTGTGAAGCTGTCCCTGAAGAGCTTTAGATTCGACATCTACAAGTGTGAACAG
GGCTACAAGATGGTGTCCATCGGATACCTGGACGTGCTGAAGAAAGATAACTACTA
CTACATCCCTAAGGACAAGTACGAGGCCGAGAAGCAGAAAAAGAAGATCAAGGAA
TCTGATCTTTTTGTGGGCAGCTTCTACTACAACGACCTCATCATGTACGAGGATGAA
CTGTTCAGAGTGATAGGAGTGAACAGCGACATCAACAATCTGGTTGAGCTAAACAT
GGTCGACATTACCTACAAGGACTTCTGCGAGGTGAACAACGTGACAGGCGAGAAA
AGAATCAAAAAGACTATCGGCAAGCGCGTGGTCCTGATCGAGAAGTACACCACAG
ATATTCTAGGCAACCTGTACAAGACTCCCCTGCCTAAGAAGCCCCAGCTTATCTTCA
AGCGGGGAGAACTG;
**SEQ ID NO: 24** - the humanized *ShaCas9* gene sequence: 
ATGAAGACAGATTACATCCTGGGCCTGGATATCGGCATCACCAGCGTGGGCTATGGC
ATCATCAACTACAATGACAAGAGCATCATCGACGCCGGAGTGCGGCTGTTCCCCGA
GGCCAATGTTGAAAACAACGAGGGCAGAAGAAGCAAGAGAGGCGCCAGAAGGCT
GAAGAGACGGCGGATTCACCGGCTGGAAAGAGTGAAGCAGCTTCTGCTGGACTAC
AAGCTGCTGGACAGCATCGACGTGATCCCTCAGTCTACCAACCCCTACGAGATCCG
GGTGCGGGGCCTGAGAGAGAAGCTGACCAAAGACGAGCTGGTGATCGCGCTGCTC
CATTTGGCTAAGCGCCGTGGAATTCACAACATCGACGTGATCGACCAGGAGGAGGA
TGCCAGCAACGAGCTGAGTACAAAGGAGCAGCTGAGCAAGAACAATCTGCTGCTG
AGAGATAAGAGCATCTGTGAAGTCCTGCTGGAACGGTACAACGAGGGCAAGGTGC
GCGGAGAGAAAAACAGATTCAAAACATCTGATATCGTGAACGAGATCAGACAGATT
CTGGAGACACAGAAGGAAGTGCACCACCTGGACGATAGCTTCATCGACAAGTACAT
CGAACTGGTGGAAACCCGAAGAGAATATTACGAGGGCCCTGGCGAAGGAAGTCCT
TACGGCTGGGGAGCCGATCTGAAGAAGTGGTACGAGCACCTGATGGGCCGGTGCA
CCTACTTCCCTGAGGAACTGAGATCCGTGAAGTACGCCTACTCAGCCGACCTGTTC
 
AATGCCCTGAACGACCTGAACAACCTGGTTATCCAGAGAGACGGCCTGAACAAGC
TGGAATATCACGAGAAGTACCACATCATTGAAAACGTGTTCAAGCAGAAAAAGAA
ACCTACACTGAAGCAGATCAGCAACGAGATCGGCGTGAACCCTGAAGACATCAAG
GGCTACCGGATCACCAAATCTGGCAAGGAGAATTTCACCGAGTTCAAGTTGTACCA
CGATCTCAAGAAGATCCTGAAAGACCAGAGCATCCTAGAAAATATCCAGCTGCTGG
ACCAGATCGCCGAGATCATCACAATCTACCAGGACAAGGAGAGCATTAAAAAAGA
ACTGGAACAGCTCACCGAGCCTATAAATGAAATCGACAAGGAATCTATTTCTGACCT
GGCTGGATACAACGGCACCCACAGACTTTCTCTGAAGTGCATCAACCTGGTGCTGG
AAGAACTGTGGCACACCAGCAGAAATCAAATGGAAATCTTCGCCTACCTGAACATC
AAACCCAGAAAGATCGACCTGCAGAAAGCTAATAAAATCCCCAAGGACATGATCGA
CGAATTCATCCTGAGCCCTGTGGTGAAGCGAACATTCGGCCAGGCCATCAACGTGA
TTAACAAGGTGATTGAGAAGTACGGCGTTCCTAAGGACATCATCATCGAGCTCGCC
AGAGAGAACAACACCAAGGACAAGCAGAAATTCATTAACGAACTCCAGAAGAAA
AACGAGAAAACCAGACAGCGGATCAATGAGATCATCGGAAAGACCGGCAACCAGA
ACGCCAAGAGACTGGTGGAAAAGATCAGACTGCACGACGAGCAGGAGGGAAAAT
GCCTGTACAGCCTGGAGAGCATCCCACTGGAGGACCTCCTCAACAACCCCAACCAC
TACGAGGTGGACCACATCATACCTAGAAGCGTGTCTTTTGATAACAGTTATCAAAAC
AAGGTGCTCGTCAAGCAAACAGAAAACAGCAAGAAGGGGAATAGAACACCTTACC
AGTACCTGAACAGCGGCGAGGCTAAGCTGAGCTACAACCAGTTCAAGCAACACGT
GCTGAACCTGAGCAAGAGCAAGGATAGAATTTCCAAGAAGAAGAAAGAGTACCTC
CTGGAGGAACGGGACATTAACAAGTTCGAGGTGCAGAAGGAATTCATCAACAGAA
ACCTGGTCGACACCCGATATGCCACCCGCGAGCTGACCAATTACCTGAAGGCCTATT
TCAGCGCTAACGATATGGACGTGAAGGTCAAGACCATCAATGGAAGCTTTACCAAC
CACCTGCGGAAGGTTTGGGACTTCAACAAGGAAAGAAACCACGGCTACAAACACC
ACGCCGAGGATGCCCTGATCATCGCCAATGCCGACTTCCTGTTTAAGGAGAACAAG
AAGCTGAAGGCCGCCAACAAAGTGCTGGAACAGCCTGAGAGAAAGGAAATTGAA
ACGAAGATCGAGGTGAATTCCGATGAGAATTACCAAGATCTCTTTGTGATCCCCCAG
CAGGTGAAAGAAATCAAGGAGTTTAGAGATTTTAAGTACAGCCACAGAGTGGACA
AAAAACCTAATAGACAGCTGATCAACGATACACTGTACTCCACAAGAGTGAAGGAC
GACGACCTGTACATCATATCTCCTATCAAGAACATCTACAGCAAAGACAACACAGAT
 
CTGAAGAAGCACTTCAACAAAAACCCAGAGAAGTTCCTGATGTACCAGAAAGACC
CCAAGACCTTCGAGAAACTGGAAGTTATTATGAAGCAGTACGCCAACGAGAAGAAT
CCTCTGGCCAAATACCACGAGGAAACAGGCGAGTACCTGACGAAGTACAGCAAGA
CCAACAACGGGCCAATCGTGAAAAGCATTAAGATGTTCGGCAAGAAAGTGGGCAA
TCACCTGGATGTGACCAACCAGTACAACAATAGCCGGAACAAGCTGGTGAAGCTG
AGCTTCAAAAGCTACAGATTCGACGTGTACCTGACAGACAAGGGCTACAAGTTCGT
GTCCATCACCTACCTGGACGTGCTGAAGAAAGAGAATTACTACTACATCAGCGAGG
CCAAGTACGACAAACTGAAACTAAACAAGGGCATCGATGACAAGGCCAAGTTCAT
CGGCAGCTTCTACTACAACGACCTGATCGAGCTGGACGGCGAGGTGTACACCGTGA
TCGGCGTGAATAATGACAAGAACAACGTCATCGAGCTCAATCTCCCCGAAATTAGAT
ACAAGGAATACTGCGAGATTAACAACATCAAGGGCTCCGGCAGACTCCGGATCACC
ATCGGCAAGAAGGTGAACTCCATTCGGAAGCTGTCCACCGACGTGCTCGGCAACC
GGTACTACCAGTCTTTCGCCAAAAAGCCTCAGCTCGTGTTCAAGAAGGGAATA;
**SEQ ID NO: 25** - the humanized *SlugCas9* gene sequence: 
ATGAACCAAAAATTCATACTGGGACTGGACATCGGAATCACCAGCGTGGGCTACGG
CCTGATCGACTACGAGACAAAGAATATCATCGATGCCGGCGTTAGACTGTTCCCCGA
GGCCAACGTGGAAAACAACGAGGGAAGAAGGTCCAAACGTGGAAGCAGAAGACT
GAAGCGACGCCGCATTCACAGACTTGAACGGGTGAAGAAGCTGCTCGAGGATTATA
ATCTGCTGGATCAGTCCCAGATTCCTCAGTCTACAAACCCCTACGCCATCCGCGTGA
AGGGCCTGTCTGAAGCCCTGAGCAAGGACGAACTCGTGATTGCCCTGCTCCATATC
GCCAAGAGAAGAGGCATCCACAAGATCGACGTGATCGACAGCAACGACGACGTGG
GGAACGAGCTCAGCACCAAGGAACAGCTGAATAAGAACAGCAAGCTGCTGAAAG
ACAAATTTGTGTGCCAGATCCAGCTGGAAAGAATGAATGAGGGCCAGGTGCGGGG
AGAGAAAAACCGGTTCAAGACCGCTGATATCATCAAGGAAATCATCCAGCTGCTGA
ATGTGCAGAAGAACTTCCACCAGCTGGACGAGAACTTCATCAACAAGTACATCGAA
CTGGTTGAGATGAGACGGGAATACTTCGAGGGCCCCGGCAAGGGCAGTCCATATGG
CTGGGAAGGCGACCCTAAGGCTTGGTACGAGACACTGATGGGCCACTGCACCTACT
TCCCAGATGAGCTGAGAAGCGTGAAATACGCCTACAGTGCCGACCTGTTCAACGCT
CTGAACGACCTGAACAACCTGGTCATCCAAAGAGATGGACTGTCTAAGCTCGAGTA
TCATGAGAAGTATCACATCATCGAGAACGTGTTCAAGCAGAAGAAGAAACCTACAC
 
TGAAGCAGATCGCCAATGAGATCAATGTCAACCCTGAAGATATCAAGGGCTACAGA
ATCACAAAGTCTGGCAAGCCCAGTTTACCGAGTTTAAGCTCTACCACGACCTGAA
AAGCGTGCTGTTTGACCAGAGCATCCTGGAGAACGAAGACGTGCTGGACCAGATC
GCTGAGATCCTGACCATCTACCAGGACAAGGATAGCATCAAATCTAAGCTGACGGA
ACTGGACATCCTGCTGAACGAGGAAGATAAGGAAAACATCGCCCAGCTGACTGGCT
ACACCGGGACCCACCGGCTCAGCCTGAAATGCATCCGGCTGGTCCTGGAAGAGCA
GTGGTATTCTAGCCGGAATCAGATGGAAATCTTCACACACCTGAACATTAAGCCTAA
GAAGATCAACCTGACAGCCGCCAACAAGATCCCGAAGGCTATGATCGACGAGTTCA
TCCTGAGCCCTGTGGTGAAGAGGACCTTCGGCCAGGCCATTAACCTTATTAACAAG
ATCATAGAAAAGTACGGCGTGCCTGAAGATATCATCATCGAGCTGGCCAGAGAAAA
TAATAGCAAGGACAAGCAGAAGTTCATCAATGAGATGCAGAAAAAGAACGAGAAC
ACCAGAAAGAGAATTAACGAAATCATCGGCAAGTATGGCAACCAGAACGCCAAGA
GACTGGTCGAGAAGATTAGACTGCACGACGAGCAGGAGGGCAAGTGCCTGTACTC
ACTGGAAAGCATCCCTCTGGAGGACCTGCTGAACAACCCCAACCACTACGAGGTG
GACCACATCATTCCAAGATCTGTGTCCTTCGACAACTCTTACCACAACAAAGTGCTC
GTGAAGCAGAGCGAGAACTCCAAAAAATCCAACCTGACCCCTTACCAGTACTTTAA
CAGCGGCAAGTCCAAGCTCTCTTACAACCAGTTTAAACAACACATCCTGAACCTGA
GCAAGTCCCAGGATAGAATCAGCAAAAAAAAGAAAGAGTATCTGCTGGAAGAACG
GGACATCAACAAGTTCGAGGTGCAAAAAGAGTTCATCAATAGAAACCTGGTGGATA
CCCGGTACGCCACAAGAGAGCTGACAAACTACCTGAAGGCCTACTTCAGCGCCAA
CAATATGAACGTGAAGGTGAAAACGATCAACGGCAGCTTCACCGATTACCTGCGGA
AAGTGTGGAAGTTTAAGAAGGAACGGAACCACGGCTACAAGCACCACGCCGAGG
ACGCCCTGATTATCGCTAATGCCGATTTCCTGTTCAAAGAGAACAAGAAGCTGAAA
GCCGTGAACTCTGTGCTGGAAAAACCTGAGATCGAGAGCAAGCAGCTGGATATCCA
GGTGGATAGCGAGGATAACTACAGCGAAATGTTCATCATCCCTAAGCAGGTCCAGG
ACATCAAGGACTTCAGAAACTTCAAGTACAGCCACAGAGTGGACAAGAAGCCTAA
CAGACAGCTGATCAACGATACACTGTACAGCACCCGGAAGAAGGACAACTCCACCT
ACATCGTGCAGACCATCAAAGATATCTATGCCAAAGATAATACCACCCTGAAGAAGC
AGTTTGACAAGTCACCCGAGAAGTTCCTCATGTACCAACACGATCCGCGGACCTTC
GAGAAGTTGGAAGTGATCATGAAGCAGTACGCTAATGAGAAGAATCCTCTGGCCAA
 
GTACCACGAGGAAACAGGCGAGTACCTGACCAAATACAGCAAAAAAAACAACGGC
CCTATCGTGAAAAGCCTGAAGTACATTGGAAACAAGCTGGGCAGCCACCTAGATGT
GACCCACCAGTTCAAGAGCAGCACCAAGAAGTTGGTGAAGCTGAGCATCAAGCCT
TATAGATTCGACGTCTACCTGACCGACAAGGGATATAAGTTCATCACCATCAGCTAC
CTGGACGTGCTGAAGAAAGACAATTACTACTACATACCCGAACAGAAGTACGACAA
GCTCAAACTGGGCAAGGCCATCGACAAAAACGCCAAGTTTATCGCTAGCTTCTACA
AGAATGATCTGATCAAGCTGGACGGCGAGATCTACAAGATCATCGGCGTGAATAGC
GACACCAGAAACATGATCGAACTGGATCTGCCTGACATCAGATACAAAGAATACTG
CGAGCTGAACAATATCAAGGGCGAACCTAGAATCAAAAAGACCATCGGCAAAAAG
GTGAATAGCATCGAAAAACTGACAACCGACGTGCTGGGCAACGTGTTCACCAACA
CCCAGTACACAAAACCTCAGCTGCTGTTCAAGCGAGGAAAT;
**SEQ ID NO: 26** - the humanized *SlutCas9* gene sequence: 
ATGAGAAACAGCTACATCCTGGGCCTGGACATCGGAATCACCAGCGTGGGATATGG
CATCATCGACAGAGTCACCAGAGAGGTGATCGACGCCGGCGTGCGGCTTTTCCCCG
AGGCCAACGTGGAGAACAACGAGGGCAGACGGAGCAAGAGAGGAGCCCGGCGGC
TGAAAAGAAGGCGGATCCACCGGCTGAATAGAATCAAGCAGCTGCTGAAAAACGC
CGGCCTGCTGGAGGGAGATGTGCTGCCTAAGTCTACCAACCCCTATGAGATCAGAG
TGCGGGGCCTCCGAAGCCCTCTGACCAAAGATGAACTGGTGATCGCCCTGCTCCAC
ATCGCCAAAAGAAGAGGCATCCACAACATCAACATCGTGGAGATGACGAAGAAA
CGGACAGCACACTGAGTACCACAGCCCAGCTGAAGAAGAACGAGAAGGCTCTCAA
GGGACAGTTTGTTTGTGAACTGCAACTGGACAGACTGGCTAATGCCCACCAAGTGC
GGGGCGAGAAAAATCGATTTAAGACAGAGGACATTGTGAAGGAAGTCAGAGCCCT
GCTTCAGCAACAGCAGAACTTCCACAACATCGATAATTCTTTCGTGGAACAGTACAT
TGCCCTGCTGGAGAGCCGGAGGACCTACTACGAGGGCCCTGGCGAAGGCTCTCCTT
ACGGCTGGGACGGCGACATTAAGAAGTGGTACGAGATGCTGATGGGCTATTGCACC
TACTTCCCTGAAGAGCTGAGAAGCGTGAAGTACGCCTACACCGCCGATCTGTTCAA
CGCCCTGAATGACCTGAACAACCTGGTGATCACCCGGGACGACAACAGCAAATTG
ACATACGCCGAGAAGTACCATATCATCGAGAACGTGTTCAAACAGAAGAAAGTACC
TACACTGAAGCAGATCGCCAAGGAAATCGGAGTGAACGAGAGCGATATTAAGGGCT
ACAGAATCAACAAATCTGAGAAACCTCTGTTCACCAGCTTCAAACTGTACCACGAT
 
CTGAAGAGCGTGTTCAGCGACCCTACAAAACTGGAAGATATCGACTTGCTGGACCG
AATCGCCGTGGTGCTGACCATGTACCAAGATGCCGAATCCATGAAAGCCGCCCTGA
ACACCTTCCCTGAAGTGTTCAGCGAAGCAGAAAGAAGCTGAGCGCCCTCAC
AGGCTACGCTGGCACCCATAGACTGTCTCTGAAGTGCATGAACCTGCTGATCCCTGA
TCTGTGGCAGACAAGCCTGAACCAGATGGAACTGTTCGTGAAGCTGAATCTGAAA
CCACAGAAGCTGGACCTGAGCCAGTGCCACCAGATTCCTACCCAGCTGGTGGACG
AGTTCATCCTGTCTCCTGTGGTGAAAAGAGCCTTCACCCAAAGCATCAAGGTGATC
AACGCCATCATCCAGAAATACGGCCTGCCCGACGACATCATAATCGAGCTGGCCAG
GGAAAAAAACAGCGCCGATAAGCGGAAGTTCCTGAATCAGCTGCAGAAGAAGAAC
GAGAAGGCCCGGCACGAGATCAATACCCTGGTGGCCCAGTACGGCCAGCCAAATG
CTAAGCGGCTGGTGGAAAAGATCACACTGCACCAGCAGCAGGAGGGCAAGTGTCT
GTACTCCCTGAAGGATATCCCCCTGGAGCAGCTGCTGAAGCAGCCCTACCTGTACG
AGGTGGACCACATCATCCCCAGAAGCGTTTCTTTCGACAACAGCATGCAGAACAAG
GTGCTGGTCCTGGCCGAAGAAAACGCCAAAAAGGGCAACCAGACCCCTTACCAGT
ACCTGAATAGCAGAGAGGCCAGCATGACCTACCCCGAATTCAAACAGCACATCCTG
AATCTGAGCAAGGCCAAGGACCGGATCAGCAAGAAGAAGCGGAACTACCTGCTCG
AGGAAAGAGATATCAACAAGTTCGACGTGCAAAAGGACTTCATCAACAGAAACCT
GGTTGACACCAGATACGCCACCAGAGAGCTCGCCTCTCTGCTGAAGGCTTATTTCA
AGACACATGAGCTCCCTGTGAAAGTGAAGACCATCAACGGCGGATTTACCCACTAC
CTGAGAAAGGTGTGGAAGTTTGACAAAGATAGAAACAAGGGCTACAAGCACCACG
CCGAGGATGCACTGATCATCGCCAACGCCGACTTTCTGTTTAAGAACCAGACTCTG
AACAAAATCGAGGCTATCCTGAACGAGCCCGGCAGAGAGGTGGAATCTGACACAG
TGAAGGTGCAGAGCGAGGACAATTACCAGGACCTGTTTGAGAACACCAAGAAGGC
TTTCGCCATCAAGAATTTCAAGGATTTCAAGTTTAGCCACAGAGTGGACCAGAAGC
CTAACCGGCAGCTCGTGAACGACACCCTGTACAGCACCAGAGAGGTGAACGAAGA
TCTGTACGTGGTGCAGACCCTGAAGGACATCTACAGCAAGGACAACAAAGACGTG
AAGCGGCTCTTCGACAAGCAACCCGAGAAGTTCCTGATGTTCCAGCACGACCCCG
AGACATTCAAGAAATTCGAGCTGGCTATGAAGCAATATGCCGAAGAGAAGAACCCT
CTGGCTAGATACTACGAGGAACAGGGCTACATCACCAAGTACGCCAAGAAAGGAG
ACGGCCCACCTGTGAAAAGCCTCAAATACATCGGCAAGAAGGTGGGAAAACACCT
 
GGATGTGACCGGCGACTACGAGGATAGCAACCGGAAGCTGGTGAAGCTGAGCCTG
AAGTCCTTTAGATTCGATATCTACCACACAGACAAGGGCTACAAGATGGTTCCAATC
ACTTACCTGGATGTGCAGAAAAAAGAGAAGTACTACTACATCCCCACCGAAATA
CGAAGCCCTGAAGCAGGAGAAGGGCATCAACCAGAATGCTCAGTTCATTGGCAGC
TTCTACTACAACGACCTGATCGAGTTCGATGGCGAGCTGTATAGAGTGATCGGCATC
AACAACGGCGACAAGAATCTCGTTGAACTCGACATGGTGGACATTAGATATAAGGA
ATACTGCGAGCTGAACTCCATCACCACCACACCTAGAATCGTTAAGACCATCAGCCC
CAAGACCCAGAGCATCGAGAAGTACACAACAGATATCCTGGGCAACCTGTACAAA
GCCCAGCCTGGCAAGAAGCCTCAGTTCATCTTCAACAAAGACGAGGAT;
**SEQ ID NO: 27** - the humanized *Sa-SauriCas9* gene sequence: 
ATGAAGCGGAACTACATCCTGGGCCTGGACATCGGCATCACCAGCGTGGGCTACGG
CATCATCGACTACGAGACACGGGACGTGATCGATGCCGGCGTGCGGCTGTTCAAAG
AGGCCAACGTGGAAAACAACGAGGGCAGGCGGAGCAAGAGAGGCGCCAGAAGG
CTGAAGCGGCGGAGGCGGCATAGAATCCAGAGAGTGAAGAAGCTGCTGTTCGACT
ACAACCTGCTGACCGACCACAGCGAGCTGAGCGGCATCAACCCCTACGAGGCCAG
AGTGAAGGGCCTGAGCCAGAAGCTGAGCGAGGAAGAGTTCTCTGCCGCCCTGCTG
CACCTGGCCAAGAGAAGAGGCGTGCACAACGTGAACGAGGTGGAAGAGGACACC
GGCAACGAGCTGTCCACCAAAGAGCAGATCAGCCGGAACAGCAAGGCCCTGGAA
GAGAAATACGTGGCCGAACTGCAGCTGGAACGGCTGAAGAAAGACGGCGAAGTG
CGGGGCAGCATCAACAGATTCAAGACCAGCGACTACGTGAAAGAAGCCAAACAGC
TGCTGAAGGTGCAGAAGGCCTACCACCAGCTGGACCAGAGCTTCATCGACACCTAC
ATCGACCTGCTGGAAACCCGGCGGACCTACTATGAGGGACCTGGCGAGGGCAGCC
CCTTCGGCTGGAAGGACATCAAAGAATGGTACGAGATGCTGATGGGCCACTGCACC
TACTTCCCCGAGGAACTGCGGAGCGTGAAGTACGCCTACAACGCCGACCTGTACAA
CGCCCTGAACGACCTGAACAATCTCGTGATCACCAGGGACGAGAACGAGAAGCTG
GAATATTACGAGAAGTTCCAGATCATCGAGAACGTGTTCAAGCAGAAGAAGAAGCC
CACCCTGAAGCAGATCGCCAAAGAAATCCTCGTGAACGAAGAGGATATTAAGGGCT
ACAGAGTGACCAGCACCGGCAAGCCCGAGTTCACCAACCTGAAGGTGTACCACGA
CATCAAGGACATTACCGCCCGGAAAGAGATTATTGAGAACGCCGAGCTGCTGGATC
AGATTGCCAAGATCCTGACCATCTACCAGAGCAGCGAGGACATCCAGGAAGAACTG
 
ACCAATCTGAACTCCGAGCTGACCCAGGAAGAGATCGAGCAGATCTCTAATCTGAA
GGGCTATACCGGCACCCACAACCTGAGCCTGAAGGCCATCAACCTGATCCTGGACG
AGCTGTGGCACACCAACGACAACCAGATCGCTATCTTCAACCGGCTGAAGCTGGTG
CCCAAGAAGGTGGACCTGTCCCAGCAGAAAGAGATCCCCACCACCCTGGTGGACG
ACTTCATCCTGAGCCCCGTCGTGAAGAGAAGCTTCATCCAGAGCATCAAAGTGATC
AACGCCATCATCAAGAAGTACGGCCTGCCCAACGACATCATTATCGAGCTGGCCCG
CGAGAAGAACTCCAAGGACGCCCAGAAAATGATCAACGAGATGCAGAAGCGGAA
CGCCGCCACCAACGAGCGGATCGAGGAAATCATCCGGACCACCGGCAAAGAGAAC
GCCAAGTACCTGATCGAGAAGATCAAGCTGCACGACATGCAGGAAGGCAAGTGCC
TGTACAGCCTGGAAGCCATCCCTCTGGAAGATCTGCTGAACAACCCCTTCAACTAT
GAGGTGGACCACATCATCCCCAGAAGCGTGTCCTTCGACAACAGCTTCAACAACA
AGGTGCTCGTGAAGCAGGAAGAAAACAGCAAGAAGGGCAACCGGACCCCATTCC
AGTACCTGAGCAGCAGCGACAGCAAGATCAGCTACGAAACCTTCAAGAAGCACAT
CCTGAATCTGGCCAAGGGCAAGGGCAGAATCAGCAAGACCAAGAAAGAGTATCTG
CTGGAAGAACGGGACATCAACAGGTTCTCCGTGCAGAAAGACTTCATCAACCGGA
ACCTGGTGGATACCAGATACGCCACCGCCGCCCTGATGAACCTGCTGCGGAGCTAC
TTCAGAGTGAACAACCTGGACGTGAAAGTGAAGTCCATCAATGGCGGCTTCACCA
GCTTTCTGCGGCGGAAGTGGAAGTTTAAGAAAGAGCGGAACAAGGGGTACAAGCA
CCACGCCGAGGACGCCCTGATCATTGCCAACGCCGATTTCATCTTCAAAGAGTGGA
AGAAACTGGACAAGGCCAAAAAAGTGATGGAAAACCAGATGTTCGAGGAAAAGC
AGGCCGAGAGCATGCCCGAGATCGAAACCGAGCAGGAGTACAAAGAGATCTTCAT
CACCCCCCACCAGATCAAGCACATTAAGGACTTCAAGGACTACAAGTACAGCCACC
GGGTGGACAAGAAGCCTAATAGAGAGCTGATTAACGACACCCTGTACTCCACCCGG
AAGGACGACAAGGGCAACACCCTGATCGTGAACAATCTGAACGGCCTGTACGACA
AGGACAATGACAAGCTGAAAAAGCTGATCAACAAGAGCCCCGAAAAGCTGCTGAT
GTACCACCACGACCCCCAGACCTACCAGAAACTGAAGCTGATTATGGAACAGTACG
GCGACGAGAAGAATCCCCTGTACAAGTACTACGAGGAAACCGGGAACTACCTGAC
CAAGTACTCCAAAAAGGACAACGGCCCCGTGATCAAGAAGATTAAGTATTACGGCA
ACAAACTGAACGCCCATCTGGACATCACCGACGACTACCCCAACAGCAGAAACAA
GGTCGTGAAGCTGTCCCTGAAGAGCTTCCGCTTCGACATCTACAAGTGCGAGCAGG
 
GCTACAAGATGGTGAGCATCGGCTACCTGGACGTGCTGAAGAAGGACAACTACTAC
TACATCCCCAAGGACAAGTACGAGGCCGAGAAGCAGAAGAAGATAAAGGAG
AGCGACCTGTTCGTGGGCAGCTTCTACTACAACGACCTGATAATGTACGAGGACGA
GCTGTTCCGCGTGATAGGCGTGAACAGCGACATCAACAACCTGGTGGAGCTGAACA
TGGTGGACATCACCTACAAGGACTTCTGCGAGGTGAACAACGTGACCGGCGAGAA
GCGCATCAAGAAGACCATCGGCAAGCGCGTGGTGCTGATAGAGAAGTACACCACC
GACATCCTGGGCAACCTGTACAAGACCCCCCTGCCCAAGAAGCCCCAGCTGATATT
CAAGCGCGGCGAGCTG;
SEQ ID NO: 28 - the humanized *Sa-SepCas9* gene sequence: 
ATGAAGCGGAACTACATCCTGGGCCTGGACATCGGCATCACCAGCGTGGGCTACGG
CATCATCGACTACGAGACACGGGACGTGATCGATGCCGGCGTGCGGCTGTTCAAAG
AGGCCAACGTGGAAAACAACGAGGGCAGGCGGAGCAAGAGAGGCGCCAGAAGG
CTGAAGCGGCGGAGGCGGCATAGAATCCAGAGAGTGAAGAAGCTGCTGTTCGACT
ACAACCTGCTGACCGACCACAGCGAGCTGAGCGGCATCAACCCCTACGAGGCCAG
AGTGAAGGGCCTGAGCCAGAAGCTGAGCGAGGAAGAGTTCTCTGCCGCCCTGCTG
CACCTGGCCAAGAGAAGAGGCGTGCACAACGTGAACGAGGTGGAAGAGGACACC
GGCAACGAGCTGTCCACCAAAGAGCAGATCAGCCGGAACAGCAAGGCCCTGGAA
GAGAAATACGTGGCCGAACTGCAGCTGGAACGGCTGAAGAAAGACGGCGAAGTG
CGGGGCAGCATCAACAGATTCAAGACCAGCGACTACGTGAAAGAAGCCAAACAGC
TGCTGAAGGTGCAGAAGGCCTACCACCAGCTGGACCAGAGCTTCATCGACACCTAC
ATCGACCTGCTGGAAACCCGGCGGACCTACTATGAGGGACCTGGCGAGGGCAGCC
CCTTCGGCTGGAAGGACATCAAAGAATGGTACGAGATGCTGATGGGCCACTGCACC
TACTTCCCCGAGGAACTGCGGAGCGTGAAGTACGCCTACAACGCCGACCTGTACAA
CGCCCTGAACGACCTGAACAATCTCGTGATCACCAGGGACGAGAACGAGAAGCTG
GAATATTACGAGAAGTTCCAGATCATCGAGAACGTGTTCAAGCAGAAGAAGAAGCC
CACCCTGAAGCAGATCGCCAAAGAAATCCTCGTGAACGAAGAGGATATTAAGGGCT
ACAGAGTGACCAGCACCGGCAAGCCCGAGTTCACCAACCTGAAGGTGTACCACGA
CATCAAGGACATTACCGCCCGGAAAGAGATTATTGAGAACGCCGAGCTGCTGGATC
AGATTGCCAAGATCCTGACCATCTACCAGAGCAGCGAGGACATCCAGGAAGAACTG
ACCAATCTGAACTCCGAGCTGACCCAGGAAGAGATCGAGCAGATCTCTAATCTGAA
 
GGGCTATACCGGCACCCACAACCTGAGCCTGAAGGCCATCAACCTGATCCTGGACG
AGCTGTGGCACACCAACGACAACCAGATCGCTATCTTCAACCGGCTGAAGCTGGTG
CCCAAGAAGGTGGACCTGTCCCAGCAGAAAGAGATCCCCACCACCCTGGTGGACG
ACTTCATCCTGAGCCCCGTCGTGAAGAGAAGCTTCATCCAGAGCATCAAAGTGATC
AACGCCATCATCAAGAAGTACGGCCTGCCCAACGACATCATTATCGAGCTGGCCCG
CGAGAAGAACTCCAAGGACGCCCAGAAAATGATCAACGAGATGCAGAAGCGGAA
CGCCGCCACCAACGAGCGGATCGAGGAAATCATCCGGACCACCGGCAAAGAGAAC
GCCAAGTACCTGATCGAGAAGATCAAGCTGCACGACATGCAGGAAGGCAAGTGCC
TGTACAGCCTGGAAGCCATCCCTCTGGAAGATCTGCTGAACAACCCCTTCAACTAT
GAGGTGGACCACATCATCCCCAGAAGCGTGTCCTTCGACAACAGCTTCAACAACA
AGGTGCTCGTGAAGCAGGAAGAAAACAGCAAGAAGGGCAACCGGACCCCATTCC
AGTACCTGAGCAGCAGCGACAGCAAGATCAGCTACGAAACCTTCAAGAAGCACAT
CCTGAATCTGGCCAAGGGCAAGGGCAGAATCAGCAAGACCAAGAAAGAGTATCTG
CTGGAAGAACGGGACATCAACAGGTTCTCCGTGCAGAAAGACTTCATCAACCGGA
ACCTGGTGGATACCAGATACGCCACCGCCGCCCTGATGAACCTGCTGCGGAGCTAC
TTCAGAGTGAACAACCTGGACGTGAAAGTGAAGTCCATCAATGGCGGCTTCACCA
GCTTTCTGCGGCGGAAGTGGAAGTTTAAGAAAGAGCGGAACAAGGGGTACAAGCA
CCACGCCGAGGACGCCCTGATCATTGCCAACGCCGATTTCATCTTCAAAGAGTGGA
AGAAACTGGACAAGGCCAAAAAAGTGATGGAAAACCAGATGTTCGAGGAAAAGC
AGGCCGAGAGCATGCCCGAGATCGAAACCGAGCAGGAGTACAAAGAGATCTTCAT
CACCCCCCACCAGATCAAGCACATTAAGGACTTCAAGGACTACAAGTACAGCCACC
GGGTGGACAAGAAGCCTAATAGAGAGCTGATTAACGACACCCTGTACTCCACCCGG
AAGGACGACAAGGGCAACACCCTGATCGTGAACAATCTGAACGGCCTGTACGACA
AGGACAATGACAAGCTGAAAAAGCTGATCAACAAGAGCCCCGAAAAGCTGCTGAT
GTACCACCACGACCCCCAGACCTACCAGAAACTGAAGCTGATTATGGAACAGTACG
GCGACGAGAAGAATCCCCTGTACAAGTACTACGAGGAAACCGGGAACTACCTGAC
CAAGTACTCCAAAAAGGACAACGGCCCCGTGATCAAGAAGATTAAGTATTACGGCA
ACAAACTGAACGCCCATCTGGACATCACCGACGACTACCCCAACAGCAGAAACAA
GGTCGTGAAGCTGTCCCTGAAGAACTACCGCTTCGACGTGTACCTGACCGAGAAG
GGCTACAAGTTCGTGACCATCGCCTACCTGAACGTGTTCAAGAAGGACAACTACTA
 
CTACATCCCCAAGGACATGTACCAGGAGCTGAAGGCCAAGAAGAAGATAAAGGAC
ACCGACCAGTTCATCGCCAGCTTCTACAAGAACGACCTGATAAAGCTGAACGGCGA
CCTGTACAAGATAATCGGCGTGAACAGCGACGACCGCAACATCATCGAGCTGGACT
ACTACGACATCAAGTACAAGGACTACTGCGAGATAAACAACATCAAGGGCGAGCCC
CGCATCAAGAAGACCATCGGCAAGAAGACCGAGAGCATCGAGAAGCTGACCACCG
ACGTGCTGGGCAACCTGTACCTGCACAGCACCGAGAAGGCCCCCCAGCTGATATTC
AAGCGCGGCCTG;
**SEQ ID NO: 29** - the humanized *Sa-SeqCas9* gene sequence: 
ATGAAGCGGAACTACATCCTGGGCCTGGACATCGGCATCACCAGCGTGGGCTACGG
CATCATCGACTACGAGACACGGGACGTGATCGATGCCGGCGTGCGGCTGTTCAAAG
AGGCCAACGTGGAAAACAACGAGGGCAGGCGGAGCAAGAGAGGCGCCAGAAGG
CTGAAGCGGCGGAGGCGGCATAGAATCCAGAGAGTGAAGAAGCTGCTGTTCGACT
ACAACCTGCTGACCGACCACAGCGAGCTGAGCGGCATCAACCCCTACGAGGCCAG
AGTGAAGGGCCTGAGCCAGAAGCTGAGCGAGGAAGAGTTCTCTGCCGCCCTGCTG
CACCTGGCCAAGAGAAGAGGCGTGCACAACGTGAACGAGGTGGAAGAGGACACC
GGCAACGAGCTGTCCACCAAAGAGCAGATCAGCCGGAACAGCAAGGCCCTGGAA
GAGAAATACGTGGCCGAACTGCAGCTGGAACGGCTGAAGAAAGACGGCGAAGTG
CGGGGCAGCATCAACAGATTCAAGACCAGCGACTACGTGAAAGAAGCCAAACAGC
TGCTGAAGGTGCAGAAGGCCTACCACCAGCTGGACCAGAGCTTCATCGACACCTAC
ATCGACCTGCTGGAAACCCGGCGGACCTACTATGAGGGACCTGGCGAGGGCAGCC
CCTTCGGCTGGAAGGACATCAAAGAATGGTACGAGATGCTGATGGGCCACTGCACC
TACTTCCCCGAGGAACTGCGGAGCGTGAAGTACGCCTACAACGCCGACCTGTACAA
CGCCCTGAACGACCTGAACAATCTCGTGATCACCAGGGACGAGAACGAGAAGCTG
GAATATTACGAGAAGTTCCAGATCATCGAGAACGTGTTCAAGCAGAAGAAGAAGCC
CACCCTGAAGCAGATCGCCAAAGAAATCCTCGTGAACGAAGAGGATATTAAGGGCT
ACAGAGTGACCAGCACCGGCAAGCCCGAGTTCACCAACCTGAAGGTGTACCACGA
CATCAAGGACATTACCGCCCGGAAAGAGATTATTGAGAACGCCGAGCTGCTGGATC
AGATTGCCAAGATCCTGACCATCTACCAGAGCAGCGAGGACATCCAGGAAGAACTG
ACCAATCTGAACTCCGAGCTGACCCAGGAAGAGATCGAGCAGATCTCTAATCTGAA
GGGCTATACCGGCACCCACAACCTGAGCCTGAAGGCCATCAACCTGATCCTGGACG
 
AGCTGTGGCACACCAACGACAACCAGATCGCTATCTTCAACCGGCTGAAGCTGGTG
CCCAAGAAGGTGGACCTGTCCCAGCAGAAAGAGATCCCCACCACCCTGGTGGACG
ACTTCATCCTGAGCCCCGTCGTGAAGAGAAGCTTCATCCAGAGCATCAAAGTGATC
AACGCCATCATCAAGAAGTACGGCCTGCCCAACGACATCATTATCGAGCTGGCCCG
CGAGAAGAACTCCAAGGACGCCCAGAAAATGATCAACGAGATGCAGAAGCGGAA
CGCCGCCACCAACGAGCGGATCGAGGAAATCATCCGGACCACCGGCAAAGAGAAC
GCCAAGTACCTGATCGAGAAGATCAAGCTGCACGACATGCAGGAAGGCAAGTGCC
TGTACAGCCTGGAAGCCATCCCTCTGGAAGATCTGCTGAACAACCCCTTCAACTAT
GAGGTGGACCACATCATCCCCAGAAGCGTGTCCTTCGACAACAGCTTCAACAACA
AGGTGCTCGTGAAGCAGGAAGAAAACAGCAAGAAGGGCAACCGGACCCCATTCC
AGTACCTGAGCAGCAGCGACAGCAAGATCAGCTACGAAACCTTCAAGAAGCACAT
CCTGAATCTGGCCAAGGGCAAGGGCAGAATCAGCAAGACCAAGAAAGAGTATCTG
CTGGAAGAACGGGACATCAACAGGTTCTCCGTGCAGAAAGACTTCATCAACCGGA
ACCTGGTGGATACCAGATACGCCACCGCCGCCCTGATGAACCTGCTGCGGAGCTAC
TTCAGAGTGAACAACCTGGACGTGAAAGTGAAGTCCATCAATGGCGGCTTCACCA
GCTTTCTGCGGCGGAAGTGGAAGTTTAAGAAAGAGCGGAACAAGGGGTACAAGCA
CCACGCCGAGGACGCCCTGATCATTGCCAACGCCGATTTCATCTTCAAAGAGTGGA
AGAAACTGGACAAGGCCAAAAAAGTGATGGAAAACCAGATGTTCGAGGAAAAGC
AGGCCGAGAGCATGCCCGAGATCGAAACCGAGCAGGAGTACAAAGAGATCTTCAT
CACCCCCCACCAGATCAAGCACATTAAGGACTTCAAGGACTACAAGTACAGCCACC
GGGTGGACAAGAAGCCTAATAGAGAGCTGATTAACGACACCCTGTACTCCACCCGG
AAGGACGACAAGGGCAACACCCTGATCGTGAACAATCTGAACGGCCTGTACGACA
AGGACAATGACAAGCTGAAAAAGCTGATCAACAAGAGCCCCGAAAAGCTGCTGAT
GTACCACCACGACCCCCAGACCTACCAGAAACTGAAGCTGATTATGGAACAGTACG
GCGACGAGAAGAATCCCCTGTACAAGTACTACGAGGAAACCGGGAACTACCTGAC
CAAGTACTCCAAAAAGGACAACGGCCCCGTGATCAAGAAGATTAAGTATTACGGCA
ACAAACTGAACGCCCATCTGGACATCACCGACGACTACCCCAACAGCAGAAACAA
GGTCGTGAAGCTGTCCCTGAAGCCCTACCGCTTCGACGTGTACCTGGACAACGGCG
TGTACAAGTTCGTGACCGTGAAGAACCTGAACGTGATAAAGAAGGAGAACTACTA
CGAGGTGAACAGCAAGTGCTACGAGAAGGCCAAGAAGCTGAAGAAGATAAGCGA
 
CCAGGCCGAGTTCATCGCCAGCTTCTACAACAACGACCTGATAAAGATAGACGGCG
AGCTGTACCGCGTGATAGGCGTGAACACCGACCTGATAAACCGCATCGAGGTGAAC
ATGGTGGACATCACCTACCGCGAGTACCTGGAGAACATGAACGACAAGCGCAGCC
CCCGCATCTTCAAGACCATCGCCAGCAAGACCCAGAGCATCAAGAAGTACAGCACC
GACATCCTGGGCACCCTGTACGAGGTGAACAGCAAGAAGCACCCCCAGATGATAAT
GAAGGGC;
**SEQ ID NO: 30** - the humanized *Sa-ShaCas9* gene sequence: 
ATGAAGCGGAACTACATCCTGGGCCTGGACATCGGCATCACCAGCGTGGGCTACGG
CATCATCGACTACGAGACACGGGACGTGATCGATGCCGGCGTGCGGCTGTTCAAAG
AGGCCAACGTGGAAAACAACGAGGGCAGGCGGAGCAAGAGAGGCGCCAGAAGG
CTGAAGCGGCGGAGGCGGCATAGAATCCAGAGAGTGAAGAAGCTGCTGTTCGACT
ACAACCTGCTGACCGACCACAGCGAGCTGAGCGGCATCAACCCCTACGAGGCCAG
AGTGAAGGGCCTGAGCCAGAAGCTGAGCGAGGAAGAGTTCTCTGCCGCCCTGCTG
CACCTGGCCAAGAGAAGAGGCGTGCACAACGTGAACGAGGTGGAAGAGGACACC
GGCAACGAGCTGTCCACCAAAGAGCAGATCAGCCGGAACAGCAAGGCCCTGGAA
GAGAAATACGTGGCCGAACTGCAGCTGGAACGGCTGAAGAAAGACGGCGAAGTG
CGGGGCAGCATCAACAGATTCAAGACCAGCGACTACGTGAAAGAAGCCAAACAGC
TGCTGAAGGTGCAGAAGGCCTACCACCAGCTGGACCAGAGCTTCATCGACACCTAC
ATCGACCTGCTGGAAACCCGGCGGACCTACTATGAGGGACCTGGCGAGGGCAGCC
CCTTCGGCTGGAAGGACATCAAAGAATGGTACGAGATGCTGATGGGCCACTGCACC
TACTTCCCCGAGGAACTGCGGAGCGTGAAGTACGCCTACAACGCCGACCTGTACAA
CGCCCTGAACGACCTGAACAATCTCGTGATCACCAGGGACGAGAACGAGAAGCTG
GAATATTACGAGAAGTTCCAGATCATCGAGAACGTGTTCAAGCAGAAGAAGAAGCC
CACCCTGAAGCAGATCGCCAAAGAAATCCTCGTGAACGAAGAGGATATTAAGGGCT
ACAGAGTGACCAGCACCGGCAAGCCCGAGTTCACCAACCTGAAGGTGTACCACGA
CATCAAGGACATTACCGCCCGGAAAGAGATTATTGAGAACGCCGAGCTGCTGGATC
AGATTGCCAAGATCCTGACCATCTACCAGAGCAGCGAGGACATCCAGGAAGAACTG
ACCAATCTGAACTCCGAGCTGACCCAGGAAGAGATCGAGCAGATCTCTAATCTGAA
GGGCTATACCGGCACCCACAACCTGAGCCTGAAGGCCATCAACCTGATCCTGGACG
AGCTGTGGCACACCAACGACAACCAGATCGCTATCTTCAACCGGCTGAAGCTGGTG
 
CCCAAGAAGGTGGACCTGTCCCAGCAGAAAGAGATCCCCACCACCCTGGTGGACG
ACTTCATCCTGAGCCCCGTCGTGAAGAGAAGCTTCATCCAGAGCATCAAAGTGATC
AACGCCATCATCAAGAAGTACGGCCTGCCCAACGACATCATTATCGAGCTGGCCCG
CGAGAAGAACTCCAAGGACGCCCAGAAAATGATCAACGAGATGCAGAAGCGGAA
CGCCGCCACCAACGAGCGGATCGAGGAAATCATCCGGACCACCGGCAAAGAGAAC
GCCAAGTACCTGATCGAGAAGATCAAGCTGCACGACATGCAGGAAGGCAAGTGCC
TGTACAGCCTGGAAGCCATCCCTCTGGAAGATCTGCTGAACAACCCCTTCAACTAT
GAGGTGGACCACATCATCCCCAGAAGCGTGTCCTTCGACAACAGCTTCAACAACA
AGGTGCTCGTGAAGCAGGAAGAAAACAGCAAGAAGGGCAACCGGACCCCATTCC
AGTACCTGAGCAGCAGCGACAGCAAGATCAGCTACGAAACCTTCAAGAAGCACAT
CCTGAATCTGGCCAAGGGCAAGGGCAGAATCAGCAAGACCAAGAAAGAGTATCTG
CTGGAAGAACGGGACATCAACAGGTTCTCCGTGCAGAAAGACTTCATCAACCGGA
ACCTGGTGGATACCAGATACGCCACCGCCGCCCTGATGAACCTGCTGCGGAGCTAC
TTCAGAGTGAACAACCTGGACGTGAAAGTGAAGTCCATCAATGGCGGCTTCACCA
GCTTTCTGCGGCGGAAGTGGAAGTTTAAGAAAGAGCGGAACAAGGGGTACAAGCA
CCACGCCGAGGACGCCCTGATCATTGCCAACGCCGATTTCATCTTCAAAGAGTGGA
AGAAACTGGACAAGGCCAAAAAAGTGATGGAAAACCAGATGTTCGAGGAAAAGC
AGGCCGAGAGCATGCCCGAGATCGAAACCGAGCAGGAGTACAAAGAGATCTTCAT
CACCCCCCACCAGATCAAGCACATTAAGGACTTCAAGGACTACAAGTACAGCCACC
GGGTGGACAAGAAGCCTAATAGAGAGCTGATTAACGACACCCTGTACTCCACCCGG
AAGGACGACAAGGGCAACACCCTGATCGTGAACAATCTGAACGGCCTGTACGACA
AGGACAATGACAAGCTGAAAAAGCTGATCAACAAGAGCCCCGAAAAGCTGCTGAT
GTACCACCACGACCCCCAGACCTACCAGAAACTGAAGCTGATTATGGAACAGTACG
GCGACGAGAAGAATCCCCTGTACAAGTACTACGAGGAAACCGGGAACTACCTGAC
CAAGTACTCCAAAAAGGACAACGGCCCCGTGATCAAGAAGATTAAGTATTACGGCA
ACAAACTGAACGCCCATCTGGACATCACCGACGACTACCCCAACAGCAGAAACAA
GGTCGTGAAGCTGTCCCTGAAGAGCTACCGCTTCGACGTGTACCTGACCGACAAGG
GCTACAAGTTCGTGAGCATCACCTACCTGGACGTGCTGAAGAAGGAGAACTACTAC
TACATCAGCGAGGCCAAGTACGACAAGCTGAAGCTGAACAAGGGCATCGACGACA
AGGCCAAGTTCATCGGCAGCTTCTACTACAACGACCTGATAGAGCTGGACGGCGAG
 
GTGTACACCGTGATAGGCGTGAACAACGACAAGAACAACGTGATAGAGCTGAACC
TGCCCGAGATACGCTACAAGGAGTACTGCGAGATAAACAACATCAAGGGCAGCGG
CAGGCTGCGCATCACCATCGGCAAGAAGGTGAACAGCATCCGCAAGCTGAGCACC
GACGTGCTGGGCAACCGCTACTACCAGAGCTTCGCCAAGAAGCCCCAGCTGGTGTT
CAAGAAGGGCATC;
**SEQ ID NO: 31** - the humanized *Sa-SlugCas9* gene sequence: 
ATGAAGCGGAACTACATCCTGGGCCTGGACATCGGCATCACCAGCGTGGGCTACGG
CATCATCGACTACGAGACACGGGACGTGATCGATGCCGGCGTGCGGCTGTTCAAAG
AGGCCAACGTGGAAAACAACGAGGGCAGGCGGAGCAAGAGAGGCGCCAGAAGG
CTGAAGCGGCGGAGGCGGCATAGAATCCAGAGAGTGAAGAAGCTGCTGTTCGACT
ACAACCTGCTGACCGACCACAGCGAGCTGAGCGGCATCAACCCCTACGAGGCCAG
AGTGAAGGGCCTGAGCCAGAAGCTGAGCGAGGAAGAGTTCTCTGCCGCCCTGCTG
CACCTGGCCAAGAGAAGAGGCGTGCACAACGTGAACGAGGTGGAAGAGGACACC
GGCAACGAGCTGTCCACCAAAGAGCAGATCAGCCGGAACAGCAAGGCCCTGGAA
GAGAAATACGTGGCCGAACTGCAGCTGGAACGGCTGAAGAAAGACGGCGAAGTG
CGGGGCAGCATCAACAGATTCAAGACCAGCGACTACGTGAAAGAAGCCAAACAGC
TGCTGAAGGTGCAGAAGGCCTACCACCAGCTGGACCAGAGCTTCATCGACACCTAC
ATCGACCTGCTGGAAACCCGGCGGACCTACTATGAGGGACCTGGCGAGGGCAGCC
CCTTCGGCTGGAAGGACATCAAAGAATGGTACGAGATGCTGATGGGCCACTGCACC
TACTTCCCCGAGGAACTGCGGAGCGTGAAGTACGCCTACAACGCCGACCTGTACAA
CGCCCTGAACGACCTGAACAATCTCGTGATCACCAGGGACGAGAACGAGAAGCTG
GAATATTACGAGAAGTTCCAGATCATCGAGAACGTGTTCAAGCAGAAGAAGAAGCC
CACCCTGAAGCAGATCGCCAAAGAAATCCTCGTGAACGAAGAGGATATTAAGGGCT
ACAGAGTGACCAGCACCGGCAAGCCCGAGTTCACCAACCTGAAGGTGTACCACGA
CATCAAGGACATTACCGCCCGGAAAGAGATTATTGAGAACGCCGAGCTGCTGGATC
AGATTGCCAAGATCCTGACCATCTACCAGAGCAGCGAGGACATCCAGGAAGAACTG
ACCAATCTGAACTCCGAGCTGACCCAGGAAGAGATCGAGCAGATCTCTAATCTGAA
GGGCTATACCGGCACCCACAACCTGAGCCTGAAGGCCATCAACCTGATCCTGGACG
AGCTGTGGCACACCAACGACAACCAGATCGCTATCTTCAACCGGCTGAAGCTGGTG
CCCAAGAAGGTGGACCTGTCCCAGCAGAAAGAGATCCCCACCACCCTGGTGGACG
 
ACTTCATCCTGAGCCCCGTCGTGAAGAGAAGCTTCATCCAGAGCATCAAAGTGATC
AACGCCATCATCAAGAAGTACGGCCTGCCCAACGACATCATTATCGAGCTGGCCCG
CGAGAAGAACTCCAAGGACGCCCAGAAAATGATCAACGAGATGCAGAAGCGGAA
CGCCGCCACCAACGAGCGGATCGAGGAAATCATCCGGACCACCGGCAAAGAGAAC
GCCAAGTACCTGATCGAGAAGATCAAGCTGCACGACATGCAGGAAGGCAAGTGCC
TGTACAGCCTGGAAGCCATCCCTCTGGAAGATCTGCTGAACAACCCCTTCAACTAT
GAGGTGGACCACATCATCCCCAGAAGCGTGTCCTTCGACAACAGCTTCAACAACA
AGGTGCTCGTGAAGCAGGAAGAAAACAGCAAGAAGGGCAACCGGACCCCATTCC
AGTACCTGAGCAGCAGCGACAGCAAGATCAGCTACGAAACCTTCAAGAAGCACAT
CCTGAATCTGGCCAAGGGCAAGGGCAGAATCAGCAAGACCAAGAAAGAGTATCTG
CTGGAAGAACGGGACATCAACAGGTTCTCCGTGCAGAAAGACTTCATCAACCGGA
ACCTGGTGGATACCAGATACGCCACCGCCGCCCTGATGAACCTGCTGCGGAGCTAC
TTCAGAGTGAACAACCTGGACGTGAAAGTGAAGTCCATCAATGGCGGCTTCACCA
GCTTTCTGCGGCGGAAGTGGAAGTTTAAGAAAGAGCGGAACAAGGGGTACAAGCA
CCACGCCGAGGACGCCCTGATCATTGCCAACGCCGATTTCATCTTCAAAGAGTGGA
AGAAACTGGACAAGGCCAAAAAAGTGATGGAAAACCAGATGTTCGAGGAAAAGC
AGGCCGAGAGCATGCCCGAGATCGAAACCGAGCAGGAGTACAAAGAGATCTTCAT
CACCCCCCACCAGATCAAGCACATTAAGGACTTCAAGGACTACAAGTACAGCCACC
GGGTGGACAAGAAGCCTAATAGAGAGCTGATTAACGACACCCTGTACTCCACCCGG
AAGGACGACAAGGGCAACACCCTGATCGTGAACAATCTGAACGGCCTGTACGACA
AGGACAATGACAAGCTGAAAAAGCTGATCAACAAGAGCCCCGAAAAGCTGCTGAT
GTACCACCACGACCCCCAGACCTACCAGAAACTGAAGCTGATTATGGAACAGTACG
GCGACGAGAAGAATCCCCTGTACAAGTACTACGAGGAAACCGGGAACTACCTGAC
CAAGTACTCCAAAAAGGACAACGGCCCCGTGATCAAGAAGATTAAGTATTACGGCA
ACAAACTGAACGCCCATCTGGACATCACCGACGACTACCCCAACAGCAGAAACAA
GGTCGTGAAGCTGTCCCTGAAGCCCTACCGCTTCGACGTGTACCTGACCGACAAGG
GCTACAAGTTCATCACCATCAGCTACCTGGACGTGCTGAAGAAGGACAACTACTAC
TACATCCCCGAGCAGAAGTACGACAAGCTGAAGCTGGGCAAGGCCATCGACAAGA
ACGCCAAGTTCATCGCCAGCTTCTACAAGAACGACCTGATAAAGCTGGACGGCGAG
ATATACAAGATAATCGGCGTGAACAGCGACACCCGCAACATGATAGAGCTGGACCT
 
GCCCGACATCCGCTACAAGGAGTACTGCGAGCTGAACAACATCAAGGGCGAGCCC
CGCATCAAGAAGACCATCGGCAAGAAGGTGAACAGCATCGAGAAGCTGACCACCG
ACGTGCTGGGCAACGTGTTCACCAACACCCAGTACACCAAGCCCCAGCTGCTGTTC
AAGCGCGGCAAC；
**SEQ ID NO: 32** - the humanized *Sa-SlutCas9* gene sequence: 
ATGAAGCGGAACTACATCCTGGGCCTGGACATCGGCATCACCAGCGTGGGCTACGG
CATCATCGACTACGAGACACGGGACGTGATCGATGCCGGCGTGCGGCTGTTCAAAG
AGGCCAACGTGGAAAACAACGAGGGCAGGCGGAGCAAGAGAGGCGCCAGAAGG
CTGAAGCGGCGGAGGCGGCATAGAATCCAGAGAGTGAAGAAGCTGCTGTTCGACT
ACAACCTGCTGACCGACCACAGCGAGCTGAGCGGCATCAACCCCTACGAGGCCAG
AGTGAAGGGCCTGAGCCAGAAGCTGAGCGAGGAAGAGTTCTCTGCCGCCCTGCTG
CACCTGGCCAAGAGAAGAGGCGTGCACAACGTGAACGAGGTGGAAGAGGACACC
GGCAACGAGCTGTCCACCAAAGAGCAGATCAGCCGGAACAGCAAGGCCCTGGAA
GAGAAATACGTGGCCGAACTGCAGCTGGAACGGCTGAAGAAAGACGGCGAAGTG
CGGGGCAGCATCAACAGATTCAAGACCAGCGACTACGTGAAAGAAGCCAAACAGC
TGCTGAAGGTGCAGAAGGCCTACCACCAGCTGGACCAGAGCTTCATCGACACCTAC
ATCGACCTGCTGGAAACCCGGCGGACCTACTATGAGGGACCTGGCGAGGGCAGCC
CCTTCGGCTGGAAGGACATCAAAGAATGGTACGAGATGCTGATGGGCCACTGCACC
TACTTCCCCGAGGAACTGCGGAGCGTGAAGTACGCCTACAACGCCGACCTGTACAA
CGCCCTGAACGACCTGAACAATCTCGTGATCACCAGGGACGAGAACGAGAAGCTG
GAATATTACGAGAAGTTCCAGATCATCGAGAACGTGTTCAAGCAGAAGAAGAAGCC
CACCCTGAAGCAGATCGCCAAAGAAATCCTCGTGAACGAAGAGGATATTAAGGGCT
ACAGAGTGACCAGCACCGGCAAGCCCGAGTTCACCAACCTGAAGGTGTACCACGA
CATCAAGGACATTACCGCCCGGAAAGAGATTATTGAGAACGCCGAGCTGCTGGATC
AGATTGCCAAGATCCTGACCATCTACCAGAGCAGCGAGGACATCCAGGAAGAACTG
ACCAATCTGAACTCCGAGCTGACCCAGGAAGAGATCGAGCAGATCTCTAATCTGAA
GGGCTATACCGGCACCCACAACCTGAGCCTGAAGGCCATCAACCTGATCCTGGACG
AGCTGTGGCACACCAACGACAACCAGATCGCTATCTTCAACCGGCTGAAGCTGGTG
CCCAAGAAGGTGGACCTGTCCCAGCAGAAAGAGATCCCCACCACCCTGGTGGACG
ACTTCATCCTGAGCCCCGTCGTGAAGAGAAGCTTCATCCAGAGCATCAAAGTGATC
 
AACGCCATCATCAAGAAGTACGGCCTGCCCAACGACATCATTATCGAGCTGGCCCG
CGAGAAGAACTCCAAGGACGCCCAGAAAATGATCAACGAGATGCAGAAGCGGAA
CGCCGCCACCAACGAGCGGATCGAGGAAATCATCCGGACCACCGGCAAAGAGAAC
GCCAAGTACCTGATCGAGAAGATCAAGCTGCACGACATGCAGGAAGGCAAGTGCC
TGTACAGCCTGGAAGCCATCCCTCTGGAAGATCTGCTGAACAACCCCTTCAACTAT
GAGGTGGACCACATCATCCCCAGAAGCGTGTCCTTCGACAACAGCTTCAACAACA
AGGTGCTCGTGAAGCAGGAAGAAAACAGCAAGAAGGGCAACCGGACCCCATTCC
AGTACCTGAGCAGCAGCGACAGCAAGATCAGCTACGAAACCTTCAAGAAGCACAT
CCTGAATCTGGCCAAGGGCAAGGGCAGAATCAGCAAGACCAAGAAAGAGTATCTG
CTGGAAGAACGGGACATCAACAGGTTCTCCGTGCAGAAAGACTTCATCAACCGGA
ACCTGGTGGATACCAGATACGCCACCGCCGCCCTGATGAACCTGCTGCGGAGCTAC
TTCAGAGTGAACAACCTGGACGTGAAAGTGAAGTCCATCAATGGCGGCTTCACCA
GCTTTCTGCGGCGGAAGTGGAAGTTTAAGAAAGAGCGGAACAAGGGGTACAAGCA
CCACGCCGAGGACGCCCTGATCATTGCCAACGCCGATTTCATCTTCAAAGAGTGGA
AGAAACTGGACAAGGCCAAAAAAGTGATGGAAAACCAGATGTTCGAGGAAAAGC
AGGCCGAGAGCATGCCCGAGATCGAAACCGAGCAGGAGTACAAAGAGATCTTCAT
CACCCCCCACCAGATCAAGCACATTAAGGACTTCAAGGACTACAAGTACAGCCACC
GGGTGGACAAGAAGCCTAATAGAGAGCTGATTAACGACACCCTGTACTCCACCCGG
AAGGACGACAAGGGCAACACCCTGATCGTGAACAATCTGAACGGCCTGTACGACA
AGGACAATGACAAGCTGAAAAAGCTGATCAACAAGAGCCCCGAAAAGCTGCTGAT
GTACCACCACGACCCCCAGACCTACCAGAAACTGAAGCTGATTATGGAACAGTACG
GCGACGAGAAGAATCCCCTGTACAAGTACTACGAGGAAACCGGGAACTACCTGAC
CAAGTACTCCAAAAAGGACAACGGCCCCGTGATCAAGAAGATTAAGTATTACGGCA
ACAAACTGAACGCCCATCTGGACATCACCGACGACTACCCCAACAGCAGAAACAA
GGTCGTGAAGCTGTCCCTGAAGAGCTTCCGCTTCGACATCTACCACACCGACAAGG
GCTACAAGATGGTGCCCATCACCTACCTGGACGTGCAGAAGAAGGAGAAGTACTAC
TACATCCCCACCGAGAAGTACGAGGCCCTGAAGCAGGAGAAGGGCATCAACCAGA
ACGCCCAGTTCATCGGCAGCTTCTACTACAACGACCTGATAGAGTTCGACGGCGAG
CTGTACCGCGTGATAGGCATCAACAACGGCGACAAGAACCTGGTGGAGCTGGACAT
GGTGGACATCCGCTACAAGGAGTACTGCGAGCTGAACAGCATCACCACCACCCCCC
 
GCATCGTGAAGACCATCAGCCCCAAGACCCAGAGCATCGAGAAGTACACCACCGA
CATCCTGGGCAACCTGTACAAGGCCCAGCCCGGCAAGAAGCCCCAGTTCATCTTCA
ACAAGGACGAGGAC;
**SEQ ID NO: 112** - the humanized *SlugCas9-HF* gene sequence:

ATGAACCAAAAATTCATACTGGGACTGGACATCGGAATCACCAGCGTGGGCTACGG
CCTGATCGACTACGAGACAAAGAATATCATCGATGCCGGCGTTAGACTGTTCCCCGA
GGCCAACGTGGAAAACAACGAGGGAAGAAGGTCCAAACGTGGAAGCAGAAGACT
GAAGCGACGCCGCATTCACAGACTTGAACGGGTGAAGAAGCTGCTCGAGGATTATA
ATCTGCTGGATCAGTCCCAGATTCCTCAGTCTACAAACCCCTACGCCATCCGCGTGA
AGGGCCTGTCTGAAGCCCTGAGCAAGGACGAACTCGTGATTGCCCTGCTCCATATC
GCCAAGAGAAGAGGCATCCACAAGATCGACGTGATCGACAGCAACGACGACGTGG
GGAACGAGCTCAGCACCAAGGAACAGCTGAATAAGAACAGCAAGCTGCTGAAAG
ACAAATTTGTGTGCCAGATCCAGCTGGAAAGAATGAATGAGGGCCAGGTGCGGG
AGAGAAAAACCGGTTCAAGACCGCTGATATCATCAAGGAAATCATCCAGCTGCTGA
ATGTGCAGAAGAACTTCCACCAGCTGGACGAGAACTTCATCAACAAGTACATCGAA
CTGGTTGAGATGAGACGGGAATACTTCGAGGGCCCCGGCAAGGGCAGTCCATATGG
CTGGGAAGGCGACCCTAAGGCTTGGTACGAGACACTGATGGGCCACTGCACCTACT
TCCCAGATGAGCTGgctAGCGTGAAATACGCCTACAGTGCCGACCTGTTCAACGCTC
TGAACGACCTGAACAACCTGGTCATCCAAAGAGATGGACTGTCTAAGCTCGAGTAT
CATGAGAAGTATCACATCATCGAGAACGTGTTCAAGCAGAAGAAGAAACCTACACT
GAAGCAGATCGCCAATGAGATCAATGTCAACCCTGAAGATATCAAGGGCTACAGAA
TCACAAAGTCTGGCAAGCCCCAGTTTACCGAGTTTAAGCTCTACCACGACCTGAAA
AGCGTGCTGTTTGACCAGAGCATCCTGGAGAACGAAGACGTGCTGGACCAGATCG
CTGAGATCCTGACCATCTACCAGGACAAGGATAGCATCAAATCTAAGCTGACGGAA
CTGGACATCCTGCTGAACGAGGAAGATAAGGAAAACATCGCCCAGCTGACTGGCTA
CACCGGGACCCACCGGCTCAGCCTGAAATGCATCCGGCTGGTCCTGGAAGAGCAG
TGGTATTCTAGCCGGgcTCAGATGGAAATCTTCgccCACCTGAACATTAAGCCTAAGA
AGATCAACCTGACAGCCGCCAACAAGATCCCGAAGGCTATGATCGACGAGTTCATC
CTGAGCCCTGTGGTGAAGAGGACCTTCGGCCAGGCCATTAACCTTATTAACAAGAT
CATAGAAAAGTACGGCGTGCCTGAAGATATCATCATCGAGCTGGCCAGAGAAAATA

ATAGCAAGGACAAGCAGAAGTTCATCAATGAGATGCAGAAAAAGAACGAGAACAC
CAGAAAGAGAATTAACGAAATCATCGGCAAGTATGGCAACCAGAACGCCAAGAGA
CTGGTCGAGAAGATTAGACTGCACGACGAGCAGGAGGGCAAGTGCCTGTACTCAC
TGGAAAGCATCCCTCTGGAGGACCTGCTGAACAACCCCAACCACTACGAGGTGGA
CCACATCATTCCAAGATCTGTGTCCTTCGACAACTCTTACCACAACAAAGTGCTCGT
GAAGCAGAGCGAGAACTCCAAAAAATCCAACCTGACCCCTTACCAGTACTTTAACA
GCGGCAAGTCCAAGCTCTCTTACAACCAGTTTAAACAACACATCCTGAACCTGAGC
AAGTCCCAGGATAGAATCAGCAAAAAAAAGAAAGAGTATCTGCTGGAAGAACGGG
ACATCAACAAGTTCGAGGTGCAAAAAGAGTTCATCAATAGAAACCTGGTGGATACC
CGGTACGCCACAgccGAGCTGACAAACTACCTGAAGGCCTACTTCAGCGCCAACAAT
ATGAACGTGAAGGTGAAAACGATCAACGGCAGCTTCACCGATTACCTGCGGAAAG
TGTGGAAGTTTAAGAAGGAACGGAACCACGGCTACAAGCACCACGCCGAGGACGC
CCTGATTATCGCTAATGCCGATTTCCTGTTCAAAGAGAACAAGAAGCTGAAAGCCG
TGAACTCTGTGCTGGAAAAACCTGAGATCGAGAGCAAGCAGCTGGATATCCAGGTG
GATAGCGAGGATAACTACAGCGAAATGTTCATCATCCCTAAGCAGGTCCAGGACATC
AAGGACTTCAGAAACTTCAAGTACAGCCACAGAGTGGACAAGAAGCCTAACAGAC
AGCTGATCAACGATACACTGTACAGCACCCGGAAGAAGGACAACTCCACCTACATC
GTGCAGACCATCAAAGATATCTATGCCAAAGATAATACCACCCTGAAGAAGCAGTTT
GACAAGTCACCCGAGAAGTTCCTCATGTACCAACACGATCCGCGGACCTTCGAGAA
GTTGGAAGTGATCATGAAGCAGTACGCTAATGAGAAGAATCCTCTGGCCAAGTACC
ACGAGGAAACAGGCGAGTACCTGACCAAATACAGCAAAAAAAACAACGGCCCTAT
CGTGAAAAGCCTGAAGTACATTGGAAACAAGCTGGGCAGCCACCTAGATGTGACC
CACCAGTTCAAGAGCAGCACCAAGAAGTTGGTGAAGCTGAGCATCAAGCCTTATA
GATTCGACGTCTACCTGACCGACAAGGGATATAAGTTCATCACCATCAGCTACCTGG
ACGTGCTGAAGAAAGACAATTACTACTACATACCCGAACAGAAGTACGACAAGCTC
AAACTGGGCAAGGCCATCGACAAAAACGCCAAGTTTATCGCTAGCTTCTACAAGAA
TGATCTGATCAAGCTGGACGGCGAGATCTACAAGATCATCGGCGTGAATAGCGACA
CCAGAAACATGATCGAACTGGATCTGCCTGACATCAGATACAAAGAATACTGCGAG
CTGAACAATATCAAGGGCGAACCTAGAATCAAAAAGACCATCGGCAAAAAGGTGA
ATAGCATCGAAAAACTGACAACCGACGTGCTGGGCAACGTGTTCACCAACACCCA

GTACACAAAACCTCAGCTGCTGTTCAAGCGAGGAAAT.

In one embodiment, the expression vector can be a plasmid vector, a retroviral vector, an adenovirus vector, an adeno-associated virus vector such as pAAV2ITR, and the like. However, it can be understood that any other suitable expression vectors are also feasible.

In the present disclosure, any sgRNA for targeting can be designed for the DNA sequence to be edited according to actual needs, and the modification as known in the art can be made to the sgRNA to a certain extent. Therefore, in one embodiment, the modification to the sgRNA comprises, but is not limited to, phosphorylation, truncation, addition, sulfurization, methylation, and hydroxylation.

In the present disclosure, any mismatch sgRNA can be designed for the DNA sequence to be edited according to actual needs, and the modification as known in the art can be made to the sgRNA to a certain extent. The modification comprises, but is not limited to phosphorylation, truncation, addition, sulfurization, methylation, and hydroxylation.

In one embodiment, in step (3), the CRISPR/Cas9 system delivered to the cell containing the target site can comprises, but is not limited to, a plasmid, a retrovirus, an adenovirus, or an adeno-associated virus vector expressing the Cas9 protein and sgRNA of the present disclosure, or the sgRNA and the protein *per se,* depending on the actual needs.

In one further embodiment, in step (3), the delivery means comprise, but are not limited to, liposomes, cationic polymers, nanoparticles, multifunctional envelope-type nanoparticles, and viral vectors.

In one further embodiment, in step (3), the cell comprises, but is not limited to, eukaryotic cells and prokaryotic cells. The eukaryotic cells comprise, for example, mammalian cells and plant cells. The mammalian cells comprise, for example, animal cells, such as Chinese hamster ovary cells, baby hamster kidney cells, mouse Sertoli cells, mouse breast tumor cells, buffalo rat hepatic cells, rat hepatoma cells, SV40-transformed monkey kidney CVI lines, monkey kidney cells, canine kidney cells; and human cells, such as human cervical cancer cells, human lung cells, human liver cells, HIH/3T3 cells, human U2-OS osteosarcoma cells, human A549 cells, human K562 cells, human HEK293 cells, human HEK293T cells, human HCT116 cells, or human MCF-7 cells or TRI cells.

In one further embodiment, the modification in step (2) comprises, but is not limited to, phosphorylation, truncation, addition, sulfurization, or methylation.

In one particular embodiment, for other Cas9 genes than the *SlugCas9-HF* gene, the Oligo-F is SEQ ID NO: 16 and the Oligo-R is SEQ ID NO: 17; and for the *SlugCas9-HF* gene, the Oligo-F and the Oligo-R comprise a first oligo forward-strand sequence (Oligo-F1) and a first oligo reverse-strand sequence (Oligo-Rl) represented by SEQ ID NO: 59 and SEQ ID NO: 60, respectively, and a second oligo forward-strand sequence (Oligo-F2) and a second oligo reverse-strand sequence (Oligo-R2) represented by SEQ ID NO: 61 and SEQ ID NO: 62, respectively.

As can be understood by those skilled in the art, the Oligo-F sequence and the Oligo-R sequence need to be annealed to become a double-stranded DNA. Therefore, in one embodiment, the annealing reaction comprises: 1 µL of 100 µM the oligo-F sequence, 1 µL of 100 µM the oligo-R sequence, and 28 µL of water. After shaking and mixing, the annealing reaction is placed in a PCR amplifier to run the annealing program as follows: 95°C for 5 min, 85°C for 1 min, 75°C for 1 min, 65°C for 1 min, 55°C for 1 min, 45°C for 1 min, 35 °C for 1 min, 25°C for 1 min, and 4°C for ever, with a cooling rate of 0.3°C/s.

In one embodiment, the expression vector cloned with Cas9, such as the plasmid pAAV2_Cas9_ITR, needs to be linearized with restriction endonuclease such as Bsal.

In one particular embodiment, the annealed product of the Oligo-F sequence and the Oligo-R sequence is ligated with the linearized expression vector cloned with Cas9, such as the pAAV2_Cas9_ITR backbone vector, via DNA ligase. In this way, an expression vector cloned with both Cas9 and sgRNA, such as pAAV2_Cas9-hU6-sgRNA, is obtained. In one more particular embodiment, the pAAV2_Cas9-hU6-sgRNA is an adeno-associated virus backbone plasmid, comprising AAV2 ITR, a CMV enhancer, a CMV promoter, SV40 NLS, Cas9, nucleoplasmin NLS, 3x HA, bGH poly(A), a human U6 promoter, a BsaI endonuclease site, and a sgRNA scaffold sequence.

In one particular embodiment, the ligation product is transformed into competent cells, and then subjected to Sanger sequencing for correct clone verification. Then the plasmid is extracted for use.

In one particular embodiment, for other Cas9 genes than the *SlugCas9-HF* gene, the cells in step (3) are HEK293T cells which contain a target site having the nucleotide sequence represented by SEQ ID NO: 18; and for the *SlugCas9-HF* gene, the target sites in the cell in step (3) have nucleotide sequences represented by SEQ ID NO: 63 and SEQ ID NO: 64, respectively.

In one particular embodiment, the delivery means in step (3) is a liposome, comprising, for example, Lipofectamine^{®} 2000 or PEI.

In one optional embodiment, the method further comprises step (4) of detecting the editing efficiency for the edited target site, for example, by PCR amplification of the edited target site, followed by T7EI digestion or next-generation sequencing.

In one more particular embodiment, the template for PCR amplification in step (4) is edited genome DNA in HEK293T cells.

In one particular embodiment, in step (4), for other Cas9 genes than the *SlugCas9-HF* gene, the primer sequences for PCR amplification are represented by SEQ ID NO: 19, SEQ ID NO: 20, SEQ ID NO: 21, and SEQ ID NO: 22; and for the *SlugCas9-HF* gene, the primer sequences for PCR amplification are represented by SEQ ID NO: 65, SEQ ID NO: 66, SEQ ID NO: 21, SEQ ID NO: 22, and SEQ ID NO: 67.

In the third aspect, the present disclosure further provides a kit of a CRISPR/Cas9 gene editing system for gene editing, the kit comprising:
(1) a Cas9 protein and a sgRNA, wherein the Cas9 protein is:
   a *SauriCas9* protein having an amino acid sequence represented by SEQ ID NO: 1,
   a ShaCas9 protein having an amino acid sequence represented by SEQ ID NO: 2,
   a *Slug*Cas9 protein having an amino acid sequence represented by SEQ ID NO: 3,
   a *Slut*Cas9 protein having an amino acid sequence represented by SEQ ID NO: 4,
   a *Sa-Sauri*Cas9 protein having an amino acid sequence represented by SEQ ID NO: 5,
   a *Sa-Sep*Cas9 protein having an amino acid sequence represented by SEQ ID NO: 6,
   a *Sa-Seq*Cas9 protein having an amino acid sequence represented by SEQ ID NO: 7,
   a *Sa-Sha*Cas9 protein having an amino acid sequence represented by SEQ ID NO: 8,
   a *Sa-Slug*Cas9 protein having an amino acid sequence represented by SEQ ID NO: 9,
   a *Sa-Slut*Cas9 protein having an amino acid sequence represented by SEQ ID NO: 10, or
   a *Slug*Cas9*-HF* protein having an amino acid sequence represented by SEQ ID NO: 58, or
   the Cas9 protein having an amino acid sequence that is at least 80% identical to the amino acid sequence represented by any one of SEQ ID NOs: 1-10 and SEQ ID NO: 58; and
   the sgRNA has a nucleotide sequence represented by SEQ ID NO: 11, or is a modified sgRNA sequence based on SEQ ID NO: 11;
      or
(2) an expression vector cloned with a Cas9 gene sequence and an oligo single-stranded DNA corresponding to the sgRNA, wherein
   the Cas9 gene sequence:
      (a) has a nucleotide sequence encoding an amino acid sequence represented by any one of SEQ ID NO: 1-10 and SEQ ID NO: 58;
      (b) has a nucleotide sequence encoding an amino acid sequence that is at least 80% identical to the amino acid sequence represented by any one of SEQ ID NO: 1-10 and SEQ ID NO: 58; or
      (c) is a humanized Cas9 gene sequence, for example, a nucleotide sequence represented by any one of SEQ ID NOs: 23-32 and SEQ ID NO: 112; and
   the sgRNA has a nucleotide sequence represented by SEQ ID NO: 11, or a modified sgRNA sequence based on SEQ ID NO: 11.

In the fourth aspect, the present disclosure provides use of the CRISPR/Cas9 system for gene editing according to the first aspect of the present disclosure in gene knockout, site-directed base change, site-directed insertion, regulation of gene transcription levels, regulation of DNA methylation, modification of DNA acetylation, modification of histone acetylation, single-base edition, or chromatin imaging tracking.

Compared with the existing CRISPR/Cas9 gene editing system in the prior art, the CRISPR/Cas9 gene editing system of the present disclosure comprises a smaller Cas9 protein with less amino acids than the prior art, and thus can be effectively packaged. Furthermore, the CRISPR/Cas9 gene editing system of the present disclosure can recognize a relatively simple PAM sequence, and thus can target more DNA sequences in the genome and has higher editing efficiency.

Hereinafter, the present disclosure will be described in more detail through the following examples with reference to the accompanying drawings. It should be understood that, unless otherwise specified, the reagents, methods, and devices used in the present disclosure are all conventional reagents, methods, and devices in the technical field. Unless otherwise specified, the reagents and materials used in the following examples are all commercially available. Experimental methods for which specific conditions are not indicated herein are usually carried out under the conditions conventional or recommended by the manufacturer(s).

### Example 1. Construction of plasmid pAAV2_Cas9-ITR

Step (1): The amino acid sequences were downloaded according to the accession numbers of the Cas9 genes on UniProt.

In the present disclosure, the amino acid sequences for *Sauri*Cas9 gene, *Sha*Cas9 gene, *Slug*Cas9 gene, *Slut*Cas9 gene, *Sa-Sauri*Cas9 gene, *Sa-Sep*Cas9 gene, *Sa-Seq*Cas9 gene, *Sa-Sha*Cas9 gene, *Sa-Slug*Cas9 gene and *Sa-Slut*Cas9 gene were downloaded according to the accession numbers of these genes on UniProt. The accession numbers of the Cas9 genes on UniProt and the amino acid sequences thereof are as follows.

| **Cas9 gene** | **accession number** | **amino acid sequence number** |
|---|---|---|
| *Sauri*Cas9 | A0A2T4M4R5 | SEQ ID NO: 1 |
| *Sha*Cas9 | A0A2T4SLN6 | SEQ ID NO: 2 |
| *Slug*Cas9 | A0A133QCR3 | SEQ ID NO: 3 |
| *Slut*Cas9 | A0A1W6BMI2 | SEQ ID NO: 4 |
| *Sa-Sauri*Cas9 | A0A2T4M4R5 | SEQ ID NO: 5 |
| *Sa-Sep*Cas9 | A0A1Q9MLU4 | SEQ ID NO: 6 |
| *Sa-Seq*Cas9 | A0A1E5TL62 | SEQ ID NO: 7 |
| *Sa-Sha*Cas9 | A0A2T4SLN6 | SEQ ID NO: 8 |
| *Sa-Slug*Cas9 | A0A133QCR3 | SEQ ID NO: 9 |
| *Sa-Slut*Cas9 | A0A1W6BMI2 | SEQ ID NO: 10 |
| *SlugCas9-HF* | A0A133QCR3 | SEQ ID NO: 58^{∗} |

| | | |
|---|---|---|
| ^{∗}Compared with SEQ ID NO: 3, R247A, N415A, T421A, and R656A mutations were introduced in SEQ ID NO: 58. | | |

Step (2): The nucleotide sequences encoding the Cas9 proteins as specified above were subjected to codon optimization to obtain the coding sequences that highly express the Cas9 proteins in human cells. The coding sequences that highly express *Sauri*Cas9 protein, *Sha*Cas9 protein, *Slug*Cas9 protein, *Slut*Cas9 protein, *Sa-Sauri*Cas9 protein *Sa-Sep*Cas9 protein, *Sa-Seq*Cas9 protein, *Sa-Sha*Cas9 protein, *Sa-Slug*Cas9 protein and *Sa-Slut*Cas9 protein in human cells are represented by SEQ ID NOs: 23-32 and SEQ ID NO: 112, respectively.

Step (3): The Cas9-coding sequences obtained in step (2) were subjected to gene synthesis and constructed into the pAAV2_ITR backbone plasmid to obtain the plasmids pAAV2_Cas9_ITR, as shown in Figure 2.

### Example 2. Preparation of linearized plasmids pAAV2_Cas9-ITR

Step (1): The plasmids pAAV2_Cas9_ITR obtained in Example 1 were linearized by digestion with BsaI restriction endonuclease. The digestion mixtures each comprised 1 µg a plasmid pAAV2_Cas9_ITR, 5 µL 10 x CutSmart buffer, 1 µL BsaI endonuclease, ddH₂O to 50 µL. The digestion mixtures were allowed to react at 37°C for 1 hour.

Step (2): The digested products were subjected to electrophoresis on 1% agarose gel at 120 V for 30 minutes.

Step (3): The DNA fragments were cut, recovered by using a kit for gel recovery according to the instructions provided by the manufacturer, and finally eluted with ddH₂O. The DNA fragments as recovered were exactly the linearized plasmids pAAV2_Cas9_ITR comprising *SauriCas9, ShaCas9, SlugCas9, SlutCas9, Sa-Sauri*, *Sa-SepCas9*, *Sa-SeqCas9*, *Sa-ShaCas9*, *Sa-SlugCas9*, *Sa-Slut*Cas9 and *SlugCas9-HF* with a size of 7447 bp, 7430 bp, 7427 bp, 7437 bp, 7433 bp, 7430 bp, 7423 bp, 7430 bp, 7430 bp, 7433 bp and 7427 bp, respectively.

Step (4): The recovered linearized plasmids pAAV2_Cas9_ITR were measured for the DNA concentration by using NanoDrop, and were stored for further use or were placed at -20°C for long-term storage.

### Example 3. Construction of plasmids pAAV2_Cas9_hU6_sgRNA

Step (1): The sgRNA sequence was designed.

Step (2): The sticky end sequences corresponding to both ends of the linearized plasmids pAAV2_Cas9_ITR were added to the sense strand and antisense strand corresponding to the designed sgRNA sequence, and oligo single-stranded DNAs were synthesized.

For genes other than *SlugCas9-HF,* the particular sequences of the oligo single-stranded DNAs were as follows:
**Oligo-F:** CACCGCTCGGAGATCATCATTGCG (SEQ ID NO: 16); and
**Oligo-R:** AAACCGCAATGATGATCTCCGAGC (SEQ ID NO: 17).

Additionally, for *SlugCas9-HF,* the particular sequences of the oligo single-stranded DNAs were as follows:
**Oligo-Fl:** CACCAGAGTAGGCTGGTAGATGGAG (SEQ ID NO: 59);
**Oligo-Rl:** AAACCTCCATCTACCAGCCTACTCT (SEQ ID NO: 60);
**Oligo-F2:** CACCGTCAGACATGAGATCACAGAT (SEQ ID NO: 61);
**Oligo-R2:** AAACATCTGTGATCTCATGTCTGAC (SEQ ID NO: 62).

Step (3): The oligo single-stranded DNAs were annealed to become double-stranded DNAs. The annealing reactions each comprised: 1 µL of 100 µM oligo-F, 1 µL of 100 µM oligo-R, and 28 µL ddH₂O. After being mixed by shaking, the annealing reactions were placed in a PCR amplifier to run the annealing program as follows: 95°C for 5 min, 85°C for 1 min, 75°C for 1 min, 65°C for 1 min, 55°C for 1 min, 45°C for 1 min, 35°C for 1 min, 25°C for 1 min, and at 4°C for ever, with a cooling rate of 0.3°C/s.

Step (4): The annealed products were ligated with the linearized plasmids pAAV2_Cas9_ITR obtained in Example 2 by using DNA ligase according to the instructions provided by the manufacturer.

Step (5): The ligated products 1 µL were taken for chemically competent transformation, and the grown bacterial clones were verified by Sanger sequencing.

Step (6): The correctly ligated clones verified by sequencing were cultured under shaking, and then used to extract the plasmids pAAV2_Cas9-hU6-sgRNA for further use.

### Example 4. Transfection of HEK293T cell line with plasmids pAAV2_Cas9-hU6-sgRNA expressing Cas9 protein and sgRNA

### 1. Transfection of the GFP-reporter HEK293T cell line with the plasmids pAAV2_Cas9-hU6-sgRNA

Step (1): On day 0, according to the requirements of transfection, the GFP-reporter HEK293T cell line was plated on a 6-well plate at a cell density of about 30%. The sequence of the target site is represented by SEQ ID NO: 18 (GCTCGGAGATCATCATTGCGNNNNN).

Step (2): On day 1, transfection was performed by the following steps:
i. Plasmids to be transfected, pAAV2_Cas9-hU6-sgRNA, 2 µg, were taken and added into 100 µL Opti-MEM medium, and mixed homogenously by gently pipetting;
ii. 5 µL of flick mixed Lipofectamine^{®} 2000 was pipetted to 100 µL Opti-MEM medium, mixed gently, and left to stand at room temperature for 5 min; and
iii. the diluted Lipofectamine^{®} 2000 and the diluted plasmid were mixed homogenously by gently pipetting, left to stand at room temperature for 20 minutes, and then added into the medium containing the cells to be transfected. The cells to be transfected contained the CMV-ATG-target site-NNNNNNN-GFP nucleotide sequence represented by SEQ ID NO: 113, which comprises the sequence represented by SEQ ID NO: 18. It should be noted that the nucleotide sequence has 7 random bases N as the PAM sequence (marked in bold and underlined) between the target site and the GFP sequence.

Step (3): The cells were continued to be cultured in a 37°C, 5% CO₂ incubator.

Step (4): After being edited for 3 days, GFP-positive cells were sorted out by flow sorting, and continued to be cultured in a 37°C, 5% CO₂ incubator.

The sequence represented by SEQ ID NO: 113 is as follows:

GACATTGATTATTGACTAGTTATTAATAGTAATCAATTACGGGGTCATTAGTTCATAGC
CCATATATGGAGTTCCGCGTTACATAACTTACGGTAAATGGCCCGCCTGGCTGACCG
CCCAACGACCCCCGCCCATTGACGTCAATAATGACGTATGTTCCCATAGTAACGCCA
ATAGGGACTTTCCATTGACGTCAATGGGTGGAGTATTTACGGTAAACTGCCCACTTG
GCAGTACATCAAGTGTATCATATGCCAAGTACGCCCCCTATTGACGTCAATGACGGT
AAATGGCCCGCCTGGCATTATGCCCAGTACATGACCTTATGGGACTTTCCTACTTGG
CAGTACATCTACGTATTAGTCATCGCTATTACCATGGTGATGCGGTTTTGGCAGTACA
TCAATGGGCGTGGATAGCGGTTTGACTCACGGGGATTTCCAAGTCTCCACCCCATTG
ACGTCAATGGGAGTTTGTTTTGGCACCAAAATCAACGGGACTTTCCAAAATGTCGT
AACAACTCCGCCCCATTGACGCAAATGGGCGGTAGGCGTGTACGGTGGGAGGTCTA
TATAAGCAGTCTAGAGATCCGACGCCGCCATCTCTAGGCCCGCGCCGGCCCCCTCGC
ACAGACTTGTGGGAGAAGCTCGGCTACTCCCCTGCCCCGGTTAATTTGCATATAATA
TTTCCTAGTAACTATAGAGGCTTAATGTGCGATAAAAGACAGATAATCTGTTCTTTTT
AATACTAGCTACATTTTACATGATAGGCTTGGATTTCTATAAGAGATACAAATACTAA
ATTATTATTTTAAAAAACAGCACAAAAGGAAACTCACCCTAACTGTAAAGTAATTGT
GTGTTTTGAGACTATAAATATGCATGCGAGAAAAGCCTTGTTTGCCACCATGGAACG
GCTCGGAGATCATCATTGCGNNNNNNNGTGAGCAAGGGCGAGGAGCTGTTCACCG

GGGTGGTGCCCATCCTGGTCGAGCTGGACGGCGACGTAAACGGCCACAAGTTCAG
CGTGTCCGGCGAGGGCGAGGGCGATGCCACCTACGGCAAGCTGACCCTGAAGTTC
ATCTGCACCACCGGCAAGCTGCCCGTGCCCTGGCCCACCCTCGTGACCACCCTGAC
CTACGGCGTGCAGTGCTTCAGCCGCTACCCCGACCACATGAAGCAGCACGACTTCT
TCAAGTCCGCCATGCCCGAAGGCTACGTCCAGGAGCGCACCATCTTCTTCAAGGAC
GACGGCAACTACAAGACCCGCGCCGAGGTGAAGTTCGAGGGCGACACCCTGGTGA
ACCGCATCGAGCTGAAGGGCATCGACTTCAAGGAGGACGGCAACATCCTGGGGCA
CAAGCTGGAGTACAACTACAACAGCCACAACGTCTATATCATGGCCGACAAGCAGA
AGAACGGCATCAAGGTGAACTTCAAGATCCGCCACAACATCGAGGACGGCAGCGT
GCAGCTCGCCGACCACTACCAGCAGAACACCCCCATCGGCGACGGCCCCGTGCTG
CTGCCCGACAACCACTACCTGAGCACCCAGTCCAAGCTGAGCAAAGACCCCAACG
AGAAGCGCGATCACATGGTCCTGCTGGAGTTCGTGACCGCCGCCGGGATCACTCTC
GGCATGGACGAGCTGTACAAG

### 2. Transfection of HEK293T cell line with pAAV2_SlugCas9-HF-hU6-sgRNA

Step (1): On day 0, according to the requirements of transfection, the HEK293T cell line containing the sgRNA target site was plated on a 6-well plate at a cell density of about 30%. The sequences of the G4 and G7 target sites for *SlugCas9-HF* are represented by SEQ ID NO: 63 (AGAGTAGGCTGGTAGATGGAGNNNN) and SEQ ID NO: 64 (ATCTGTGATCTCATGTCTGACNNNN), respectively.

Step (2): On day 1, transfection was performed via the following steps:
i. 2 µg of the plasmid to be transfected, pAAV2_SlugCas9-HF-hU6-sgRNA, was taken and added into 100 µL Opti-MEM medium, and mixed homogenously by gently pipetting;
ii. 5 µL of flick mixed Lipofectamine^{®} 2000 was pipetted to 100 µL Opti-MEM medium, mixed gently, and left to stand at room temperature for 5 min; and
iii. The diluted Lipofectamine^{®} 2000 and the diluted plasmid were mixed homogenously by gently pipetting, left to stand at room temperature for 20 minutes, and then added into the medium containing the cells to be transfected.

Step (3): The cells were continued to be cultured in a 37°C, 5% CO₂ incubator.

### Example 5. Preparation of next-generation sequencing library

Step (1): HEK293T cells after being edited for 3 days or the GFP-reporter HEK293T cell line after flow sorting were collected and used to extract the genome DNA by using a DNA kit according to the instructions provided by the manufacturer.

Step (2): The first round of PCR for PCR library construction was performed with 2xQ5 Mastermix. For genes other than *SlugCas9-HF,* the PCR primers are represented by SEQ ID NO: 19 and SEQ ID NO: 20; and for *SlugCas9-HF,* the PCR primers are represented by SEQ ID NO: 65 and SEQ ID NO: 66, as follows:

F1-ACACTCTTTCCCTACACGACGCTCTTCCGATCTNNNNGCGAGAAAAGCC
TTGTTT (SEQ ID NO: 19);

R1-ACTGGAGTTCAGACGTGTGCTCTTCCGATCTCTGAACTTGTGGCCGTTTA
C (SEQ ID NO: 20);

F1-ACACTCTTTCCCTACACGACGCTCTTCCGATCTNNNNTGTCAGGCAGCA
GAGCTC (SEQ ID NO: 65);

R1-ACTGGAGTTCAGACGTGTGCTCTTCCGATCTNNNNGGCGATGGCTTCCT
GGTC (SEQ ID NO: 66).

The reaction was as follows:

| **Reagents** | **25 µL reaction** | | |
|---|---|---|---|
| 10 µM F1 | 1.25 µL | | |
| 10 µM R1 | 1.25 µL | | |
| 2X Q5 master mix | 12.5 µL | | |
| genomic DNA | 5 µg | | |
| ddH₂O | to 25 µL | | |

The PCR procedure was as follows:

| | | | |
|---|---|---|---|
| Initial denaturation | | 98°C | 2 min |
| | | 95°C | 7 s |
| 35 cycles | | 65°C | 20 s |
| | | 72°C | 10 s |
| Final extension | | 72°C | 2 min |
| Hold | | 4°C | ∞ |

Step (3): The second round of PCR for PCR library construction was performed with 2xQ5 Mastermix. For genes other than *SlugCas9-HF,* the PCR primers are represented by SEQ ID NO: 21 and SEQ ID NO: 22; and for *SlugCas9-HF,* the products from the first round of PCR for the G4 site were amplified with the primers represented by SEQ ID NO: 21 and SEQ ID NO: 22; and the products from the first round of PCR for the G7 site were amplified with the primers represented by SEQ ID NO: 21 and SEQ ID NO: 67.
F2-AATGATACGGCGACCACCGAGATCTACACTCTTTCCCTACACGAC (SEQ ID NO: 21)
R2-CAAGCAGAAGACGGCATACGAGATCACTGTGTGACTGGAGTTCAGACGTGTG (SEQ ID NO: 22)
F2-AATGATACGGCGACCACCGAGATCTACACTCTTTCCCTACACGAC (SEQ ID NO: 21)
R2-1-CAAGCAGAAGACGGCATACGAGATCACTGTGTGACTGGAGTTCAGACGTGTG (SEQ ID NO: 22)
F2-AATGATACGGCGACCACCGAGATCTACACTCTTTCCCTACACGAC (SEQ ID NO: 21)
R2-2-CAAGCAGAAGACGGCATACGAGATATTGGCGTGACTGGAGTTCAGACGTGTG (SEQ ID NO: 67)

The reaction was as follows:

| **Reagents:** | **25 µL reaction** | | |
|---|---|---|---|
| 10 µM F2 | 1.25 µL | | |
| 10 µM R2 | | | 1.25 µL |
| 2X Q5 master mix | | | 12.5 µL |
| The products from the first round | | | 3 µL |
| ddH₂O | | | 7 µL |

The PCR procedure was as follows

| | | | |
|---|---|---|---|
| Initial denaturation | | 98°C | 30 s |
| 15 cycles | | 95°C | 7 s |
| | | 65°C | 20 s |
| | | 72°C | 10 s |
| Final extension | | 72°C | 2 min |
| Hold | | 4°C | ∞ |

Step (4): The products from the second round of PCR were purified with a kit for gel recovery according to the instructions provided by the manufacturer to obtain the DNA fragments with a size of 366 bp or 406 bp (the latter is only for *SlugCas9-HF*), thereby completing the preparation of the library for next-generation sequencing.

### Example 6. Analysis of next-generation sequencing results

Step (1): The prepared library for next-generation sequencing was subjected to paired-end sequencing on HiseqXTen.

Step (2): The next-generation sequencing results were analyzed via Bioinformatics. Some of the editing results are shown in Figure 3a to Figure 3j. It can be seen from the figures that the editing results comprise deletions, insertions or mismatches, and the last 4 bp or 5 bp represents the PAM sequence, which is NNGG, NNGRM, NNGG, NNGR, NNGG, NNGG, NNGRM, NNGRM, NNGG and NNGRR, respectively, from Figures 3a to 3j. Figure 3k shows the editing of the *SlugCas9-HF* gene editing system at two target sites, where the X axis represents the G4 and G7 target sites, and the Y axis represents the indel efficiency.

### Example 7. Verification of endogenous sites

Step (1): The plasmids pAAV2_Cas9-hU6-sgRNA expressing Cas9 and sgRNA were transfected into HEK293T cells by Lipofectamine^{®} 2000 according to the instructions provided by the manufacturer. The particular sequences for different Cas9s, crRNAs and target sites are given in Table 1.

Step (2): The genomic DNA in cells which had been edited for 5 days was extracted, and the target DNA sequence was amplified with 2x Q5 Master mix and Test-F and Test-R primers. The detailed sequences of the Test-F and Test-R primers are given in Table 1 below.

Step (3): The PCR products were recovered and purified by agarose gel electrophoresis, to obtain the DNA fragments of different sizes. The sizes of the DNA fragments are shown in Table 1.

Step (4): The purified DNA fragments were digested according to the instructions for T7 Endonuclease I, and then detected by gel electrophoresis.

The results are shown in Figures 4a-4j. In each figure, the left lane corresponds to the negative control group which did not involve sgRNA during transfection, and there was no cleaved fragment after the target sequence was cleaved by T7 Endonuclease I, indicating that no editing has occurred; and the right lane corresponds to the treatment group, which involved sgRNA during transfection, and the cleaved fragments appeared after the target sequence was cleaved by T7 Endonuclease I, indicating that editing has occurred.

**Table 1**

| **Serial number** | **Cas9 genes** | **crRNAs** | **target sites** | **Test-F and Test-R** | **DNA fragments** |
|---|---|---|---|---|---|
| 1 | SauriCas9 | AGATGCGGGTGATGATG CTCT (SEQ ID NO: 33) | AGATGCGGGTGATGATG CTCTTTGG (SEQ ID NO: 40) | CAGGGAGTCGACGAGTTGAA(SEQ ID NO: 47); | 570 bp |
| | | | | TAATTGCTGGCCTATCCACGC(SEQ ID NO: 53) | |
| 2 | ShaCas9 | AGATGCGGGTGATGATG CTCT (SEQ ID NO: 33) | AGATGCGGGTGATGATG CTCTTTGG (SEQ ID NO: 40) | CAGGGAGTCGACGAGTTGAA (SEQ ID NO: 47); | 570 bp |
| | | | | TAATTGCTGGCCTATCCACGC (SEQ ID NO: 53); | |
| 3 | SlugCas9 | ATAGGGTTAGGGGCCCC AGGC (SEQ ID NO: 34) | ATAGGGTTAGGGGCCCC AGGCCGGG (SEQ ID NO: 41) | ACGCAGTGGGTCATAGGCTC (SEQ ID NO: 48); | 509 bp |
| | | | | GGACTCAGGCCCTTCCTCCT (SEQ ID NO: 54) | |
| 4 | SlutCas9 | GGCGCAGTTTACTGCAC AGGT (SEQ ID NO: 35) | GGCGCAGTTTACTGCAC AGGTGCGG (SEQ ID NO: 42) | AGGGAAGAGGAAATGCTGGG (SEQ ID NO: 49); | 276 bp |
| | | | | TGAGCCGCCAGTGTACAGA (SEQ ID NO: 55) | |
| 5 | Sa-SauriCas9 | ATAGGGTTAGGGGCCCC AGGC (SEQ ID NO: 34) | ATAGGGTTAGGGGCCCC AGGCCGGG (SEQ ID NO: 41) | ACGCAGTGGGTCATAGGCTC (SEQ ID NO: 48); | 509 bp |
| | | | | GGACTCAGGCCCTTCCTCCT (SEQ ID NO: 54) | |
| 6 | Sa-SepCas9 | GGGAAGAGTGAGGGGA ACAAA (SEQ ID NO: 36) | GGGAAGAGTGAGGGGA ACAAAGTGG (SEQ ID NO: 43) | TCCTTGAACAGCCTGCAAAC (SEQ ID NO: 50); | 493 bp |
| | | | | AAGGAGGTCTCTGTCTGTGC (SEQ ID NO: 56) | |
| 7 | Sa-SeqCas9 | GAGCTGGTGGACCTAGT ACA (SEQ ID NO: 37) | GAGCTGGTGGACCTAGT ACAATGGA (SEQ ID NO: 44) | ATCAACCCGGAGCAGATTC (SEQ ID NO: 51); | 475 bp |
| | | | | CCTCATTGTCCAGAAAGACCA (SEQ ID NO: 57) | |
| 8 | Sa-ShaCas9 | AGTGAGGGGAACAAAG TGGAC (SEQ ID NO: 38) | AGTGAGGGGAACAAAG TGGACATGGC (SEQ ID NO: 45) | GCTGCTTTCCTGCTGTCTTC (SEQ ID NO: 52); | 284 bp |
| | | | | AAGGAGGTCTCTGTCTGTGC (SEQ ID NO: 56) | |
| 9 | Sa-SlugCas9 | GGGAAGAGTGAGGGGA ACAAA (SEQ ID NO: 36) | GGGAAGAGTGAGGGGA ACAAAGTGG (SEQ ID NO: 43) | TCCTTGAACAGCCTGCAAAC (SEQ ID NO: 50); | 493 bp |
| | | | | AAGGAGGTCTCTGTCTGTGC (SEQ ID NO: 56) | |
| 10 | Sa-SlutCas9 | GCGAGCCTGAGGCGAAC AATG (SEQ ID NO: 39) | GCGAGCCTGAGGCGAAC AATGGCGGA (SEQ ID NO: 46) | AGGGAAGAGGAAATGCTGGG (SEQ ID NO: 49); | 276bp |
| | | | | TGAGCCGCCAGTGTACAGA (SEQ ID NO: 55) | |

### Example 8. Detection of CRISPR/Cas9 system Specificity

In this example, *SlugCas9-HF* was taken as an example to verify the specificity of the CRISPR/Cas9 system. The particular protocols were as follows:
1. Plasmid pAAV2_SlugCas9-HF_ITR was constructed by following the protocols in Example 1.
2. Linearized plasmid pAAV2_SlugCas9-HF_ITR was prepared by following the protocols in Example 2.
3. Plasmids pAAV2_SlugCas9-HF-hU6-on target sgRNA and pAAV2_SlugCas9-HF-hU6-mismatch sgRNA were constructed.

Step (1): The on-target sgRNA sequence and the mismatch sgRNA sequences were designed.

Step (2): The sticky end sequences corresponding to both ends of the linearized plasmid pAAV2_SlugCas9-HF_ITR were added to the sense strand and antisense strand corresponding to the designed on-target sgRNA sequence and the mismatch sgRNA sequence, and oligo single-stranded DNAs were synthesized. The particular sequences are as follows (wherein the bases underlined and in bold are mismatch bases):
Oligo-F3: CACCGGCTCGGAGATCATCATTGCG (SEQ ID NO: 68) (on-target)
Oligo-R3: AAACCGCAATGATGATCTCCGAGCC (SEQ ID NO: 89) (on-target)
Oligo-F4: CACC**AA**CTCGGAGATCATCATTGCG (SEQ ID NO: 69) (mismatch)
Oligo-F5: CACCG**AT**TCGGAGATCATCATTGCG (SEQ ID NO: 70) (mismatch)
Oligo-F6: CACCGG**TC**CGGAGATCATCATTGCG (SEQ ID NO: 71) (mismatch)
Oligo-F7: CACCGGC**CT**GGAGATCATCATTGCG (SEQ ID NO: 72) (mismatch)
Oligo-F8: CACCGGCT**TA**GAGATCATCATTGCG (SEQ ID NO: 73) (mismatch)
Oligo-F9: CACCGGCTC**AA**AGATCATCATTGCG (SEQ ID NO: 74) (mismatch)
Oligo-F10: CACCGGCTCG**AG**GATCATCATTGCG (SEQ ID NO: 75) (mismatch)
Oligo-F11: CACCGGCTCGG**GA**ATCATCATTGCG (SEQ ID NO: 76) (mismatch)
Oligo-F12: CACCGGCTCGGA**AG**TCATCATTGCG (SEQ ID NO: 77) (mismatch)
Oligo-F13: CACCGGCTCGGAG**GC**CATCATTGCG (SEQ ID NO: 78) (mismatch)
Oligo-F14: CACCGGCTCGGAGA**CT**ATCATTGCG (SEQ ID NO: 79) (mismatch)
Oligo-F15: CACCGGCTCGGAGAT**TG**TCATTGCG (SEQ ID NO: 80) (mismatch)
Oligo-F16: CACCGGCTCGGAGATC**GC**CATTGCG (SEQ ID NO: 81) (mismatch)
Oligo-F17: CACCGGCTCGGAGATCA**CT**ATTGCG (SEQ ID NO: 82) (mismatch)
Oligo-F18: CACCGGCTCGGAGATCAT**TG**TTGCG (SEQ ID NO: 83) (mismatch)
Oligo-F19: CACCGGCTCGGAGATCATC**GC**TGCG (SEQ ID NO: 84) (mismatch)
Oligo-F20: CACCGGCTCGGAGATCATCA**CC**GCG (SEQ ID NO: 85) (mismatch)
Oligo-F21: CACCGGCTCGGAGATCATCAT**CA**CG (SEQ ID NO: 86) (mismatch)
Oligo-F22: CACCGGCTCGGAGATCATCATT**AT**G (SEQ ID NO: 87) (mismatch)
Oligo-F23: CACCGGCTCGGAGATCATCATTG**TA** (SEQ ID NO: 88) (mismatch)
Oligo-R4: AAACCGCAATGATGATCTCCGAG**TT** (SEQ ID NO: 90) (mismatch)
Oligo-R5: AAACCGCAATGATGATCTCCGA**AT**C (SEQ ID NO: 91) (mismatch)
Oligo-R6: AAACCGCAATGATGATCTCCG**GA**CC (SEQ ID NO: 92) (mismatch)
Oligo-R7: AAACCGCAATGATGATCTCC**AG**GCC (SEQ ID NO: 93) (mismatch)
Oligo-R8: AAACCGCAATGATGATCTC**TA**AGCC (SEQ ID NO: 94) (mismatch)
Oligo-R9: AAACCGCAATGATGATCT**TT**GAGCC (SEQ ID NO: 95) (mismatch)
Oligo-R10: AAACCGCAATGATGATC**CT**CGAGCC (SEQ ID NO: 96) (mismatch)
Oligo-R11: AAACCGCAATGATGAT**TC**CCGAGCC (SEQ ID NO: 97) (mismatch)
Oligo-R12: AAACCGCAATGATGA**CT**TCCGAGCC (SEQ ID NO: 98) (mismatch)
Oligo-R13: AAACCGCAATGATG**GC**CTCCGAGCC (SEQ ID NO: 99) (mismatch)
Oligo-R14: AAACCGCAATGAT**AG**TCTCCGAGCC (SEQ ID NO: 100) (mismatch)
Oligo-R15: AAACCGCAATGA**CA**ATCTCCGAGCC (SEQ ID NO: 101) (mismatch)
Oligo-R16: AAACCGCAATG**GC**GATCTCCGAGCC (SEQ ID NO: 102) (mismatch)
Oligo-R17: AAACCGCAAT**AG**TGATCTCCGAGCC (SEQ ID NO: 103) (mismatch)
Oligo-R18: AAACCGCAA**CA**ATGATCTCCGAGCC (SEQ ID NO: 104) (mismatch)
Oligo-R19: AAACCGCA**GC**GATGATCTCCGAGCC (SEQ ID NO: 105) (mismatch)
Oligo-R20: AAACCGC**GG**TGATGATCTCCGAGCC (SEQ ID NO: 106) (mismatch)
Oligo-R21: AAACCG**TG**ATGATGATCTCCGAGCC (SEQ ID NO: 107) (mismatch)
Oligo-R22: AAACC**AT**AATGATGATCTCCGAGCC (SEQ ID NO: 108) (mismatch)
Oligo-R23: AAAC**TA**CAATGATGATCTCCGAGCC (SEQ ID NO: 109) (mismatch).

Step (3): The oligo single-stranded DNAs were annealed to be double-stranded DNAs, and the annealing reactions each comprised: 1 µL of 100 µM oligo-F, 1 µL of 100 µM oligo-R, and 28 µL ddH₂O. After being mixed by shaking, the annealing reactions were placed in a PCR amplifier to run the annealing program as follows: 95°C for 5 min, 85°C for 1 min, 75°C for 1 min, 65°C for 1 min, 55°C for 1 min, 45°C for 1 min, 35°C for 1 min, 25°C for 1 min, and 4°C for ever, with a cooling rate of 0.3°C/s.

Step (4): The annealed products were ligated with the linearized plasmid pAAV2_SlugCas9-HF_ITR by using DNA ligase according to the instructions provided by the manufacturer.

Step (5): The ligated products (1 µL) were taken for chemically competent transformation, and the grown bacterial clones were verified by Sanger sequencing.

Step (6): The correctly ligated clones verified by sequencing were cultured under shaking and then used to extract the plasmids, pAAV2_SlugCas9-HF-hU6-on target sgRNA and pAAV2_SlugCas9-HF-hU6-mismatch sgRNAs for further use.

4. The GFP-reporter HEK293T cell line was transfected with pAAV2_SlugCas9-HF-hU6-on target sgRNA and pAAV2_SlugCas9-HF-hU6-mismatch sgRNAs, respectively. The particular steps are as follows.

Step (1): On day 0, according to the requirements of transfection, the GFP-reporter HEK293T cell line was plated on a 6-well plate at a cell density of about 30%. The sequence of the target site is represented by SEQ ID NO: 110 (GGCTCGGAGATCATCATTGCGNNNN).

Step (2): On day 1, transfection was performed by the following steps:
i. the plasmids to be transfected, pAAV2_SlugCas9-HF-hU6-on target sgRNA/mismatch sgRNA, 2 µg, were taken and added into 100 µL Opti-MEM medium, and mixed homogenously by gently pipetting;
ii. 5µL of flick mixed Lipofectamine^{®} 2000 was pipetted to 100µL Opti-MEM medium, mixed gently, and left to stand at room temperature for 5 min; and
iii. the diluted Lipofectamine^{®} 2000 and the diluted plasmid were mixed homogenously by gently pipetting, left to stand at room temperature for 20 minutes, and then added into the medium containing the cells to be transfected. The cells to be transfected comprised the CMV-ATG-target site-CTGG-GFP nucleotide sequence represented by SEQ ID NO: 111, which comprised the sequence represented by SEQ ID NO: 110.

Step (3): The cells were continued to be cultured in a 37°C, 5% CO₂ incubator.

5. Analysis of the editing efficiency and off-target rate of *SlugCas9-HF* by flow cytometry.

Step (1): The cells edited for 3 days were collected, and were subjected to the flow cytometry.

Step (2): The GFP positive ratio was analyzed by using FlowJo analysis software and plotted.

The sequence represented by SEQ ID NO: 111 is as follows:

GACATTGATTATTGACTAGTTATTAATAGTAATCAATTACGGGGTCATTAGTTCATAGC
CCATATATGGAGTTCCGCGTTACATAACTTACGGTAAATGGCCCGCCTGGCTGACCGC

CCAACGACCCCCGCCCATTGACGTCAATAATGACGTATGTTCCCATAGTAACGCCAAT
AGGGACTTTCCATTGACGTCAATGGGTGGAGTATTTACGGTAAACTGCCCACTTGGC
AGTACATCAAGTGTATCATATGCCAAGTACGCCCCCTATTGACGTCAATGACGGTAAA
TGGCCCGCCTGGCATTATGCCCAGTACATGACCTTATGGGACTTTCCTACTTGGCAGT
ACATCTACGTATTAGTCATCGCTATTACCATGGTGATGCGGTTTTGGCAGTACATCAAT
GGGCGTGGATAGCGGTTTGACTCACGGGGATTTCCAAGTCTCCACCCCATTGACGTC
AATGGGAGTTTGTTTTGGCACCAAAATCAACGGGACTTTCCAAAATGTCGTAACAAC
TCCGCCCCATTGACGCAAATGGGCGGTAGGCGTGTACGGTGGGAGGTCTATATAAGC
AGTCTAGAGATCCGACGCCGCCATCTCTAGGCCCGCGCCGGCCCCCTCGCACAGACT
TGTGGGAGAAGCTCGGCTACTCCCCTGCCCCGGTTAATTTGCATATAATATTTCCTAGT
AACTATAGAGGCTTAATGTGCGATAAAAGACAGATAATCTGTTCTTTTTAATACTAGCT
ACATTTTACATGATAGGCTTGGATTTCTATAAGAGATACAAATACTAAATTATTATTTTA
AAAAACAGCACAAAAGGAAACTCACCCTAACTGTAAAGTAATTGTGTGTTTTGAGA
CTATAAATATGCATGCGAGAAAAGCCTTGTTTGCCACCATGGCTCGGAGATCATCAT
TGCGCTGGGTGAGCAAGGGCGAGGAGCTGTTCACCGGGGTGGTGCCCATCCTGGTC
GAGCTGGACGGCGACGTAAACGGCCACAAGTTCAGCGTGTCCGGCGAGGGCGAGG
GCGATGCCACCTACGGCAAGCTGACCCTGAAGTTCATCTGCACCACCGGCAAGCTG
CCCGTGCCCTGGCCCACCCTCGTGACCACCCTGACCTACGGCGTGCAGTGCTTCAGC
CGCTACCCCGACCACATGAAGCAGCACGACTTCTTCAAGTCCGCCATGCCCGAAGG
CTACGTCCAGGAGCGCACCATCTTCTTCAAGGACGACGGCAACTACAAGACCCGCG
CCGAGGTGAAGTTCGAGGGCGACACCCTGGTGAACCGCATCGAGCTGAAGGGCATC
GACTTCAAGGAGGACGGCAACATCCTGGGGCACAAGCTGGAGTACAACTACAACA
GCCACAACGTCTATATCATGGCCGACAAGCAGAAGAACGGCATCAAGGTGAACTTC
AAGATCCGCCACAACATCGAGGACGGCAGCGTGCAGCTCGCCGACCACTACCAGCA
GAACACCCCCATCGGCGACGGCCCCGTGCTGCTGCCCGACAACCACTACCTGAGCA
CCCAGTCCAAGCTGAGCAAAGACCCCAACGAGAAGCGCGATCACATGGTCCTGCTG
GAGTTCGTGACCGCCGCCGGGATCACTCTCGGCATGGACGAGCTGTACAAG

Figure 4k shows the detection results of the specificity of the *SlugCas9-HF* gene editing system in the GFP-reporter HEK293T cell line. It can be seen from Figure 4 that the complex of *SlugCas9-HF* and the sgRNA specifically cleaved the target sequence (the sequence shown in Figure 1), but was essentially incapable of or was incapable of cleaving the non-target sequences (the sequences shown in Figure 2-21). Accordingly, the system of the present disclosure shows high specificity and a low off-target rate.

## Claims

1. A CRISPR/Cas9 system for gene editing in cells or *in vitro,* wherein the CRISPR/Cas9 system is a complex of a Cas9 protein and a sgRNA, which is capable of accurately locating and cleaving a target DNA sequence so as to cause double-strand break damage to the target DNA sequence;
the Cas9 protein is:
a *Sauri*Cas9 protein having an amino acid sequence represented by SEQ ID NO: 1,
a *Sha*Cas9 protein having an amino acid sequence represented by SEQ ID NO: 2,
a *Slug*Cas9 protein having an amino acid sequence represented by SEQ ID NO: 3,
a *Slut*Cas9 protein having an amino acid sequence represented by SEQ ID NO: 4,
a *Sa-Sauri*Cas9 protein having an amino acid sequence represented by SEQ ID NO: 5,
a *Sa-Sep*Cas9 protein having an amino acid sequence represented by SEQ ID NO: 6,
a *Sa-Seq*Cas9 protein having an amino acid sequence represented by SEQ ID NO: 7,
a *Sa-Sha*Cas9 protein having an amino acid sequence represented by SEQ ID NO: 8,
a *Sa-Slug*Cas9 protein having an amino acid sequence represented by SEQ ID NO: 9,
a *Sa-Slut*Cas9 protein having an amino acid sequence represented by SEQ ID NO: 10, or
a *SlugCas9-HF* protein having an amino acid sequence represented by SEQ ID NO: 58, or
a Cas9 protein having an amino acid sequence that is at least 80% identical to the amino acid sequence represented by any one of SEQ ID NOs: 1-10 and SEQ ID NO: 58; and
the sgRNA has a nucleotide sequence represented by SEQ ID NO: 11, or is a modified sgRNA sequence based on SEQ ID NO: 11.

2. The CRISPR/Cas9 system for gene editing according to claim 1, wherein the cell comprises eukaryotic cells and prokaryotic cells; the eukaryotic cells comprise, for example, mammalian cells and plant cells; the mammalian cells comprise, for example, Chinese hamster ovary cells, baby hamster kidney cells, mouse Sertoli cells, mouse breast tumor cells, buffalo rat hepatic cells, rat hepatoma cells, SV40-transformed monkey kidney CVI lines, monkey kidney cells, canine kidney cells, human cervical cancer cells, human lung cells, human liver cells, HIH/3T3 cells, human U2-OS osteosarcoma cells, human A549 cells, human K562 cells, human HEK293 cells, human HEK293T cells, human HCT116 cells, or human MCF-7 cells or TRI cells.

3. The CRISPR/Cas9 system for gene editing according to claim 1, wherein the Cas9 protein comprises a Cas9 protein that has no cleavage activity, or only has single-stranded cleavage activity, or has double-stranded cleavage activity.

4. The CRISPR/Cas9 system for gene editing according to claim 1, wherein accurately locating the target DNA sequence comprises forming a complementary base pairing structure from a 20 bp or 21 bp sequence at the 5' end of the sgRNA and the target DNA sequence.

5. The CRISPR/Cas9 system for gene editing according to claim 1, wherein accurately locating the target DNA sequence comprises recognizing, by the complex of the Cas9 protein and the sgRNA, a PAM sequence on the target DNA sequence.

6. The CRISPR/Cas9 system for gene editing according to claim 1, wherein a 20 bp or 21 bp sequence at the 5' end of the sgRNA in the complex of the *SlugCas9-HF* protein and the sgRNA is capable of forming an imperfect complementary base pairing structure with a non-target DNA sequence; preferably, the non-target sequence has two or more base mismatches to the sgRNA.

7. The CRISPR/Cas9 system for gene editing according to claim 6, wherein the complex of the Cas9 protein and the sgRNA is capable of recognizing a PAM sequence on the non-target DNA sequence.

8. The CRISPR/Cas9 system for gene editing according to any one of claims 5-7, wherein:
for the *Sauri*Cas9 protein, the PAM is NNGG, and the target DNA sequence is represented by SEQ ID NO: 12;
for the *Sha*Cas9 protein, the PAM is NNGRM, and the target DNA sequence is represented by SEQ ID NO: 13;
for the *Slug*Cas9 protein, the PAM is NNGG, and the target DNA sequence is represented by SEQ ID NO: 12;
for the *Slut*Cas9 protein, the PAM is NNGR, and the target DNA sequence is represented by SEQ ID NO: 14;
for the *Sa-Sauri*Cas9 protein, the PAM is NNGG, and the target DNA sequence is represented by SEQ ID NO: 12;
for the *Sa-Sep*Cas9 protein, the PAM is NNGG, and the target DNA sequence is represented by SEQ ID NO: 12;
for the *Sa-Seq*Cas9 protein, the PAM is NNGRM, and the target DNA sequence is represented by SEQ ID NO: 13;
for the *Sa-Sha*Cas9 protein, the PAM is NNGRM, and the target DNA sequence is represented by SEQ ID NO: 13;
for the *Sa-Slug*Cas9 protein, the PAM is NNGG, and the target DNA sequence is represented by SEQ ID NO: 12;
for the *Sa-Slut*Cas9 protein, the PAM is NNGRR, and the target DNA sequence is represented by SEQ ID NO: 15; and
for the *SlugCas9-HF* protein, the PAM is NNGG, and the target DNA sequence is represented by SEQ ID NO: 12.

9. The CRISPR/Cas9 system for gene editing according to claim 1, wherein the sgRNA is modified via phosphorylation, shortening, lengthening, sulfurization, methylation, or hydroxylation.

10. The CRISPR/Cas9 system for gene editing according to claim 1, wherein the complex of the Cas9 protein and the sgRNA being capable of accurately locating the target DNA sequence means that the complex of the Cas9 protein and the sgRNA is capable of recognizing and binding to the target DNA sequence, or that the complex of the Cas9 protein and the sgRNA is capable of carrying a further protein fused with the Cas9 protein or a protein that specifically recognizes the sgRNA to the place where the target DNA sequence is located.

11. The CRISPR/Cas9 system for gene editing according to claim 10, wherein the complex of the Cas9 protein and the sgRNA, or the further protein fused with the Cas9 protein, or the protein that specifically recognizes the sgRNA is capable of making modification and regulation to the target DNA region, and the modification and regulation comprises regulation of gene transcription level, DNA methylation regulation, DNA acetylation modification, histone acetylation modification, single-base conversion or chromatin imaging tracking.

12. The CRISPR/Cas9 system for gene editing according to any one of claims 6-8, wherein the complex of the *SlugCas9-HF* protein and the sgRNA is essentially incapable of or is incapable of recognizing and binding to the non-target DNA sequence, or the complex of the Cas9 protein and the sgRNA is essentially incapable of or is incapable of carrying the further protein fused with the *SlugCas9-HF* protein or the protein that specifically recognizes the sgRNA to the place where the non-target DNA sequence is located.

13. The CRISPR/Cas9 system for gene editing according to claim 12, wherein the complex of the *SlugCas9-HF* protein and the sgRNA, or the further protein fused with the *SlugCas9-HF* protein, or the protein that specifically recognizes the sgRNA is essentially incapable of or is incapable of making modification and regulation to a non-target DNA region, and the modification and regulation comprises regulation of gene transcription level, DNA methylation regulation, DNA acetylation modification, histone acetylation modification, single-base conversion or chromatin imaging tracking.

14. The CRISPR/Cas9 system for gene editing according to claim 11 or 13, wherein the single-base conversion comprises conversion of adenine to guanine, or conversion of cytosine to thymine, or conversion of cytosine to uracil, or conversion between other bases.

15. A method for gene editing in cells with the CRISPR/Cas9 system for gene editing according to any one of claims 1-14, wherein the method edits a target DNA sequence by recognizing and locating the target DNA sequence with a complex of a Cas9 protein and a sgRNA, and the method comprises the steps of:
(1) synthesizing a Cas9 gene sequence and cloning it into an expression vector such as pAAV2_ITR, to obtain an expression vector cloned with the Cas9 gene sequence, such as pAAV2_Cas9_ITR, wherein the Cas9 gene sequence:
(a) has a nucleotide sequence encoding an amino acid sequence represented by any one of SEQ ID NOs: 1-10 and SEQ ID NO: 58; and
(b) has a nucleotide sequence encoding an amino acid sequence that is at least 80% identical to the amino acid sequence represented by any one of SEQ ID NOs: 1-10 and SEQ ID NO: 58; or
(c) is a humanized Cas9 gene sequence, for example, the one having a nucleotide sequence represented by any one of SEQ ID NOs: 23-32 and SEQ ID NO: 112;
(2) synthesizing oligo single-stranded DNAs corresponding to the sgRNA, *i.e*., an oligo forward-strand sequence and an oligo reverse-strand sequence, and annealing and ligating the oligo forward-strand sequence and the oligo reverse-strand sequence to a restriction site of the expression vector cloned with the Cas9 gene sequence, such as the BsaI digestion site of plasmid pAAV2_Cas9_U6_BsaI, to obtain an expression vector, such as pAAV2_Cas9-hU6-sgRNA, for expressing the Cas9 protein and the sgRNA; wherein the sgRNA has a nucleotide sequence represented by SEQ ID NO: 11, or a nucleotide sequence that is at least 80% identical to the nucleotide sequence represented by SEQ ID NO: 11, or a modification comprising, for example, phosphorylation, shortening, lengthening, sulfurization, methylation or hydroxylation, based on the nucleotide sequence represented by SEQ ID NO: 11;
(3) delivering the expression vector expressing the Cas9 protein and the sgRNA to cells comprising a target site to edit the target site;
(4) optionally detecting editing efficiency towards the target site, for example, by PCR amplification on the edited target site followed by T7EI digestion or next-generation sequencing.

16. The method according to claim 15, wherein the pAAV2_Cas9-hU6-sgRNA is an adeno-associated virus backbone plasmid, comprising AAV2 ITR, a CMV enhancer, a CMV promoter, SV40 NLS, Cas9, nucleoplasmin NLS, 3x HA, bGH poly(A), a human U6 promoter, a BsaI endonuclease site, and a sgRNA scaffold sequence.

17. The method according to claim 15, wherein the CRISPR/Cas9 system delivered to the cell comprises: a plasmid, a retrovirus, an adenovirus, or an adeno-associated virus vector expressing the Cas9 protein and the sgRNA; or the sgRNA and the Cas9 protein.

18. The method according to claim 15, wherein, for other Cas9 genes than the *SlugCas9-HF* gene, the oligo forward-strand sequence and the oligo reverse-strand sequence have the nucleotide sequences represented by SEQ ID NO: 16 and SEQ ID NO: 17, respectively; for the *SlugCas9-HF* gene, the oligo forward-strand sequence and the oligo reverse-strand sequence comprise a first oligo forward-strand sequence and a first oligo reverse-strand sequence represented by SEQ ID NO: 59 and SEQ ID NO: 60, respectively, and a second oligo forward-strand sequence and a second oligo reverse-strand sequence represented by SEQ ID NO: 61 and SEQ ID NO: 62, respectively.

19. The method according to claim 15, wherein, for other Cas9 genes than the *SlugCas9-HF* gene, the target site in the cell in step (3) has a nucleotide sequence represented by SEQ ID NO: 18; and for the *SlugCas9-HF* gene, the target site in the cell in step (3) has nucleotide sequences represented by SEQ ID NO: 63 and SEQ ID NO: 64, respectively.

20. The method according to claim 15, wherein the PCR template in step (4) is an edited DNA; for other Cas9 genes than the *SlugCas9-HF* gene, the primer sequences for PCR amplification are represented by SEQ ID NO: 19, SEQ ID NO: 20, SEQ ID NO: 21, and SEQ ID NO: 22; and for the *SlugCas9-HF* gene, the primer sequences for PCR amplification are represented by SEQ ID NO: 65, SEQ ID NO: 66, SEQ ID NO: 21, SEQ ID NO: 22, and SEQ ID NO: 67.

21. The method according to claim 11, wherein the cell comprises eukaryotic cells and prokaryotic cells; the eukaryotic cells comprise, for example, mammalian cells and plant cells. The mammalian cells comprise, for example, Chinese hamster ovary cells, baby hamster kidney cells, mouse Sertoli cells, mouse breast tumor cells, buffalo rat hepatic cells, rat hepatoma cells, SV40-transformed monkey kidney CVI lines, monkey kidney cells, canine kidney cells, human cervical cancer cells, human lung cells, human liver cells, HIH/3T3 cells, human U2-OS osteosarcoma cells, human A549 cells, human K562 cells, human HEK293 cells, human HEK293T cells, human HCT116 cells, or human MCF-7 cells or TRI cells.

22. A kit of a CRISPR/Cas9 gene editing system for gene editing, the kit comprising:
(1) a Cas9 protein and a sgRNA, wherein the Cas9 protein is:
a SauriCas9 protein having an amino acid sequence represented by SEQ ID NO: 1,
a ShaCas9 protein having an amino acid sequence represented by SEQ ID NO: 2,
a *Slug*Cas9 protein having an amino acid sequence represented by SEQ ID NO: 3,
a *Slut*Cas9 protein having an amino acid sequence represented by SEQ ID NO: 4,
a *Sa-Sauri*Cas9 protein having an amino acid sequence represented by SEQ ID NO: 5,
a *Sa-Sep*Cas9 protein having an amino acid sequence represented by SEQ ID NO: 6,
a *Sa-Seq*Cas9 protein having an amino acid sequence represented by SEQ ID NO: 7,
a *Sa-Sha*Cas9 protein having an amino acid sequence represented by SEQ ID NO: 8,
a *Sa-Slug*Cas9 protein having an amino acid sequence represented by SEQ ID NO: 9,
a *Sa-Slut*Cas9 protein having an amino acid sequence represented by SEQ ID NO: 10, or
a *SlugCas9-HF* protein having an amino acid sequence represented by SEQ ID NO: 58, or
a Cas9 protein having an amino acid sequence that is at least 80% identical to the amino acid sequence represented by any one of SEQ ID NOs: 1-10 and SEQ ID NO: 58; and
the sgRNA has a nucleotide sequence represented by SEQ ID NO: 11, or is a modified sgRNA sequence based on SEQ ID NO: 11;
or
(2) an expression vector cloned with a Cas9 gene sequence and an oligo single-stranded DNA corresponding to a sgRNA, wherein
the Cas9 gene sequence:
(a) has a nucleotide sequence encoding an amino acid sequence represented by any one of SEQ ID NO: 1-10 and SEQ ID NO: 58; and
(b) has a nucleotide sequence encoding an amino acid sequence that is at least 80% identical to the amino acid sequence represented by any one of SEQ ID NO: 1-10 and SEQ ID NO: 58; or
(c) is a humanized Cas9 gene sequence, for example, the one having a nucleotide sequence represented by any one of SEQ ID NOs: 23-32 and SEQ ID NO: 112; and
the sgRNA has a nucleotide sequence represented by SEQ ID NO: 11, or a modified sgRNA sequence based on SEQ ID NO: 11.

23. Use of the CRISPR/Cas9 system for gene editing according to any one of claims 1-14 in gene knockout, site-directed base change, site-directed insertion, regulation of gene transcription level, regulation of DNA methylation, modification of DNA acetylation, modification of histone acetylation, single-base conversion or chromatin imaging tracking.
